(19) Europäisches Patentamt
European Patent Office
Office européen des brevets

(11) EP 2 016 949 A1

(12) **EUROPEAN PATENT APPLICATION**
published in accordance with Art. 153(4) EPC

(43) Date of publication:
**21.01.2009 Bulletin 2009/04**

(21) Application number: **07740621.3**

(22) Date of filing: **30.03.2007**

(51) Int Cl.:
***A61K 38/00*** *(2006.01)*

(86) International application number:
**PCT/JP2007/057185**

(87) International publication number:
**WO 2007/119623 (25.10.2007 Gazette 2007/43)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **31.03.2006 JP 2006101057**

(71) Applicant: **Riken**
**Wako-shi, Saitama 351-0198 (JP)**

(72) Inventors:
- **HIGASHI, Hideyoshi**
**Wako-shi, Saitama 351-0198 (JP)**
- **KOHYAMA, Ayako**
**Wako-shi, Saitama 351-0198 (JP)**
- **HIRABAYASHI, Yoshio**
**Wako-shi, Saitama 351-0198 (JP)**

(74) Representative: **Duckworth, Timothy John**
**J.A. Kemp & Co.,**
**14 South Square,**
**Gray's Inn**
**London WC1R 5JJ (GB)**

# (54) NOVEL USE OF G-PROTEIN-CONJUGATED RECEPTOR AND LIGAND THEREOF

(57) The present invention provides a screening method for a substance that alters the bindability of RAIG2 or RAIG3 and glucose or 1,5-anhydroglucitol, including using RAIG2 or RAIG3 and glucose or 1,5-anhydroglucitol, and a regulator of sugar/lipid metabolism, a regulator of pancreas/pituitary function, a regulator of insulin secretion/blood glucose and ACTH secretion, and a prophylactic/therapeutic agent for a disease involved by an abnormality thereof, containing an RAIG2 or RAIG3 agonist or antagonist.

EP 2 016 949 A1

SEQUENCE LISTING

```
<110>  RIKEN

<120>  NOVEL USE OF G-PROTEIN-CONJUGATED RECEPTOR AND LIGAND THEREOF

<130>  091065

<150>  JP 2006-101057
<151>  2006-03-31

<160>  4

<170>  PatentIn version 3.3

<210>  1
<211>  1209
<212>  DNA
<213>  Homo sapiens


<220>
<221>  CDS
<222>  (1)..(1209)

<400>  1
atg ttc gtg gca tca gag aga aag atg aga gct cac cag gtg ctc acc        48
Met Phe Val Ala Ser Glu Arg Lys Met Arg Ala His Gln Val Leu Thr
1               5                   10                  15

ttc ctc ctg ctc ttc gtg atc acc tcg gtg gcc tct gaa aac gcc agc        96
Phe Leu Leu Leu Phe Val Ile Thr Ser Val Ala Ser Glu Asn Ala Ser
            20                  25                  30

aca tcc cga ggc tgt ggg ctg gac ctc ctc cct cag tac gtg tcc ctg       144
Thr Ser Arg Gly Cys Gly Leu Asp Leu Leu Pro Gln Tyr Val Ser Leu
        35                  40                  45

tgc gac ctg gac gcc atc tgg ggc att gtg gtg gag gcg gtg gcc ggg       192
Cys Asp Leu Asp Ala Ile Trp Gly Ile Val Val Glu Ala Val Ala Gly
    50                  55                  60

gcg ggc gcc ctg atc aca ctg ctc ctg atg ctc atc ctc ctg gtg cgg       240
Ala Gly Ala Leu Ile Thr Leu Leu Leu Met Leu Ile Leu Leu Val Arg
65                  70                  75                  80

ctg ccc ttc atc aag gag aag gag aag aag agc cct gtg ggc ctc cac       288
Leu Pro Phe Ile Lys Glu Lys Glu Lys Lys Ser Pro Val Gly Leu His
                85                  90                  95

ttt ctg ttc ctc ctg ggg acc ctg ggc ctc ttt ggg ctg acg ttt gcc       336
Phe Leu Phe Leu Leu Gly Thr Leu Gly Leu Phe Gly Leu Thr Phe Ala
            100                 105                 110

ttc atc atc cag gag gac gag acc atc tgc tct gtc cgc cgc ttc ctc       384
Phe Ile Ile Gln Glu Asp Glu Thr Ile Cys Ser Val Arg Arg Phe Leu
        115                 120                 125

tgg ggc gtc ctc ttt gcg ctc tgc ttc tcc tgc ctg ctg agc cag gca       432
Trp Gly Val Leu Phe Ala Leu Cys Phe Ser Cys Leu Leu Ser Gln Ala
    130                 135                 140

tgg cgc gtg cgg agg ctg gtg cgg cat ggc acg ggc ccc gcg ggc tgg       480
Trp Arg Val Arg Arg Leu Val Arg His Gly Thr Gly Pro Ala Gly Trp
145                 150                 155                 160

cag ctg gtg ggc ctg gcg ctg tgc ctg atg ctg gtg caa gtc atc atc       528
Gln Leu Val Gly Leu Ala Leu Cys Leu Met Leu Val Gln Val Ile Ile
                165                 170                 175

gct gtg gag tgg ctg gtg ctc acc gtg ctg cgt gac aca agg cca gcc       576
Ala Val Glu Trp Leu Val Leu Thr Val Leu Arg Asp Thr Arg Pro Ala
            180                 185                 190
```

```
tgc gcc tac gag ccc atg gac ttt gtg atg gcc ctc atc tac gac atg        624
Cys Ala Tyr Glu Pro Met Asp Phe Val Met Ala Leu Ile Tyr Asp Met
        195                 200                 205

gta ctg ctt gtg gtc acc ctg ggg ctg gcc ctc ttc act ctg tgc ggc        672
Val Leu Leu Val Val Thr Leu Gly Leu Ala Leu Phe Thr Leu Cys Gly
    210                 215                 220

aag ttc aag agg tgg aag ctg aac ggg gcc ttc ctc ctc atc aca gcc        720
Lys Phe Lys Arg Trp Lys Leu Asn Gly Ala Phe Leu Leu Ile Thr Ala
225                 230                 235                 240

ttc ctc tct gtg ctc atc tgg gtg gcc tgg atg acc atg tac ctc ttc        768
Phe Leu Ser Val Leu Ile Trp Val Ala Trp Met Thr Met Tyr Leu Phe
                245                 250                 255

ggc aat gtc aag ctg cag cag ggg gat gcc tgg aac gac ccc acc ttg        816
Gly Asn Val Lys Leu Gln Gln Gly Asp Ala Trp Asn Asp Pro Thr Leu
            260                 265                 270

gcc atc acg ctg gcg gcc agc ggc tgg gtc ttc gtc atc ttc cac gcc        864
Ala Ile Thr Leu Ala Ala Ser Gly Trp Val Phe Val Ile Phe His Ala
        275                 280                 285

atc cct gag atc cac tgc acc ctt ctg cca gcc ctg cag gag aac acg        912
Ile Pro Glu Ile His Cys Thr Leu Leu Pro Ala Leu Gln Glu Asn Thr
    290                 295                 300

ccc aac tac ttc gac acg tcg cag ccc agg atg cgg gag acg gcc ttc        960
Pro Asn Tyr Phe Asp Thr Ser Gln Pro Arg Met Arg Glu Thr Ala Phe
305                 310                 315                 320

gag gag gac gtg cag ctg ccg cgg gcc tat atg gag aac aag gcc ttc       1008
Glu Glu Asp Val Gln Leu Pro Arg Ala Tyr Met Glu Asn Lys Ala Phe
                325                 330                 335

tcc atg gat gaa cac aat gca gct ctc cga aca gca gga ttt ccc aac       1056
Ser Met Asp Glu His Asn Ala Ala Leu Arg Thr Ala Gly Phe Pro Asn
            340                 345                 350

ggc agc ttg gga aaa aga ccc agt ggc agc ttg ggg aaa aga ccc agc       1104
Gly Ser Leu Gly Lys Arg Pro Ser Gly Ser Leu Gly Lys Arg Pro Ser
        355                 360                 365

gct ccg ttt aga agc aac gtg tat cag cca act gag atg gcc gtc gtg       1152
Ala Pro Phe Arg Ser Asn Val Tyr Gln Pro Thr Glu Met Ala Val Val
    370                 375                 380

ctc aac ggt ggg acc atc cca act gct ccg cca agt cac aca gga aga       1200
Leu Asn Gly Gly Thr Ile Pro Thr Ala Pro Pro Ser His Thr Gly Arg
385                 390                 395                 400

cac ctt tgg                                                           1209
His Leu Trp


<210>  2
<211>  403
<212>  PRT
<213>  Homo sapiens

<400>  2

Met Phe Val Ala Ser Glu Arg Lys Met Arg Ala His Gln Val Leu Thr
1               5                   10                  15


Phe Leu Leu Leu Phe Val Ile Thr Ser Val Ala Ser Glu Asn Ala Ser
            20                  25                  30


Thr Ser Arg Gly Cys Gly Leu Asp Leu Leu Pro Gln Tyr Val Ser Leu
        35                  40                  45
```

Cys Asp Leu Asp Ala Ile Trp Gly Ile Val Val Glu Ala Val Ala Gly
50 55 60

Ala Gly Ala Leu Ile Thr Leu Leu Leu Met Leu Ile Leu Leu Val Arg
65 70 75 80

Leu Pro Phe Ile Lys Glu Lys Glu Lys Lys Ser Pro Val Gly Leu His
85 90 95

Phe Leu Phe Leu Leu Gly Thr Leu Gly Leu Phe Gly Leu Thr Phe Ala
100 105 110

Phe Ile Ile Gln Glu Asp Glu Thr Ile Cys Ser Val Arg Arg Phe Leu
115 120 125

Trp Gly Val Leu Phe Ala Leu Cys Phe Ser Cys Leu Leu Ser Gln Ala
130 135 140

Trp Arg Val Arg Arg Leu Val Arg His Gly Thr Gly Pro Ala Gly Trp
145 150 155 160

Gln Leu Val Gly Leu Ala Leu Cys Leu Met Leu Val Gln Val Ile Ile
165 170 175

Ala Val Glu Trp Leu Val Leu Thr Val Leu Arg Asp Thr Arg Pro Ala
180 185 190

Cys Ala Tyr Glu Pro Met Asp Phe Val Met Ala Leu Ile Tyr Asp Met
195 200 205

Val Leu Leu Val Val Thr Leu Gly Leu Ala Leu Phe Thr Leu Cys Gly
210 215 220

Lys Phe Lys Arg Trp Lys Leu Asn Gly Ala Phe Leu Leu Ile Thr Ala
225 230 235 240

Phe Leu Ser Val Leu Ile Trp Val Ala Trp Met Thr Met Tyr Leu Phe
245 250 255

Gly Asn Val Lys Leu Gln Gln Gly Asp Ala Trp Asn Asp Pro Thr Leu
260 265 270

Ala Ile Thr Leu Ala Ala Ser Gly Trp Val Phe Val Ile Phe His Ala
275 280 285

Ile Pro Glu Ile His Cys Thr Leu Leu Pro Ala Leu Gln Glu Asn Thr
290 295 300

Pro Asn Tyr Phe Asp Thr Ser Gln Pro Arg Met Arg Glu Thr Ala Phe
305 310 315 320

Glu Glu Asp Val Gln Leu Pro Arg Ala Tyr Met Glu Asn Lys Ala Phe
325 330 335

Ser Met Asp Glu His Asn Ala Ala Leu Arg Thr Ala Gly Phe Pro Asn
340 345 350

Gly Ser Leu Gly Lys Arg Pro Ser Gly Ser Leu Gly Lys Arg Pro Ser
355 360 365

Ala Pro Phe Arg Ser Asn Val Tyr Gln Pro Thr Glu Met Ala Val Val
370 375 380

Leu Asn Gly Gly Thr Ile Pro Thr Ala Pro Pro Ser His Thr Gly Arg
385 390 395 400

His Leu Trp

<210> 3
<211> 1359
<212> DNA
<213> Homo sapiens

<220>
<221> CDS
<222> (1)..(1359)

<400> 3
atg ggg acc caa cca gag cct ggc ctg gga gcc agg atg gcc atc cac 48
Met Gly Thr Gln Pro Glu Pro Gly Leu Gly Ala Arg Met Ala Ile His
1 5 10 15

aaa gcc ttg gtg atg tgc ctg gga ctg cct ctc ttc ctg ttc cca ggg 96
Lys Ala Leu Val Met Cys Leu Gly Leu Pro Leu Phe Leu Phe Pro Gly
20 25 30

gcc tgg gcc cag ggc cat gtc cca ccc ggc tgc agc caa ggc ctc aac 144
Ala Trp Ala Gln Gly His Val Pro Pro Gly Cys Ser Gln Gly Leu Asn
35 40 45

ccc ctg tac tac aac ctg tgt gac cgc tct ggg gcg tgg ggc atc gtc 192
Pro Leu Tyr Tyr Asn Leu Cys Asp Arg Ser Gly Ala Trp Gly Ile Val
50 55 60

ctg gag gcc gtg gct ggg gcg ggc att gtc acc acg ttt gtg ctc acc 240
Leu Glu Ala Val Ala Gly Ala Gly Ile Val Thr Thr Phe Val Leu Thr
65 70 75 80

atc atc ctg gtg gcc agc ctc ccc ttt gtg cag gac acc aag aaa cgg 288
Ile Ile Leu Val Ala Ser Leu Pro Phe Val Gln Asp Thr Lys Lys Arg
85 90 95

agc ctg ctg ggg acc cag gta ttc ttc ctt ctg ggg acc ctg ggc ctc 336
Ser Leu Leu Gly Thr Gln Val Phe Phe Leu Leu Gly Thr Leu Gly Leu
100 105 110

ttc tgc ctc gtg ttt gcc tgt gtg gtg aag ccc gac ttc tcc acc tgt 384
Phe Cys Leu Val Phe Ala Cys Val Val Lys Pro Asp Phe Ser Thr Cys
115 120 125

gcc tct cgg cgc ttc ctc ttt ggg gtt ctg ttc gcc atc tgc ttc tct 432
Ala Ser Arg Arg Phe Leu Phe Gly Val Leu Phe Ala Ile Cys Phe Ser
130 135 140

tgt ctg gcg gct cac gtc ttt gcc ctc aac ttc ctg gcc cgg aag aac 480
Cys Leu Ala Ala His Val Phe Ala Leu Asn Phe Leu Ala Arg Lys Asn
145 150 155 160

cac ggg ccc cgg ggc tgg gtg atc ttc act gtg gct ctg ctg ctg acc 528
His Gly Pro Arg Gly Trp Val Ile Phe Thr Val Ala Leu Leu Leu Thr
165 170 175

```
ctg gta gag gtc atc atc aat aca gag tgg ctg atc atc acc ctg gtt       576
Leu Val Glu Val Ile Ile Asn Thr Glu Trp Leu Ile Ile Thr Leu Val
            180                 185                 190

cgg ggc agt ggc gag ggc ggc cct cag ggc aac agc agc gca ggc tgg       624
Arg Gly Ser Gly Glu Gly Gly Pro Gln Gly Asn Ser Ser Ala Gly Trp
        195                 200                 205

gcc gtg gcc tcc ccc tgt gcc atc gcc aac atg gac ttt gtc atg gca       672
Ala Val Ala Ser Pro Cys Ala Ile Ala Asn Met Asp Phe Val Met Ala
    210                 215                 220

ctc atc tac gtc atg ctg ctg ctg ctg ggt gcc ttc ctg ggg gcc tgg       720
Leu Ile Tyr Val Met Leu Leu Leu Leu Gly Ala Phe Leu Gly Ala Trp
225                 230                 235                 240

ccc gcc ctg tgt ggc cgc tac aag cgc tgg cgt aag cat ggg gtc ttt       768
Pro Ala Leu Cys Gly Arg Tyr Lys Arg Trp Arg Lys His Gly Val Phe
                245                 250                 255

gtg ctc ctc acc aca gcc acc tcc gtt gcc ata tgg gtg gtg tgg atc       816
Val Leu Leu Thr Thr Ala Thr Ser Val Ala Ile Trp Val Val Trp Ile
            260                 265                 270

gtc atg tat act tac ggc aac aag cag cac aac agt ccc acc tgg gat       864
Val Met Tyr Thr Tyr Gly Asn Lys Gln His Asn Ser Pro Thr Trp Asp
        275                 280                 285

gac ccc acg ctg gcc atc gcc ctc gcc gcc aat gcc tgg gcc ttc gtc       912
Asp Pro Thr Leu Ala Ile Ala Leu Ala Ala Asn Ala Trp Ala Phe Val
    290                 295                 300

ctc ttc tac gtc atc ccc gag gtc tcc cag gtg acc aag tcc agc cca       960
Leu Phe Tyr Val Ile Pro Glu Val Ser Gln Val Thr Lys Ser Ser Pro
305                 310                 315                 320

gag caa agc tac cag ggg gac atg tac ccc acc cgg ggc gtg ggc tat      1008
Glu Gln Ser Tyr Gln Gly Asp Met Tyr Pro Thr Arg Gly Val Gly Tyr
                325                 330                 335

gag acc atc ctg aaa gag cag aag ggt cag agc atg ttc gtg gag aac      1056
Glu Thr Ile Leu Lys Glu Gln Lys Gly Gln Ser Met Phe Val Glu Asn
            340                 345                 350

aag gcc ttt tcc atg gat gag ccg gtt gca gct aag agg ccg gtg tca      1104
Lys Ala Phe Ser Met Asp Glu Pro Val Ala Ala Lys Arg Pro Val Ser
        355                 360                 365

cca tac agc ggg tac aat ggg cag ctg ctg acc agt gtg tac cag ccc      1152
Pro Tyr Ser Gly Tyr Asn Gly Gln Leu Leu Thr Ser Val Tyr Gln Pro
    370                 375                 380

act gag atg gcc ctg atg cac aaa gtt ccg tcc gaa gga gct tac gac      1200
Thr Glu Met Ala Leu Met His Lys Val Pro Ser Glu Gly Ala Tyr Asp
385                 390                 395                 400

atc atc ctc cca cgg gcc acc gcc aac agc cag gtg atg ggc agt gcc      1248
Ile Ile Leu Pro Arg Ala Thr Ala Asn Ser Gln Val Met Gly Ser Ala
                405                 410                 415

aac tcg acc ctg cgg gct gaa gac atg tac tcg gcc cag agc cac cag      1296
Asn Ser Thr Leu Arg Ala Glu Asp Met Tyr Ser Ala Gln Ser His Gln
            420                 425                 430

gcg gcc aca ccg ccg aaa gac ggc aag aac tct cag gtc ttt aga aac      1344
Ala Ala Thr Pro Pro Lys Asp Gly Lys Asn Ser Gln Val Phe Arg Asn
        435                 440                 445

ccc tac gtg tgg gac                                                  1359
Pro Tyr Val Trp Asp
    450
```

<210> 4
<211> 453
<212> PRT
<213> Homo sapiens

<400> 4

Met Gly Thr Gln Pro Glu Pro Gly Leu Gly Ala Arg Met Ala Ile His
1 5 10 15

Lys Ala Leu Val Met Cys Leu Gly Leu Pro Leu Phe Leu Phe Pro Gly
20 25 30

Ala Trp Ala Gln Gly His Val Pro Pro Gly Cys Ser Gln Gly Leu Asn
35 40 45

Pro Leu Tyr Tyr Asn Leu Cys Asp Arg Ser Gly Ala Trp Gly Ile Val
50 55 60

Leu Glu Ala Val Ala Gly Ala Gly Ile Val Thr Thr Phe Val Leu Thr
65 70 75 80

Ile Ile Leu Val Ala Ser Leu Pro Phe Val Gln Asp Thr Lys Lys Arg
85 90 95

Ser Leu Leu Gly Thr Gln Val Phe Phe Leu Leu Gly Thr Leu Gly Leu
100 105 110

Phe Cys Leu Val Phe Ala Cys Val Val Lys Pro Asp Phe Ser Thr Cys
115 120 125

Ala Ser Arg Arg Phe Leu Phe Gly Val Leu Phe Ala Ile Cys Phe Ser
130 135 140

Cys Leu Ala Ala His Val Phe Ala Leu Asn Phe Leu Ala Arg Lys Asn
145 150 155 160

His Gly Pro Arg Gly Trp Val Ile Phe Thr Val Ala Leu Leu Leu Thr
165 170 175

Leu Val Glu Val Ile Ile Asn Thr Glu Trp Leu Ile Ile Thr Leu Val
180 185 190

Arg Gly Ser Gly Glu Gly Gly Pro Gln Gly Asn Ser Ser Ala Gly Trp
195 200 205

Ala Val Ala Ser Pro Cys Ala Ile Ala Asn Met Asp Phe Val Met Ala
210 215 220

Leu Ile Tyr Val Met Leu Leu Leu Leu Gly Ala Phe Leu Gly Ala Trp
225 230 235 240

Pro Ala Leu Cys Gly Arg Tyr Lys Arg Trp Arg Lys His Gly Val Phe
245 250 255

Val Leu Leu Thr Thr Ala Thr Ser Val Ala Ile Trp Val Val Trp Ile
260 265 270

```
Val Met Tyr Thr Tyr Gly Asn Lys Gln His Asn Ser Pro Thr Trp Asp
        275                 280                 285

Asp Pro Thr Leu Ala Ile Ala Leu Ala Ala Asn Ala Trp Ala Phe Val
    290                 295                 300

Leu Phe Tyr Val Ile Pro Glu Val Ser Gln Val Thr Lys Ser Ser Pro
305                 310                 315                 320

Glu Gln Ser Tyr Gln Gly Asp Met Tyr Pro Thr Arg Gly Val Gly Tyr
                325                 330                 335

Glu Thr Ile Leu Lys Glu Gln Lys Gly Gln Ser Met Phe Val Glu Asn
            340                 345                 350

Lys Ala Phe Ser Met Asp Glu Pro Val Ala Ala Lys Arg Pro Val Ser
        355                 360                 365

Pro Tyr Ser Gly Tyr Asn Gly Gln Leu Leu Thr Ser Val Tyr Gln Pro
    370                 375                 380

Thr Glu Met Ala Leu Met His Lys Val Pro Ser Glu Gly Ala Tyr Asp
385                 390                 395                 400

Ile Ile Leu Pro Arg Ala Thr Ala Asn Ser Gln Val Met Gly Ser Ala
                405                 410                 415

Asn Ser Thr Leu Arg Ala Glu Asp Met Tyr Ser Ala Gln Ser His Gln
            420                 425                 430

Ala Ala Thr Pro Pro Lys Asp Gly Lys Asn Ser Gln Val Phe Arg Asn
        435                 440                 445

Pro Tyr Val Trp Asp
    450
```

## Description

### Technical Field

The present invention relates to the identification of a glucose receptor and a ligand thereof other than glucose, and a use thereof. More specifically, the present invention relates to a novel pharmaceutical use for a glucose receptor or a ligand thereof, and a screening method for a novel pharmaceutical candidate compound using the receptor (and a ligand thereof) and the like.

### Background Art

Conventionally, it has been thought that blood glucose sensing and insulin secretion are based on intracellular sensing of glucose phosphate resulting from phosphorylation of glucose incorporated in cells. Specifically, in pancreatic β cells, glucose is incorporated in cells by GLUT2, and converted to glucose-6-phosphate (G6P) by glucokinase. G6P is metabolized in cytoplasm (glycolysis system) and mitochondria (TCA cycle and respiratory chain) to produce ATP, and the ATP/ADP ratio increases. As a result, the ATP-dependent $K^+$ channel on the cell membrane closes to cause depolarization, and the voltage-dependent $Ca^{2+}$ channel (VDC) opens to allow the cells to incorporate $Ca^{2+}$ from outside, causing $Ca^{2+}$-dependent release of insulin-secreting granules.

However, this mechanism cannot explain the insulin secretion action by sugars that are not metabolized, such as 1,5-anhydroglucitol (widely used as a useful diabetes marker since the blood concentration thereof decreases remarkably because of loss in the urine, and normalizes with amelioration of the pathologic condition, in diabetic patients; hereinafter sometimes referred to as "1,5-AG"), and is inconsistent with the fact that glucose metabolism or energy production and insulin secretion action are separable. The mechanism is also inconsistent with the fact that insulin secretion occurs before the glycolysis product in the pancreatic islets increases. Although these inconsistencies can be explained by a hypothesis of the presence of a receptor that senses blood glucose in the cell surface, no such receptors have been identified to date.

About half of the existing pharmaceutical products are known to exhibit the actions thereof via receptors on the cell membrane; in particular, 7-pass transmembrane G protein-coupled receptors (hereinafter also referred to as GPCR) account for a particularly high percentage, and are therefore attracting attention as the most promising target for drug discovery.

A subfamily of orphan GPCRs as classified according to sequence homology [G Protein-coupled Receptor, family C, group 5 (GPRC5), members A-D] have been reported (see, for example, non-patent document 1). Of the members, GPRC5A, B and C are also called Retinoic Acid-Inducible Gene (RIAG)-1, 2 and 3, respectively, because the expression thereof is induced by retinoic acid. The receptors exhibit a mutual homology of about 30 to 40%, and are characterized by shorter N-terminal domains than those of the other GPCR members belonging to the family C. It has been reported that RAIG2 is expressed mainly in the central nervous system, and that RAIG3 and GPRC5D are expressed mainly in peripheral tissues, including the pancreas (non-patent documents 1 to 4). Also, since food consumption increased and the blood glucose level rose in a mouse model of obesity having the expression of GPRC5D suppressed therein, prophylaxis/treatment of type 2 diabetes mellitus and other lifestyle-related diseases by enhancement of the expression or activity of the receptor has been proposed (patent document 1).

However, physiological ligands of these receptors remain unidentified, and the physiological significance thereof has not yet fully been clarified.

Patent document 1: Pamphlet for WO 03/055507 (whole text)

Non-patent document 1: M.J. Robbins et al., "Brain Res. Mol. Brain Res." (Netherlands), Vol. 106, pp. 136-144 (2002)

Non-patent document 2: H. Brauner-Osborne and P. Krogsgaard-Larsen, "Genomics" (USA), Vol. 65, pp. 121-128 (2000)

Non-patent document 3: M.J. Robbins et al., "Genomics" (USA), Vol. 67, pp. 8-18 (2000)

Non-patent document 4: H. Brauner-Osborne et al., "Biochim. Biophys. Acta" (Netherlands), Vol. 1518, pp. 237-248 (2001)

### Disclosure of the Invention

### Problems to Be Solved by the Invention

It is an object of the present invention to identify a sugar receptor on the cell surface to demonstrate the presence of a mechanism for detection of blood sugar concentrations, and to provide a screening system for an agonist and/or antagonist of the receptor, i.e., a pharmaceutical candidate compound that is effective as a prophylactic/therapeutic drug for diabetes mellitus, insulin resistance, hypertension, hyperlipemia and the like, using the receptor and a ligand thereof.

**Means of Solving the Problems**

The present inventors diligently investigated with the aim of accomplishing the above-described object, and identified two kinds of GPCR that had conventionally been regarded as orphan receptors, i.e., RAIG2 and RAIG3, as cell surface glucose receptors. These receptors caused insulin secretion from insulin-secreting cells around glucose concentrations equivalent to normal blood glucose levels. The present inventors found that these receptors were also reactive with 1,5-AG, a conventionally known diabetes marker, to induce insulin secretion. Furthermore, the present inventors showed that these receptors also regulated the secretion of adrenocorticotropic hormone (hereinafter also referred to as "ACTH") in the pituitary, depending on glucose concentration.
Taking note of the fact that a RAIG2 homologues (bride of sevenless (boss)) in the drosophila is expressed in its organs corresponding to the pancreas and pituitary, the present inventors compared the blood glucose levels obtained from a boss gene-deficient mutant and a transgenic counterpart having the gene expressed forcibly therein, and found that the blood glucose level rose in the former compared to the wild type, and that this increase was suppressed in the latter. This finding suggested that BOSS, like RAIG2 and RAIG3, might also sense blood glucose concentrations and regulate insulin signals.
The present inventors conducted further investigations based on these findings, and developed the present invention.
Accordingly, the present invention provides:

[1] a pharmaceutical or animal drug comprising RAIG2 or a partial peptide thereof and/or RAIG3 or a partial peptide thereof,
[2] the pharmaceutical or animal drug according to [1] above, which is a regulator of sugar/lipid metabolism,
[3] the pharmaceutical or animal drug according to [1] above, which is a regulator of pancreas and/or pituitary function,
[4] the pharmaceutical or animal drug according to [1] above, which is an insulin secretion promoter and/or an agent for reducing blood glucose level and/or an adrenocorticotropic hormone secretion regulator,
[5] the pharmaceutical or animal drug according to [1] above, which is a prophylactic/therapeutic agent for a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia and memory and learning disorder,
[6] a pharmaceutical or animal drug comprising a nucleic acid that encodes RAIG2 or a partial peptide thereof and/or a nucleic acid that encodes RAIG3 or a partial peptide thereof,
[7] the pharmaceutical or animal drug according to [6] above, which is a regulator of sugar/lipid metabolism,
[8] the pharmaceutical or animal druq according to [6] above, which is a regulator of pancreas and/or pituitary function,
[9] the pharmaceutical or animal drug according to [6] above, which is an insulin secretion promoter and/or an agent for reducing blood glucose level and/or an adrenocorticotropic hormone secretion regulator,
[10] the pharmaceutical or animal drug according to [6] above, which is a prophylactic/therapeutic agent for a disease selected from the group consisting of diabetes mellitus, impaired glucose tolerance, ketosis, acidosis, diabetic neuropathy, diabetic nephropathy; diabetic retinopathy, hyperlipemia, eating disorder, depression, Cushing's disease, gonadal dysfunction, dermatopathies, arthropathies, osteopenia, arteriosclerosis, thrombotic disease, dyspepsia and memory and learning disorder,
[11] a diagnostic reagent comprising a nucleic acid containing a base sequence that encodes RAIG2 or a portion thereof and/or a nucleic acid containing a base sequence that encodes RAIG3 or a portion thereof,
[12] the reagent according to [11] above, which is for diagnosing a sugar/lipid metabolism abnormality,
[13] the reagent according to [11] above, which is for diagnosing a pancreas and/or pituitary function abnormality,
[14] the reagent according to [11] above, which is for diagnosing an insulin secretion and/or blood glucose abnormality, and/or an adrenocorticotropic hormone secretion abnormality,
[15] the reagent according to [11] above, which is for diagnosing a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder, obesity, hypoglycemia, hypertension, edema, insulin resistance syndrome, brittle diabetes, fatty atrophy, insulin allergy, insulinoma, fat toxicity and cancers,
[16] a diagnostic reagent comprising an antibody against RAIG2 and/or an antibody against RAIG3,
[17] the reagent according to [16] above, which is for diagnosing a sugar/lipid metabolism abnormality,
[18] the reagent according to [16] above, which is for diagnosing a pancreas and/or pituitary function abnormality,
[19] the reagent according to [16] above, which is for diagnosing an insulin secretion and/or blood glucose abnormality, and/or an adrenocorticotropic hormone secretion abnormality,
[20] the reagent according to [16] above, which is for diagnosing a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder, obesity, hypoglycemia, hypertension, edema, insulin resistance syndrome, brittle diabetes, fatty atrophy, insulin allergy, insulinoma, fat toxicity and cancers,

[21] a pharmaceutical or animal drug comprising a neutralizing antibody against RAIG2 and/or a neutralizing antibody against RAIG3,

[22] the pharmaceutical or animal drug according to [21] above, which is a regulator of sugar/lipid metabolism,

[23] the pharmaceutical or animal drug according to [21] above, which is a regulator of pancreas and/or pituitary function,

[24] the pharmaceutical or animal drug according to [21] above, which is an insulin secretion suppressor and/or an agent for increasing blood glucose level and/or an adrenocorticotropic hormone secretion regulator,

[25] the pharmaceutical or animal drug according to [21] above, which is a prophylactic/therapeutic agent for a disease selected from the group consisting of obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers,

[26] a pharmaceutical or animal drug comprising a nucleic acid containing a complementary strand sequence of a base sequence that encodes RAIG2 or a portion thereof and/or a nucleic acid containing a complementary strand sequence of a base sequence that encodes RAIG3 or a portion thereof,

[27] the pharmaceutical or animal drug according to [26] above, which is a regulator of sugar/lipid metabolism,

[28] the pharmaceutical or animal drug according to [26] above, which is a regulator of pancreas and/or pituitary function,

[29] the pharmaceutical or animal drug according to [26] above, which is an insulin secretion suppressor and/or an agent for increasing blood glucose level and/or an adrenocorticotropic hormone secretion regulator,

[30] the pharmaceutical or animal drug according to [26] above, which is a prophylactic/therapeutic agent for a disease selected from the group consisting of obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers,

[31] a screening method for a substance that regulates sugar/lipid metabolism, comprising using RAIG2 or a partial peptide thereof or RAIG3 or a partial peptide thereof,

[32] a screening method for a substance that regulates pancreas and/or pituitary function, comprising using RAIG2 or a partial peptide thereof or RAIG3 or a partial peptide thereof,

[33] a screening method for a substance that regulates insulin secretion and/or blood glucose and/or adrenocorticotropic hormone secretion, comprising using RAIG2 or a partial peptide thereof or RAIG3 or a partial peptide thereof,

[34] a screening method for a prophylactic/therapeutic substance for a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder, obesity, hypoglycemia, hypertension, edema, insulin resistance syndrome, brittle diabetes, fatty atrophy, insulin allergy, insulinoma, fat toxicity and cancers, comprising using RAIG2 or a partial peptide thereof or RAIG3 or a partial peptide thereof,

[35] the method according to any one of [31] to [34] above, wherein RAIG2 or a partial peptide thereof or RAIG3 or a partial peptide thereof is supplied in the form of a eukaryotic cell that produces the same,

[36] the method according to [35] above, comprising further using a nucleic acid containing a base sequence that encodes RAIG2 or a portion thereof or a nucleic acid containing a base sequence that encodes RAIG3 or a portion thereof, or an antibody against RAIG2 or an antibody against RAIG3,

[37] the method according to [35] above, wherein secretion of insulin or adrenocorticotropic hormone serves as an index,

[38] the method according to [35] above, wherein the intracellular $Ca^{2+}$ concentration or cAMP production serves as an index,

[39] the method according to [35] above, wherein the expression of a reporter or selection marker gene serves as an index,

[40] a screening method for a substance that alters the bindability of RAIG2 or RAIG3 and glucose or 1,5-anhydroglucitol, comprising using RAIG2 or a partial peptide thereof or RAIG3 or a partial peptide thereof, and glucose or 1,5-anhydroglucitol,

[41] the method according to [40] above, wherein RAIG2 or a partial peptide thereof or RAIG3 or a partial peptide thereof is supplied in the form of a cell that produces the same,

[42] the method according to [41] above, wherein secretion of insulin or adrenocorticotropic hormone serves as an index,

[43] the method according to [41] above, wherein the intracellular $Ca^{2+}$ concentration or cAMP production serves as an index,

[44] the method according to [41] above, wherein the expression of a reporter or selection marker gene serves as an index,

[45] a pharmaceutical or animal drug comprising a substance that enhances the expression or activity of RAIG2 or RAIG3,

[46] the pharmaceutical or animal drug according to [45] above, wherein the substance that enhances the expression or activity of RAIG2 or RAIG3 is an agonist of RAIG2 or RAIG3,

[47] the pharmaceutical or animal drug according to [45] or [46] above, which is a regulator of sugar/lipid metabolism,

[48] the pharmaceutical or animal drug according to [45] or [46] above, which is a regulator of pancreas and/or pituitary function,

[49] the pharmaceutical or animal drug according to [45] or [46] above, which is an insulin secretion promoter and/or an agent for reducing blood glucose level and/or an adrenocorticotropic hormone secretion regulator,

[50] the pharmaceutical or animal drug according to [45] or [46] above, which is a prophylactic/therapeutic agent for a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia and memory and learning disorder,

[51] a pharmaceutical or animal drug comprising a substance that inhibits the expression or activity of RAIG2 or RAIG3,

[52] the pharmaceutical or animal drug according to [51] above, wherein the substance that inhibits the expression or activity of RAIG2 or RAIG3 is an antagonist of RAIG2 or RAIG3,

[53] the pharmaceutical or animal drug according to [51] or [52] above, which is a regulator of sugar/lipid metabolism,

[54] the pharmaceutical or animal drug according to [51] or [52] above, which is a regulator of pancreas and/or pituitary function,

[55] the pharmaceutical or animal drug according to [51] or [52] above, which is an insulin secretion suppressor and/or an agent for increasing blood glucose level and/or an adrenocorticotropic hormone secretion regulator,

[56] the pharmaceutical or animal drug according to [51] or [52] above, which is a prophylactic/therapeutic agent for a disease selected from the group consisting of obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers,

[57] a method of regulating any one selected from the group consisting of sugar/lipid metabolism, pancreatic function, pituitary function, insulin secretion, blood glucose and adrenocorticotropic hormone secretion in a mammal, comprising regulating the expression or activity of RAIG2 and/or RAIG3 in the animal,

[58] a prophylactic/therapeutic method for a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia and memory and learning disorder in a mammal, comprising administering an effective amount of a substance that enhances the expression or activity of RAIG2 and/or a substance that enhances the expression or activity of RAIG3 to the mammal, and

[59] a prophylactic/therapeutic method for a disease selected from the group consisting of obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers in the mammal, comprising administering an effective amount of a substance that inhibits the expression or activity of RAIG2 and/or a substance that inhibits the expression or activity of RAIG3 to a mammal,

and the like.

**Effect of the Invention**

Because RAIG2 and RAIG3 function as receptors of glucose and 1,5-AG to sense blood glucose concentrations and regulate insulin secretion in the pancreas and ACTH secretion in the pituitary, it is possible to screen for a pharmaceutical or animal drug candidate compound that is effective in the regulation of sugar/lipid metabolism and pancreas/pituitary function regulation (for example, regulation of insulin secretion, blood glucose/ACTH secretion and the like), and in the prophylaxis/treatment of a disease involved by an abnormality of sugar/lipid metabolism or pancreas/pituitary function (for example, diabetes mellitus, obesity and the like), by using RAIG2 or RAIG3 (or also with glucose).

**Brief Description of the Drawings**

FIG. 1 is a graph showing insulin secretion at various glucose concentrations in mouse insulinoma MIN6 cells having RAIG3 overexpressed therein. In this figure, ** and *** indicate $p<0.01$ and $p<0.001$, respectively.

FIG. 2 is a graph showing insulin secretion under glucose (2-8 mM and 20 mM) and 2-8 mM glucose + 0.6 mM 1,5-AG conditions in mouse insulinoma MIN6 cells having RAIG2 (a) or RAIG3 (b) overexpressed therein. In this figure, *, **, and *** indicate $p<0.05$, $p<0.01$, and $p<0.001$, respectively.

FIG. 3 is a graph showing CRF-stimulated ACTH secretion at low (2.8 mM) and high (20 mM) glucose concentration conditions in mouse pituitary tumor AtT-20/D16v-F2 cells having siRNA for RAIG2 (or RAIG3) transferred thereto. In this figure, * and *** indicate $p<0.05$ and $p<0.001$, respectively.

FIG. 4 is a graph showing glucose and trehalose concentrations in Hemolympho of the wild type drosophila, a boss-deficient mutant drosophila, and a transgenic drosophila having boss expressed forcibly therein (HRP-Boss). In this figure, the outlined bars, hatched bars and solid bars show the results for glucose, glucose + trehalose, and trehalose, respectively.

**Best Mode for Carrying out the Invention**

The RAIG2 and RAIG3 proteins used in the present invention are proteins comprising the same or substantially the same amino acid sequences as the amino acid sequences shown by SEQ ID NO:2 and SEQ ID NO:4, respectively. RAIG2 and RAIG3 are, for example, the protein of the present invention may be a protein isolated and purified from cells [for example, liver cells, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, adipocytes, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells or interstitial cells, or corresponding precursor cells, stem cells, cancer cells, and the like], or from any tissues where these cells are present [for example, brain, brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, lung, gastrointestinal organs (e.g., large intestine, small intestine), blood vessels, heart, thymus, spleen, submandibular gland, peripheral blood, prostate, testis, ovary, placenta, uterus, bone, joints, adipose tissues (e.g., brown adipose tissue, white adipose tissue), skeletal muscles and the like] of mammals (for example, humans, mice, rats, rabbits, sheep, swine, cattle, horses, cats, dogs, monkeys, chimpanzee and the like) and the like. The protein may also be a chemically synthesized protein or a protein biochemically synthesized using a cell-free translation system, or a recombinant protein produced from a transformant incorporating a nucleic acid having the base sequence encoding the above-described amino acid sequence.

As substantially the same amino acid sequence as the amino acid sequence shown by SEQ ID NO:2 (or SEQ ID NO:4), an amino acid sequence having a homology of about 60% or more, preferably about 70% or more, more preferably about 80% or more, and particularly preferably about 90% or more, to the amino acid shown by SEQ ID NO: 2 (or SEQ ID NO:4). As used herein, "homology" means the proportion (%) of the same and similar amino acid residues to all overlapping amino acid residues in the optimal alignment where two amino acid sequences are aligned using a mathematic algorithm known in the relevant technical field (preferably, the algorithm is such that a gap can be introduced into one or both of the sequences for the optimal alignment). "A similar amino acid" means an amino acid having similar physiochemical properties; as examples, amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxy group (Ser, Thr), and amino acids having a small side chain (Gly, Ala, Ser, Thr, Met), can be mentioned. Substitution by such a similar amino acid is expected to produce no change in the phenotype of protein (i.e., conservative amino acid substitution). Specific examples of conservative amino acid substitution are known in the relevant technical field and described in various pieces of the literature (see, for example, Bowie et al., Science, 247: 1306-1310 (1990)).

Amino acid sequence homology in the present description can be calculated using the homology calculation algorithm NCBI BLAST (National Center for Biotechnology Information Basic Local Alignment Search Tool) under the following conditions (expectancy=10; gap allowed; matrix=BLOSUM62; filtering=OFF).

The other algorithms to determine the homology of an amino acid sequence include, for example, the algorithm described in Karlin et al., Proc. Natl. Acad. Sci. USA, 90: 5873-5877 (1993) [the algorithm is incorporated in the NBLAST and XBLAST programs (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in the GCG software package], the algorithm described in Myers and Miller, CABIOS, 4: 11-17 (1988) [the algorithm is incorporated in the ALIGN program (version 2.0), which is part of the CGC sequence alignment software package], the algorithm described in Pearson et al., Proc. Natl. Acad. Sci. USA, 85: 2444-2448 (1988) [the algorithm is incorporated in the FASTA program in the GCG software package] and the like, which can also be used preferably.
More preferably, substantially the same amino acid sequence as the amino acid sequence shown by SEQ ID NO:2 (or SEQ ID NO: 4) is an amino acid sequence having a homology of about 60% or more, preferably about 70% or more, more preferably about 80% or more, and particularly preferably about 90% or more, to the amino acid sequence shown by SEQ ID NO:2 (or SEO ID NO:4).

The protein comprising substantially the same amino acid sequence as the amino acid sequence shown by SEQ ID NO:2 (or SEQ ID NO:4) is preferably, for example, a protein comprising the same or substantially the same amino acid sequence as the aforementioned amino acid sequence shown by SEQ ID NO:2 (or SEQ ID NO:4), and having substantially the same quality of activity as a protein comprising the amino acid sequence shown by SEQ ID NO: 2 (or SEQ ID NO:4), or the like.

Examples of substantially the same quality of activity include ligand (i.e., glucose or 1,5-AG) binding activity, regulatory activity on secretion of hormones such as insulin and ACTH, and the like. "Substantially the same quality" means that the activities are qualitatively (for example, physiologically or pharmacologically) equivalent to each other. Therefore, it is preferable that the activities such as ligand binding activity and hormone secretion regulatory activity be equivalent to each other (for example, about 0.5 to 2 times), but quantitative factors such as the extent of activity and the molecular weight of the protein may be different.
A measurement of ligand binding activity or hormone secretion regulatory activity can be performed by a method known per se, for example, a method used in the agonist or antagonist screening method described below and the like.

Examples of the RAIG2 (or RAIG3) of the present invention also include proteins comprising (1) an amino acid sequence having 1 or 2 or more (preferably about 1 to 100, preferably about 1 to 50, more preferably about 1 to 10, particularly preferably 1 to several (2, 3, 4 or 5)) amino acids deleted from the amino acid sequence shown by SEQ ID NO:2 (or SEQ ID NO:4), (2) an amino acid sequence having 1 or 2 or more (preferably about 1 to 100, preferably about 1 to 50, more preferably about 1 to 10, particularly preferably 1 to several (2, 3, 4 or 5)) amino acids added to the amino acid sequence shown by SEQ ID NO:2 (or SEQ ID NO:4), (3) an amino acid sequence having 1 or 2 or more (preferably about 1 to 50, preferably about 1 to 10, more preferably 1 to several (2, 3, 4 or 5)) amino acids inserted to the amino acid sequence shown by SEQ ID NO:2 (or SEQ ID NO:4), (4) an amino acid sequence having 1 or 2 or more (preferably about 1 to 50, preferably about 1 to 10, more preferably 1 to several (2, 3, 4 or 5)) amino acids substituted by other amino acids in the amino acid sequence shown by SEQ ID NO:2 (or SEQ ID NO:4), or (5) an amino acid sequence being a combination thereof, and the like.
When an amino acid sequence is inserted, deleted or substituted as described above, the position of the insertion, deletion or substitution is not subject to limitation, as long as the protein activities are retained.

The RAIG2 of the present invention is preferably the human RAIG2 protein having the amino acid sequence shown by SEQ ID NO:2 (GenBank Accession Number:NP_057319), or a homologue thereof in another mammal [for example, mouse, rat and crab-eating macaque homologues registered with GenBank under Accession Numbers NP_071865, XP_215095 and BAE89480, respectively (having a homology of about 85%, about 85% and 99.8%, respectively, to human RAIG2) and the like].
The RAIG3 of the present invention is preferably the human RAIG3 protein having the amino acid sequence shown by SEQ ID NO:4 (GenBank Accession Number:NP_061123), or a homologue thereof in another mammal [for example, mouse, rat and crab-eating macaque homologues registered with GenBank under Accession Numbers NP_671750, XP_213518 and BAE89439, respectively (having a homology of about 89%, about 90% and about 95%, respectively, to human RAIG2) and the like].

In the present specification, proteins and peptides are denoted with the N-terminus (amino terminus) described as the left end and the C-terminus (carboxyl terminus) as the right end, in accordance with the common way of describing peptides. The RAIG2 (or RAIG3) of the present invention, including a protein comprising the amino acid sequence shown by SEQ ID NO: 2, may have any of a carboxyl group (-COOH), a carboxylate ($-COO^-$), an amide ($-CONH_2$) or an ester (-COOR) at the C-terminus thereof.
As used herein, R in the ester is exemplified by $C_{1-6}$ alkyl groups, for example, as methyl, ethyl, n-propyl, isopropyl, n-butyl and the like; $C_{3-8}$ cycloalkyl groups, for example, cyclopentyl, cyclohexyl and the like; $C_{6-12}$ aryl groups, for example, phenyl, $\alpha$-naphthyl and the like; phenyl-$C_{1-2}$ alkyl groups, for example, benzyl, phenethyl and the like; $C_{7-14}$ aralkyl groups such as $\alpha$-naphthyl-$C_{1-2}$-alkyl groups such as $\alpha$-naphthylmethyl; pivaloyloxymethyl groups; and the like.
When the RAIG2 (or RAIG3) of the present invention has a carboxyl group (or carboxylate) at a position other than the C-terminus, the carboxyl group may be amidated or esterified, and such proteins are also included in the protein of the present invention. As examples of the ester used in this case, the above-described esters at the C-terminus can be mentioned.

Furthermore, the protein of the present invention also includes those wherein the amino group of the amino acid residue at the N-terminus is protected with a protecting group (for example, $C_{1-6}$ acyl groups such as $C_{1-6}$ alkanoyl groups such as formyl group and acetyl group); those wherein the glutamyl group at the N-terminus, which can be produced upon cleavage in vivo, is pyroglutaminated; those wherein a substituent (for example, - OH, -SH, amino group, imidazole group, indole group, guanidino group and the like) on the side chain of an amino acid in the molecule is protected with an appropriate protecting group (for example, $C_{1-6}$ acyl groups such as $C_{1-6}$ alkanoyl groups such as formyl group and acetyl group, and the like), conjugated proteins such as what are called glycoproteins having sugar chains bound thereto, and the like.

A partial peptide of RAIG2 (or RAIG3) (hereinafter sometimes simply abbreviated as "the partial peptide of the present invention") may be any peptide having the above-described partial amino acid sequence of RAIG2 (or RAIG3), and having substantially the same quality of activity to that of RAIG2 (or RAIG3). As used herein, "substantially the same quality of activity" has the same meaning as described above. A measurement of "substantially the same quality of activity" can be performed in the same manner as RAIG2 (or RAIG3).

Specifically, as the partial peptide of the present invention, for example, one having a partial amino acid sequence comprising a region involved in the binding with the ligand glucose or 1,5-AG (for example, in the case of human RAIG2, for example, extracellular regions of Amino Acid Nos. 28-56, 116-126, 184-196 and 255-271 in the amino acid sequence shown by SEQ ID NO:2 and the like) and a region involved in signal transduction via an interaction with the ligand (for example, a region having an activity to bind to the $\alpha$ subunit of trimer G protein to promote the GDP/GTP exchange reaction thereof (hereinafter also referred to as "a G$\alpha$ activation domain") and the like) in the amino acid sequence shown by SEQ ID NO:2 (or SEQ ID NO:4), and the like are used. The partial peptide of the present invention is not particularly limited with respect to the size thereof, as long as it comprises the above-described region involved in the binding to the ligand and the region involved in signal transduction; preferably, the partial peptide of the present invention comprises 100 or more partial amino acid sequences, more preferably comprises 200 or more partial amino acid sequences. Each of the partial amino acid sequences may be a single continuous partial amino acid sequence, or a plurality of incontinual partial amino acid sequences.

Meanwhile, peptides that comprise a partial amino acid sequence of RAIG2 (or RAIG3), but that do not have substantially the same quality of activity as RAIG2 (or RAIG3), for example. those having a partial amino acid sequence that comprises a region involved in the binding to the ligand, but that does not comprise a region involved in signal transduction, in the amino acid sequence shown by SEQ ID NO:2 (or SEQ ID NO:4), and the like are not included in "the partial peptide of the present invention". However, because such peptides are capable of blocking the signal transduction action via RAIG2 (or RAIG3) by binding to the ligand, the same can be useful when suppression of the signal transduction action is wanted and in other cases.

Also, the partial peptide of the present invention may have any of a carboxyl group (-COOH), a carboxylate ($-COO^-$), an amide ($-CONH_2$), or an ester (-COOR) at the C-terminus thereof. As used herein, R in the ester is exemplified by the same examples as those mentioned with respect to RAIG2 (or RAIG3). When the partial peptide of the present invention has a carboxyl group (or carboxylate) at a position other than the C-terminus, the carboxyl group may be amidated or esterified, and such partial peptides are also included in the partial peptide of the present invention. In this case, the ester is exemplified by the same examples as those mentioned with respect to the ester at the C-terminus.

Furthermore, the partial peptide of the present invention, like the above-described RAIG2 (or RAIG3), also includes those wherein the amino group of the amino acid residue at the N-terminus is protected with a protecting group, those wherein the glutamine residue at the N-terminus is pyroglutaminated; those wherein a substituent on the side chain of an amino acid in the molecule is protected with an appropriate protecting group, conjugated peptides such as what are called glycopeptides having sugar chains bound thereto, and the like.

As used in the present invention, RAIG2 (or RAIG3) or a partial peptide thereof may be in the form of a salt. For example, salts with physiologically acceptable acids (e.g., inorganic. acids, organic acids) or bases (e.g., alkali metal salts) and the like are used, and physiologically acceptable acid addition salts are particularly preferable. Examples of such salts include salts with inorganic acids (for example, hydrochloric acid, phosphoric acid, hydrobromic acid, sulfuric acid), salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid), and the like.

RAIG2 (or RAIG3) can be produced from cells or tissues of mammals described above by a method of protein purification known per se. Specifically, RAIG2 (or RAIG3) can be prepared by homogenizing a tissue or cells of a mammal, removing the cell debris by low-speed centrifugation, centrifuging the supernatant at high speed to precipitate the cell membrane-containing fraction (if necessary, the cell membrane fraction is purified by density gradient centrifugation and the like), and subjecting the fraction to a chromatography such as reverse phase chromatography, ion exchange chromatography, or affinity chromatography, and the like.

RAIG2 (or RAIG3) or a partial peptide thereof (hereinafter, in the explanation for the chemical synthesis thereof, these are comprehensively simply referred to as "RAIG2 (or RAIG3)" unless otherwise specified) can also be produced by a publicly known method of peptide synthesis.

The method of peptide synthesis may, for example, be any of solid phase synthesis and liquid phase synthesis. The desired protein can be produced by condensing a partial peptide or amino acids that can constitute RAIG2 (or RAIG3) and the remaining portion, and, when the product has a protecting group, removing the protecting group.

Condensation and protecting group removal can be achieved by methods known per se, for example, the methods described in (1) to (5) below.

(1) M. Bodanszky and M.A. Ondetti: Peptide Synthesis, Interscience Publishers, New York (1966)
(2) Schroeder and Luebke: The Peptide, Academic Press, New York (1965)
(3) Nobuo Izumiya et al.: Peptide Gosei-no-Kiso to Jikken (Basics and experiments of peptide synthesis), Maruzen Co. (1975)
(4) Haruaki Yajima and Shunpei Sakakibara: Seikagaku Jikken Koza (Biochemical Experiment) 1, Tanpakushitsu no Kagaku (Chemistry of Proteins) IV, 205 (1977)
(5) Haruaki Yajima ed.: Zoku Iyakuhin no Kaihatsu (A sequel to Development of Pharmaceuticals), Vol. 14, Peptide Synthesis, Hirokawa Shoten

The RAIG2 (or RAIG3) thus obtained can be purified and isolated using a known method of purification. Here, as examples of the method of purification, solvent extraction, distillation, column chromatography, liquid chromatography, recrystallization, combinations thereof and the like can be mentioned.

When the RAIG2 (or RAIG3) obtained by the above-described method is the free form, the free form can be converted into an appropriate salt by a known method or a method based thereon; conversely, when the RAIG2 (or RAIG3) is obtained in the form of a salt, the salt can be converted into the free form or another salt by a known method or a method based thereon.

To synthesize RAIG2 (or RAIG3), ordinary commercially available resins for protein synthesis may be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin and the like can be mentioned. Using such a resins, amino acids having $\alpha$-amino groups and side-chain functional groups protected as appropriate are condensed on the resin in accordance with the sequence of the desired RAIG2 (or RAIG3) according to various methods of condensation methods known per se. At the end of the reaction, the protein and the like are excised from the resin and the various protecting groups are removed simultaneously; then, an intramolecular disulfide bond-forming reaction is performed in a highly diluted solution to give the desired RAIG2 (or RAIG3) or an amide thereof.

For the above-described condensation of protected amino acids, various activation reagents for protein synthesis may be used, with preference given to carbodiimides. Useful carbodiimides include DCC, N,N'-diisopropylcarbodiimide, N-ethyl-N'-(3-dimethylaminopropyl)carbodiimide and the like. For activation with these reagents, protected amino acids, along with a racemization inhibitor (for example, HOBt, HOOBt), may be added directly to the resin, or may be added to the resin after being previously activated in the form of a symmetric acid anhydride, HOBt ester, or HOOBt ester.

Solvents used in the activation of protected amino acids or condensation with the resin can be selected as appropriate from among solvents known to be usable for protein condensation reactions. For example, acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethylsulfoxide; amines such as pyridine; ethers such as dioxane and tetrahydrofuran; nitriles such as acetonitrile and propionitrile; esters such as methyl acetate and ethyl acetate; appropriate mixtures of these solvents, and the like can be used. Reaction temperature is selected as appropriate from the range known to be useful for peptide binding reactions, and is normally selected as appropriate from the range of about -20°C to 50°C. The activated amino acid derivative is normally used in an excess of 1.5 to 4 times. If a test using the ninhydrin reaction reveals insufficient condensation, the condensation can be completed by repeating the condensation reaction without splitting off the protecting groups. If the condensation is yet insufficient even after repeating the reaction, unreacted amino acids can be acetylated with acetic anhydride or acetylimidazole.

Protection and protecting groups for the functional groups that should not involve the reaction of the starting materials, splitting off the protecting groups, activation of the functional groups involved in the reaction, and the like can be selected as appropriate from among known groups or known means.

Examples of the protecting groups for the amino groups of the starting materials include Z, Boc, tertiary pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc and the like.
A carboxyl group can be protected by, for example, alkyl esterification (for example, esterification with a linear, branched or cyclic alkyl such as methyl, ethyl, propyl, butyl, tertiary butyl, cyclopentyl, cyclohexyl, cycloheptyl, cyclooctyl, or 2-adamantyl), aralkyl esterification (for example, benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, tertiary butoxycarbonyl hydrazidation, trityl hydrazidation and the like.
The hydroxyl group of serine can be protected by, for example, esterification or etherification. Examples of qroups suitable for this esterification include lower alkanoyl groups such as acetyl group, aroyl groups such as benzoyl group, groups derived from carbonic acid, such as benzyloxycarbonyl group and ethoxycarbonyl group, and the like. As examples of groups suitable for the etherification, benzyl group, tetrahydropyranyl group, t-butyl group and the like can be mentioned.
Examples of the protecting group for the phenolic hydroxyl group of tyrosine include Bzl, $Cl_2$-Bzl, 2-nitrobenzyl, Br-Z, tertiary butyl and the like.
Examples of the protecting group for the imidazole moiety of histidine include Tos, 4-methoxy-2,3,6-trimethylbenzenesulfonyl, DNP, benzyloxymethyl, Bum, Boc, Trt, Fmoc and the like.

As examples of the method used to remove (split off) the protecting group, catalytic reduction in a hydrogen gas stream in the presence of a catalyst such as Pd-black or Pd-carbon; acid treatment with anhydrous hydrogen fluoride, methanesulfonic acid, trifluoromethanesulfonic acid or trifluoroacetic acid, or a mixed solution thereof; treatment with a base such as diisopropylethylamine, triethylamine, piperidine or piperazine; reduction with sodium in liquid ammonia, and the like can be mentioned. The reaction of splitting of the protecting group by the above-described acid treatment is normally performed at a temperature of about -20°C to 40°C; in the acid treatment, addition of a cation scavenger such as anisole, phenol, thioanisole, meta-cresol, para-cresol, dimethylsulfide, 1,4-butanedithiol or 1,2-ethanedithiol is effective. The 2,4-dinitrophenyl group used as the protecting group for the imidazole moiety of histidine is removed by thiophenol treatment; the formyl group used as the protecting group for the indole moiety of tryptophan is removed by alkali treatment with dilute sodium hydroxide solution, dilute ammonia or the like, as well as by the above-described acid treatment in the presence of 1,2-ethanedithiol, 1,4-butanedithiol or the like.

Examples of activated carboxyl groups in the starting material include corresponding acid anhydrides, azides, activated esters (esters with alcohols (for example, pentachlorophenol, 2,4,5-trichlorophenol, 2,4-dinitrophenol, cyanomethyl alcohol, para-nitrophenol, HONB, N-hydroxysuccimide, N-hydroxyphthalimide, HOBt)]. Examples of activated amino groups in the starting material include corresponding phosphoric amides.

In another method of obtaining an amide of the RAIG2 (or RAIG3), for example, the $\alpha$-carboxyl group of the carboxy-terminal amino acid is first protected by amidation, and the peptide chain on the amino group side is then extended to a desired length; thereafter, a protein (peptide) having only the protecting group for the N-terminal $\alpha$-amino group in the peptide chain removed and a protein (peptide) having only the protecting group for the C-terminal carboxyl group removed are prepared, and the two proteins (peptides) are condensed in a mixed solvent as described above. Details of the condensation reaction are the same as those described above. After the protected protein (protected peptide) obtained by the condensation is purified, all the protecting groups are removed by the above-described method to give the desired crude protein (crude peptide). This crude protein (crude peptide) may be purified by various known means of purification, and the major fraction may be lyophilized to give an amide of the desired RAIG2 (or RAIG3).
An ester of the RAIG2 (or RAIG3) can be obtained by, for example, condensing the $\alpha$-carboxyl group of the carboxy-terminal amino acid with a desired alcohol to prepare an amino acid ester, and then following the same procedures as those for the above-described amide of the RAIG2 (or RAIG3).

The partial peptide of the present invention can also be produced by cleaving the RAIG2 (or RAIG3) full-length protein with an appropriate peptidase.

Furthermore, RAIG2 (or RAIG3) can also be produced by culturing a transformant comprising a nucleic acid encoding the same, and separating and purifying the RAIG2 (or RAIG3) from the culture obtained. The nucleic acid encoding RAIG2 (or RAIG3) or a partial peptide thereof may be a DNA or RNA, or may be a DNA/RNA chimera. The nucleic acid is preferably a DNA. Also, the nucleic acid may be double stranded or single stranded. When the nucleic acid is double stranded, it may be a double-stranded DNA, a double-stranded RNA or a DNA:RNA hybrid. When the nucleic acid is single stranded, it may be a sense strand (i.e., code strand) or an antisense strand (i.e., non-code strand).
As the DNA encoding RAIG2 (or RAIG3) or a partial peptide thereof, genomic DNAs, genomic DNA libraries, cDNAs derived from any cells [for example, liver cells, splenocytes, nerve cells, glial cells, $\beta$ cells of pancreas, bone marrow cells, mesangial cells, Langerhans' cells, epidermic cells, epithelial cells, goblet cells, endothelial cells, smooth muscle cells, fibroblasts, fibrocytes, myocytes, adipocytes, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells or interstitial cells, or corresponding precursor cells, stem cells, cancer cells, and the like], or any tissues where such cells are present [for example, brain, brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, lung, gastrointestinal organs (e.g., large intestine, small intestine), blood vessels, heart, thymus, spleen, submandibular gland, peripheral blood, prostate, testis, ovary, placenta, uterus, bone, joints, adipose tissues (e.g., brown adipose tissue, white adipose tissue), skeletal muscles and the like] of mammals (for example, humans, cattle, monkeys, horses, swine, sheep, goat, dogs, cats, guinea pigs, rats, mice, rabbits, hamsters and the like), synthetic DNAs and the like can be mentioned. Genomic DNA and cDNA that encode RAIG2 (or RAIG3) or a partial peptide thereof can also be amplified directly by Polymerase Chain Reaction (hereinafter abbreviated as "the PCR method") and Reverse Transcriptase-PCR (hereinafter abbreviated as "the RT-PCR method") using a genomic DNA fraction and total RNA or mRNA fraction prepared from the above-described cell or tissue as the template, respectively. Alternatively, a genomic DNA and cDNA that encodes RAIG2 (or RAIG3) or a partial peptide thereof can also be cloned from a genomic DNA library and cDNA library prepared by inserting a genomic DNA and a fragment of total RNA or mRNA, prepared from the above-described cell or tissue, to an appropriate vector, by the colony or plaque hybridization method or the PCR method and the like, respectively. The vector used for the library may be any of bacteriophage, plasmid, cosmid, phagemid and the like.

Examples of the DNA that encodes RAIG2 (or RAIG3) include a DNA comprising the base sequence shown by SEQ ID NO:1 (or SEQ ID NO:3), a DNA that comprises a base sequence that hybridizes with a complementary strand sequence of the base sequence shown by SEQ ID NO:1 (or SEQ ID NO:3) under stringent conditions, and that encodes a protein having substantially the same quality of activity as RAIG2 (or RAIG3) described above (e.g., glucose or 1,5-AG binding activity, regulatory activity on secretion of hormones such as insulin and ACTH and the like) and the like.

As examples of the DNA capable of hybridizing with a complementary strand sequence of the base sequence shown by SEQ ID NO:1 (or SEQ ID NO:3) under stringent conditions, a DNA comprising a base sequence having a homology of about 60% or more, preferably about 70% or more, more preferably about 80% or more, particularly preferably about 90% or more, to the base sequence shown by SEQ ID NO:1 (or SEQ ID NO:3) and the like are used.

Base sequence homology in the present description can be calculated using the homology calculation algorithm NCBI BLAST (National Center for Biotechnology Information Basic Local Alignment Search Tool) under the following conditions (expectancy=10; gap allowed; filtering=ON; match score=1; mismatch score=-3). As preferable examples of other algorithms for determining base sequence homology, the above-described amino acid sequence homology calculation algorithm can be mentioned.

The hybridization can be performed by a method known per se or a method based thereon, for example, the method described in Molecular Cloning, 2nd edition (J. Sambrook et al., Cold Spring Harbor Lab. Press, 1989) and the like. When a commercially available library is used, the hybridization can be performed according to the method described in the instruction manual attached. The hybridization can be performed preferably under highly stringent conditions.

Examples of stringent conditions include those wherein a hybridization reaction at 45°C in 6×SSC (sodium chloride/sodium citrate) is followed by one time or more of washing at 65°C in 0.2×SSC/0.1% SDS and the like. Those skilled in the art are able to easily obtain desired stringency by changing the salt concentration of the hybridization solution, hybridization reaction temperature, probe concentration, probe length, the number of mismatches, hybridization reaction time, the salt concentration of the washing solution, washing temperature and the like as appropriate.

The DNA that encodes RAIG2 is preferably a DNA comprising a base sequence that encodes the human RAIG2 protein of the base sequence shown by the SEQ ID NO:1 (GenBank Accession Number:NM_016235), or a homologue thereof in another mammal [for example, mouse, rat and crab-eating macaque homologues registered with GenBank under Accession Numbers NM_022420, XM_215095 and AB172418, respectively (having a homology of about 85%, about 85% and about 98%, respectively, to human RAIG2 cDNA) and the like].

The DNA that encodes RAIG3 is preferably a DNA comprising a base sequence that encodes the human RAIG3 protein of the base sequence shown by SEQ ID NO:3 [GenBank Accession Number:NM_018653], or a homologue thereof in another mammal (for example, mouse, rat and crab-eating macaque homologues registered with GenBank under Accession Numbers NM_147217, XM_213518 and AB172377, respectively (having a homology of about 87%, about 86% and about 88%, respectively, to human RAIG3 cDNA) and the like).

The DNA that encodes the partial peptide of the present invention may be any DNA, as long as it comprises a base sequence that encodes a peptide having the same or substantially the same amino acid sequence as a portion of the amino acid sequence shown by SEQ ID NO:2 (or SEQ ID NO:4). Specifically, as examples of the DNA that encodes the partial peptide of the present invention, (1) a DNA having a partial base sequence of the base sequence shown by SEQ ID NO:1 (or SEQ ID NO:3), (2) a DNA that has a base sequence that hybridizes with a DNA having the base sequence shown by SEQ ID NO:1 (or SEQ ID NO:3) under stringent conditions, and that encodes a peptide having substantially the same quality of activity as RAIG2 (or RAIG3) described above (e.g., glucose or 1,5-AG binding activity, regulatory activity on secretion of hormones such as insulin and ACTH and the like) and the like are used.

The DNA encoding RAIG2 (or RAIG3) or a partial peptide thereof can be cloned by amplification by the PCR method using a synthetic DNA primer having a portion of the base sequence encoding the RAIG2 (or RAIG3) or a partial peptide thereof, or by hybridization of the DNA incorporated in an appropriate expression vector with a labeled DNA fragment or synthetic DNA encoding a portion or whole of the RAIG2 (or RAIG3) protein. The hybridization can be performed according to, for example, the method described in Molecular Cloning, 2nd edition (ibidem) and the like. When a commercially available library is used, the hybridization can be performed according to the method described in the instruction manual attached to the library.

The base sequence of the DNA can be converted by a method known per se such as the ODA-LA PCR method, the Gapped duplex method, or the Kunkel method, or a method based thereon, using a known kit, for example, Mutan™-super Express Km (Takara Shuzo Co., Ltd.), Mutan™-K (Takara Shuzo Co., Ltd.) and the like.

The cloned DNA can be used as is or, if desired, after digestion with a restriction enzyme or addition of a linker, depending on the purpose of use. The DNA may have ATG as a translation initiation codon at the 5' end thereof, and may have TAA, TGA or TAG as a translation termination codon at the 3' end thereof. These translation initiation and termination codons may be added using an appropriate synthetic DNA adapter.

An expression vector comprising the DNA encoding RAIG2 (or RAIG3) or a partial peptide thereof can be produced by, for example, excising the desired DNA fragment from the DNA encoding RAIG2 (or RAIG3), and joining the DNA fragment downstream of a promoter in an appropriate expression vector.
As the expression vector, plasmids derived from *Escherichia coli* (e.g., pBR322, pBR325, pUC12, pUC13); plasmids derived from *Bacillus subtilis* (e.g., pUB110, pTP5, pC194); plasmids derived from yeast (e.g., pSH19, pSH15); insect cell expression plasmids (e.g., pFast-Bac); animal cell expression plasmids (e.g., pA1-11, pXT1, pRc/CMV, pRc/RSV, pcDNAI/Neo); bacteriophages such as λ phage; insect virus vectors such as baculovirus (e.g., BmNPV, AcNPV); animal virus vectors such as retrovirus, vaccinia virus, and adenovirus, and the like can be used.
The promoter may be any promoter that matches well with the host used for gene expression.
For example, when the host is an animal cell, the SRα promoter, the SV40 promoter, the LTR promoter, the CMV (cytomegalovirus) promoter, the RSV (Rous sarcoma virus) promoter, the MoMuLV (Moloney mouse leukemia virus) LTR, the HSV-TK (herpes simplex virus thymidine kinase) promoter and the like can be used. In particular, the CMV promoter, the SRα promoter and the like are preferable.
When the host is a bacterium of the genus *Escherichia,* the trp promoter, the lac promoter, the recA promoter, the $\lambda P_L$ promoter, the lpp promoter, the T7 promoter and the like are preferable.
When the host is a bacterium of the genus *Bacillus,* the SPO1 promoter, the SPO2 promoter, the penP promoter and the like are preferable.
When the host is a yeast, the PHO5 promoter, the PGK promoter, the GAP promoter, the ADH promoter and the like are preferable.
When the host is an insect cell, the polyhedrin promoter, the P10 promoter and the like are preferable.

As the expression vector, one optionally comprising an enhancer, a splicing signal, a poly A-addition signal, a selection marker, an SV40 replication origin (hereinafter sometimes abbreviated as SV40 ori) and the like, in addition to the above-described examples, can be used. As examples of the selection marker, the dihydrofolate reductase gene (hereinafter sometimes abbreviated as dhfr, methotrexate (MTX) resistance), the ampicillin resistance gene (hereinafter sometimes abbreviated as $amp^r$), the neomycin resistance gene (hereinafter sometimes abbreviated as $neo^r$, G418 resistance) and the like can be mentioned. In particular, when Chinese hamster cells lacking the dhfr gene are used in combination with the dhfr gene as the selection marker, it is also possible to select the desired gene on a thymidine-free medium.
Also, a base sequence encoding a signal sequence (signal codon) that matches with the host may be added as necessary to the 5' end side of the DNA encoding RAIG2 (or 'RAIG3) or a partial peptide thereof (or substituted with a native signal codon). For example, when the host is a bacterium of the genus *Escherichia,* a PhoA signal sequence, a OmpA signal sequence and the like can be used; when the host is a bacterium of the genus *Bacillus,* an α-amylase signal sequence, a subtilisin signal sequence and the like can be used; when the host is a yeast, an MFα signal sequence, an SUC2 signal sequence and the like can be used; when the host is an animal cell, an insulin signal sequence, an α-interferon signal sequence, an antibody molecule signal sequence and the like can be used.

By transforming the host with the above-described expression vector comprising the DNA encoding RAIG2 (or RAIG3) or a partial peptide thereof, and culturing the transformant obtained, RAIG2 (or RAIG3) or a partial peptide thereof can be produced.
As examples of the host used, bacteria of the genus *Escherichia,* bacteria of the genus *Bacillus,* yeasts, insect cells, insects, animal cells and the like can be mentioned.
As examples of the bacteria of the genus *Escherichia, Escherichia coli* K12.DH1 [Proceedings of the National Academy of Sciences of the USA (Proc. Natl. Acad. Sci. USA), Vol. 60, 160 (1968)], *Escherichia coli* JM103 [Nucleic Acids Research, Vol. 9, 309 (1981)], *Escherichia coli* JA221 [Journal of Molecular Biology, Vol. 120, 517 (1978)], *Escherichia coli* HB101 [Journal of Molecular Biology, Vol. 41, 459 (1969)], *Escherichia coli* C600 [Genetics, Vol. 39, 440 (1954)] and the like can be used.
As examples of the bacteria of the genus *Bacillus, Bacillus subtilis* MI114 [Gene, Vol. 24, 255 (1983)], *Bacillus subtilis* 207-21 [Journal of Biochemistry, Vol. 95, 87 (1984)] and the like can be used.
As examples of the yeasts, *Saccharomyces cerevisiae* AH22, $AH22R^-$, NA87-11A, DKD-5D, and 20B-12, *Schizosaccharomyces pombe* NCYC1913 and NCYC2036, *Pichia pastoris* KM71 and the like can be used.

As the insect cells, an established cell line derived from the cabbage armyworm (*Spodoptera frugiperda* cells; Sf cells), MG1 cells derived from the *Trichoplusia ni* mid-intestine, High Five™ cells derived from *Trichoplusia ni* eggs, cells derived from *Mamestra brassicae,* cells derived from *Estigmena acrea,* and the like can be used when the virus is AcNPV, for example. When the virus is BmNPV, an established cell line derived from *Bombyx mori (Bombyx mori* N cells; BmN cells) and the like can be used as the insect cells. As examples of the Sf cells, Sf9 cells (ATCC CRL1711), Sf21 cells (both types of cells are described in Vaughn, J.L. et al., In Vivo, 13, 213-217 (1977)) and the like can be used. As examples of the insects, *Bombyx mori* larvae and the like can be used [Maeda et al., Nature, Vol. 315, 592 (1985)].

As examples of the animal cells, monkey COS-7 cells, monkey Vero cells, Chinese hamster cells CHO (hereinafter abbreviated as CHO cells), Chinese hamster cells CHO lacking the dhfr gene (hereinafter abbreviated as CHO ($dhfr^-$) cells), mouse L cells, mouse AtT-20 cells, mouse myeloma cells, rat GH3 cells, human FL cells and the like can be used.

Transformation can be performed according to a known method depending on the kind of the host.
Bacteria of the genus *Escherichia* can be transformed according to, for example, the methods described in Proceedings of the National Academy of Sciences of the USA (Proc. Natl. Acad. Sci. U.S.A.), Vol. 69, 2110 (1972), Gene, Vol. 17, 107 (1982) and the like.
Bacteria of the genus *Bacillus* can be transformed according to, for example, the methods described in Molecular & General Genetics, Vol. 168, 111 (1979) and the like.
Yeasts can be transformed according to, for example, the methods described in Methods in Enzymology, Vol. 194, 182-187 (1991), Proceedings of the National Academy of Sciences of the USA (Proc. Natl. Acad. Sci. U.S.A.), Vol. 75, 1929 (1978) and the like.
Insect cells and insects can be transformed according to, for example, the methods described in Bio/Technology, Vol. 6,47-55 (1988) and the like.
Animal cells can be transformed, for example, according to the methods described in Saibo Kogaku (Cell Engineering), extra issue 8, Shin Saibo Kogaku Jikken Protocol (New Cell Engineering Experimental Protocol), 263-267 (1995), published by Shujunsha, and Virology, Vol. 52, 456 (1973).

Cultivation of a transformant can be performed according to a known method depending on the kind of the host. For example, when culturing a transformant whose host is a bacterium of the genus *Escherichia* or *Bacillus,* the medium used for cultivation is preferably a liquid medium. The medium preferably contains substances required for the growth of the transformant, such as carbon sources, nitrogen sources, and inorganic substances. As examples of the carbon sources, glucose, dextrin, soluble starch, sucrose and the like can be mentioned; as examples of the nitrogen sources, inorganic or organic substances such as ammonium salts, nitrate salts, corn steep liquor, peptone, casein, meat extract, soybean cake, and potato extract can be mentioned; as examples of the inorganic substances, calcium chloride, sodium dihydrogenphosphate, magnesium chloride and the like can be mentioned. In addition, yeast extract, vitamins, growth promoting factors and the like may be added to the medium. The pH of the medium is preferably about 5 to about 8.
The medium for cultivation of a transformant whose host is a bacterium of the genus *Escherichia* is preferably, for example, an M9 medium containing glucose and Casamino acids [Miller, Journal of Experiments in Molecular Genetics, 431-433, Cold Spring Harbor Laboratory, New York, 1972]. Chemicals such as 3β-indolylacrylic acid may be added to the medium as necessary to allow the promoter to function efficiently.
Cultivation of a transformant whose host is a bacterium of the genus *Escherichia* is normally performed at about 15°C to about 43°C for about 3 to about 24 hours. The culture may be aerated or agitated as necessary.
Cultivation of a transformant whose host is a bacterium of the genus *Bacillus* is normally performed at about 30°C to about 40°C for about 6 to about 24 hours. The culture may be aerated or agitated as necessary.
As examples of the medium for culturing a transformant whose host is a yeast, Burkholder's minimal medium [Bostian, K.L. et al., Proceedings of the National Academy of Sciences of the USA (Proc. Natl. Acad. Sci. USA), Vol. 77, 4505 (1980)], an SD medium containing 0.5% Casamino acid [Bitter, G.A. et al., Proceedings of the National Academy of Sciences of the USA (Proc. Natl. Acad. Sci. USA), Vol. 81, 5330 (1984)] and the like can be mentioned. The pH of the medium is preferably about 5 to about 8. Cultivation is normally performed at about 20°C to about 35°C for about 24 to about 72 hours. The culture may be aerated or agitated as necessary.
As examples of the medium for culturing a transformant whose host is an insect cell or an insect, Grace's Insect Medium [Grace, T.C.C., Nature, Vol. 195, 788 (1962)] having additives such as 10% inactivated bovine serum added thereto as appropriate, and the like can be used. The pH of the medium is preferably about 6.2 to about 6.4. Cultivation is normally performed at about 27°C for about 3 to about 5 days. The culture may be aerated or agitated as necessary.
As examples of the medium for culturing a transformant whose host is an animal cell, a minimal essential medium (MEM) containing about 5% to about 20% fetal bovine serum [Science, Vol. 122, 501 (1952)], Dulbecco's modified Eagle's medium (DMEM) [Virology, Vol. 8, 396 (1959)], RPMI 1640 medium [The Journal of the American Medical Association, Vol. 199, 519 (1967)], 199 medium [Proceeding of the Society for the Biological Medicine, Vol. 73, 1 (1950)] and the like can be used. The pH of the medium is preferably about 6 to about 8. Cultivation is normally performed at about 30°C to about 40°C for about 15 to about 60 hours. The culture may be aerated or agitated as necessary.

As described above, RAIG2 (or RAIG3) or a partial peptide thereof can be produced in or outside the cells of a transformant.

The RAIG2 (or RAIG3) or a partial peptide thereof can be separated and purified from the culture obtained by culturing the aforementioned transformant according to a method known per se.

For example, when the RAIG2 (or RAIG3) or a partial peptide thereof is extracted from the cytoplasm of cultured cells, a method that comprises suspending the cells collected from the culture by a known method in an appropriate buffer, disrupting the cells by ultrasonication, lysozyme and/or freeze-thawing and the like, and performing centrifugation and filtration, to yield a crude extract of the soluble protein, and the like can be used as appropriate. The buffer may contain a protein denaturant such as urea or guanidine hydrochloride and a surfactant such as Triton X-100™. On the other hand, when the RAIG2 (or RAIG3) or a partial peptide thereof is extracted from a membrane fraction, a method that comprises disrupting cells in the same manner as described above, performing low-speed centrifugation to precipitate and remove cell debris, and centrifuging the supernatant at a high speed to precipitate a fraction containing the cell membrane (the cell membrane fraction is purified by density gradient centrifugation and the like as necessary) and the like can be used. When the RAIG2 (or RAIG3) or a partial peptide thereof is secreted outside the cells, a method that comprises separating the culture supernatant from the culture by centrifugation, filtration or the like, and the like can be used.

The RAIG2 (or RAIG3) or a partial peptide thereof contained in the soluble fraction, membrane fraction or culture supernatant thus obtained can be isolated and purified according to a method known per se. Such methods include methods based on differences in solubility, such as salting-out and solvent precipitation; methods based mainly on differences in molecular weight, such as dialysis, ultrafiltration, gel filtration, and SDS-polyacrylamide gel electrophoresis; methods based on differences in electric charge, such as ion exchange chromatography; methods based on specific affinity, such as affinity chromatography; methods based on differences in hydrophobicity, such as reverse phase high performance liquid chromatography; methods based on differences in isoelectric point, such as isoelectric focusing; and the like. These methods may be combined as appropriate.

When the protein or peptide thus obtained is in the free form, the free form can be converted into a salt by a method known per se or a method based thereon; when the protein or peptide is obtained in the form of a salt, the salt can be converted into the free form or another salt by a method known per se or a method based thereon.

The RAIG2 (or RAIG3) or a partial peptide thereof produced by the transformant can be treated with a suitable protein-modifying enzyme before or after the purification, so as to make an optionally chosen modification or to partially remove a polypeptide. As examples of the protein-modifying enzyme used, trypsin, chymotrypsin, arginyl endopeptidase, protein kinase, glycosidase and the like can be mentioned.

The presence of the RAIG2 (or RAIG3) or a partial peptide thereof thus obtained can be confirmed by an enzyme immunoassay, Western blotting and the like using an antibody to RAIG2 (or RAIG3).

Furthermore, RAIG2 (or RAIG3) or a partial peptide thereof can be synthesized in vitro using a cell-free protein translation system comprising rabbit reticulocyte lysate, wheat germ lysate, *Escherichia coli* lysate or the like, with an RNA corresponding to the DNA encoding the above-described RAIG2 (or RAIG3) or a partial peptide thereof as the template. Alternatively, RAIG2 (or RAIG3) or a partial peptide thereof can also be synthesized using a cell-free transcription/translation system further comprising RNA polymerase, with the DNA encoding RAIG2 (or RAIG3) or a partial peptide thereof as the template. As the cell-free protein (transcription/) translation system, a commercially available one can be used, and specifically, an *Escherichia* coli extract can also be prepared by a method known *per se,* such as the method described in Pratt J.M. et al., Transcription and Tranlation, 179-209, Hames B.D. & Higgins S.J. eds., IRL Press, Oxford (1984) and the like. Commercially available cell lysates include those derived from *Escherichia* coli, such as the *E. coli* S30 extract system (manufactured by Promega) and the RTS 500 Rapid Tranlation System (manufactured by Roche), those derived from rabbit reticulocytes, such as the Rabbit Reticulocyte Lysate System (manufactured by Promega), and those derived from wheat germ, such as PROTEIOS™ (manufactured by TOYOBO). Of these, ones using a wheat germ lysate are suitable. Examples of useful methods of producing a wheat germ lysate include the methods described in Johnston F.B. et al., Nature, 179: 160-161 (1957) or Erickson A.H. et al., Meth. Enzymol., 96: 38-50 (1996) and the like.

As the system or apparatus for protein synthesis, the batch method (Pratt, J.M. et al. (1984), mentioned above), the continuous cell-free protein synthesis system (Spirin A.S. et al., Science, 242: 1162-1164 (1988)) wherein amino acids, energy sources and the like are continuously supplied to the reaction system, the dialysis method (Kikawa et al., The 21st Annual Meeting of the Molecule Biology Society of Japan, WID6), or the overlay method (manual of PROTEIOS™ Wheat germ cell-free protein synthesis core kit: manufactured by TOYOBO) and the like can be mentioned. Moreover, a method (JP-A-2000-333673) wherein template RNA, amino acids, energy sources and the like are supplied to a synthesis reaction system as necessary and a synthesized substance and decomposed product are discharged as necessary, and the like can be used.

A nucleic acid comprising the base sequence encoding the RAIG2 (or RAIG3) protein, i.e., a protein comprising the same or substantially the same amino acid sequence as the amino acid sequence shown by SEQ ID NO:2 (or SEQ ID NO:4), or a portion thereof, or a base sequence complementary to the base sequence or a portion thereof, is meant to include not only the above-described nucleic acid encoding SS169 or a partial peptide thereof, but also base sequences comprising a mismatch frame.

A nucleic acid comprising a base sequence complementary to the target region of the desired nucleic acid, i.e., a nucleic acid capable of hybridizing with the desired nucleic acid, can be said to be "antisense" against the desired nucleic acid. On the other hand, a nucleic acid comprising a base sequence having a homology to the target region of the desired nucleic acid can be said to be "sense" against the target nucleic acid. As used herein, "having a homology" or "(being) complementary" means having a homology or complementarity of about 70% or more, preferably about 80% or more, more preferably about 90% or more, and most preferably about 95% or more, between the base sequences.

A nucleic acid comprising a base sequence complementary to the base sequence encoding the RAIG2 (or RAIG3) protein or a portion thereof (hereinafter also referred to as "the antisense nucleic acid of the present invention") can be designed and synthesized on the basis of the base sequence information of a cloned or sequenced RAIG2 (or RAIG3)-encoding nucleic acid. Such nucleic acids are capable of inhibiting the replication or expression of the RAIG2 (or RAIG3) gene. Hence, the antisense nucleic acid of the present invention is capable of hybridizing with the RNA transcribed from the RAIG2 (or RAIG3) gene, and of inhibiting the synthesis (processing) or function (translation into protein) of mRNA.

The target region of the antisense nucleic acid of the present invention is not subject to limitation as to length, as long as hybridization of the antisense nucleic acid results in the inhibition of the translation of the RAIG2 (or RAIG3) protein, and may be the whole sequence or a partial sequence of RAIG2 (or RAIG3) mRNA; for example, about 15 bases for the shortest and the whole sequence of mRNA or the initial transcription product for the longest can be mentioned. Considering the issues of the ease of synthesis and antigenicity, an oligonucleotide comprising about 15 to about 30 bases is preferred, but the length is not limited thereto. Specifically, for example, the 5'-end hairpin loop, 5'-end 6-base-pair repeat, 5'-end untranslated region, polypeptide translation initiation codon, protein coding region, ORF translation initiation codon, 3'-end untranslated region, 3'-end palindrome region, and 3'-end hairpin loop of the nucleic acid encoding the RAIG2 (or RAIG3) precursor polypeptide, may be selected as the target region, but any other region within the RAIG2 (or RAIG3) gene may also be selected as the target. For example, it is also preferable that the intron portion of the gene be the target region.

Furthermore, the antisense nucleic acid of the present invention may be capable of inhibiting RNA transcription by binding with the RAIG2 (or RAIG3) gene, which is a double-stranded DNA, to form a triple strand (triplex, as well as inhibiting translation into protein by hybridizing with RAIG2 (or RAIG3) mRNA or the initial transcription product. Alternatively, the antisense nucleic acid of the present invention may be one that forms a DNA:RNA hybrid to induce degradation by RNaseH.

Examples of the antisense nucleic acid include deoxypolynucleotides comprising 2-deoxy-D-ribose, ribonucleotides comprising D-ribose, other types of nucleotides which are N-glycosides of the purine or pyrimidine base, or other polymers having a non-nucleotide backbone (for example, commercially available nucleic acid polymers specific for protein nucleic acids and synthetic sequences) or other polymers comprising a special linkage (provided that the polymers comprise nucleotides having such an alignment that allows base pairing or base attachment, as found in DNA or RNA) and the like. These may be double-stranded DNAs; single-stranded DNAs, double-stranded RNAs, single-stranded RNAs, or DNA:RNA hybrids, and may also be unmodified polynucleotides (or unmodified oligonucleotides); those with known modifications, for example, those with labels known in the art, those with caps, those methylated, those with substitution of one or more naturally occurring nucleotides with their analogue, those with intramolecular modifications of nucleotides such as those with uncharged linkages (for example, methyl phosphonates, phosphotriesters, phosphoramidates, carbamates and the like) and those with charged linkages or sulfur-containing linkages (for example, phosphorothioates, phosphorodithioates and the like); those having side chain groups such as proteins (nucleases, nuclease inhibitors, toxins, antibodies, signal peptides, poly-L-lysine and the like) or saccharides (for example, monosaccharides and the like); those with intercalators (for example, acridine, psoralen and the like); those with chelators (for example, metals, radioactive metals, boron, oxidative metals and the like); those with alkylating agents; or those with modified linkages (for example, $\alpha$ anomeric nucleic acids and the like). As used herein, "nucleoside", "nucleotide" and "nucleic acid" may comprise not only the purine and pyrimidine bases, but also other modified heterocyclic bases. Such modified produced may comprise a methylated purine and pyrimidine, acylated purine and pyrimidine, and another heterocyclic ring. The modified nucleotide and modified nucleotide may have a modification in the sugar moiety thereof; for example, one or more hydroxyl groups may be substituted with halogens, aliphatic groups and the like, or may be converted into functional groups such as ethers and amines.

The antisense nucleic acid is an RNA, a DNA, or a modified nucleic acid (RNA, DNA). Specific examples of the modified nucleic acid include, but are not limited to, sulfur and thiophosphate derivatives of nucleic acids and those resistant to degradation of polynucleoside amides and oligonucleoside amides. The antisense nucleic acid of the present invention can be designed preferably on the following plan, that is, to increase the intracellular stability of the antisense nucleic acid, to increase the cell permeability of the antisense nucleic acid, to increase the affinity for the targeted sense strand, and to reduce the toxicity, if any, of the antisense nucleic acid. Many such modifications are known in the art, as disclosed in, for example, J. Kawakami, et al., Pharm. Tech. Japan, Vol. 8, p. 247, 1992; Vol. 8, p. 395, 1992; S. T. Crooke et al. ed., Antisense Research and Applications, CRC Press, 1993; and the like.

The antisense nucleic acid may comprise an altered or modified sugar, base, and bond, and can be supplied in a special form such as liposomes or microspheres, can be applied to gene therapy, and can be given in the form of an adduct. As substances for the adduct, polycations such as polylysine, which acts to neutralize the electric charge of the phosphate group backbone, and hydrophobic substances such as lipids that enhance the interaction with the cell membrane or increase nucleic acid uptake (for example, phospholipids, cholesterols and the like) can be mentioned. As lipids preferred for the adduct, cholesterol and derivatives thereof (for example, cholesteryl chloroformate, cholic acid and the like) can be mentioned. These substances may be attached to the 3' or 5' end of the nucleic acid, and can be attached via a base, sugar, or intramolecular nucleoside bond. As other groups, capping groups specifically placed at the 3' or 5' end of the nucleic acid to prevent its degradation by nucleases such as exonuclease and RNase, can be mentioned. Such capping groups include, but are not limited to, hydroxyl group protecting groups known in the art, including glycols such as polyethylene glycol and tetraethylene glycol.

Ribozymes capable of specifically cleaving RAIG2 (or RAIG3) mRNA or the initial transcription product thereof inside the coding region (comprising the intron moiety in the case of the initial transcription product) can also be included in the antisense nucleic acid of the present invention. "A ribozyme" refers to an RNA having enzyme activity to cleave nucleic acids; since it has recently been shown that oligo-DNAs having the base sequence of the enzyme activity moiety likewise have nucleic acid cleavage activity, this term is used herein as a concept including DNAs, as lonq as they have sequence-specific nucleic acid cleavage activity. The most versatile ribozymes include self- splicing RNAs found in infectious RNAs such as viroid and virusoid, and are known to occur in the hammerhead type, the hairpin type and the like. The hammerhead type exhibits enzyme activity with about 40 bases, and is capable of specifically cleaving only the target mRNA by rendering several bases at each end adjacent to the hammerhead structure moiety (about 10 bases in total) a sequence complementary to the desired cleavage site of mRNA. Because this type of ribozyme utilizes only RNA as the substrate, it offers a further advantage of not attacking genomic DNA. When RAIG2 (or RAIG3) mRNA itself has a double strand structure, the target sequence can be made single-stranded by using a hybrid ribozyme joined with an RNA motif derived from a viral nucleic acid capable of specifically binding to RNA helicase [Proc. Natl. Acad. Sci. USA, 98(10): 5572-5577 (2001)]. Furthermore, when ribozyme is used in the form of an expression vector comprising the DNA encoding the ribozyme, it is also possible to make the ribozyme a hybrid ribozyme further joined with a sequence with tRNA modified to promote the transfer of the transcription product to cytoplasm [Nucleic Acids Res., 29(13): 2780-2788 (2001)].

Herein, a double stranded RNA consisting of an oligo-RNA complementary to a partial sequence in the coding region of the mRNA or initial transcription product of RAIG2 (or RAIG3) (including the intron portion in the case of the initial transcription product) and a strand complementary thereto, what is called siRNA, is defined as being also included in the antisense nucleic acid of the present invention. It had been known that so-called RNA interference (RNAi), the phenomenon in which when a short double-stranded RNA is introduced into cells, an mRNA homologous to the RNA is degraded, occurs in nematodes, insects, plants and the like; since this phenomenon was confirmed to also occur widely in animal cells [Nature, 411(6836): 494-498 (2001)], it has been commonly utilized as an alternative technique to ribozymes. An siRNA can be designed as appropriate on the basis of the base sequence information on the mRNA being the target using commercially available software (e.g., RNAi Designer; Invitrogen).

The antisense oligonucleotide and ribozyme of the present invention can be prepared by determining the target region of mRNA or the initial transcription product thereof on the basis of RAIG2 (or RAIG3) cDNA sequence or genomic DNA sequence information, and synthesizing a sequence complementary thereto using a commercially available automated DNA/RNA synthesizer (Applied Biosystems Company, Beckman Company and the like). siRNA having RNAi activity can be prepared by synthesizing a sense strand and an antisense strand, respectively, using an automated DNA/RNA synthesizer, denaturing them in an appropriate annealing buffer, for example, at about 90°C to about 95°C for about 1 minute, and annealing them at about 30°C to about 70°C for about 1 to about 8 hours. A longer double-stranded polynucleotide can be prepared by synthesizing complementary oligonucleotide strands to overlap alternately, annealing them, and ligating them using ligase. Alternatively, an siRNA can also be designed to be processed by an enzyme dicer and the like in the animal cell to be transfected, by synthesizing an RNA wherein a sense strand and an antisense strand have been joined via a linker with an appropriate length (for example, about 3 to about 10 bases) (shRNA). Furthermore, an expression vector wherein the DNAs that encode a sense strand and an antisense strand are placed under the control of respective Pol III system promoters such as U6 and H1, or an expression vector wherein the DNA that encodes an RNA strand wherein the above-described sense strand and antisense strand have been joined via a linker is placed under the control of a Pol III system promoter, may be prepared and allowed to be expressed in an animal cell to form of an siRNA.

The gene expression inhibitory activity of the antisense nucleic acid of the present invention can be examined using a transformant comprising the nucleic acid encoding RAIG2 (or RAIG3), an in vivo or in vitro RAIG2 (or RAIG3) gene expression system, or an in vivo or in vitro RAIG2 (or RAIG3) protein translation system. The nucleic acid can be applied to cells by various methods known per se.

The present invention also provides an antibody against RAIG2 (or RAIG3). The antibody may be a monoclonal antibody or a polyclonal antibody, as long as it has specific affinity for RAIG2 (or RAIG3). An antibody against RAIG2 (or RAIG3) can be produced using the RAIG2 (or RAIG3) or a partial peptide thereof as the antigen according to a method of antibody or antiserum production known per se.

Hereinafter, a method of preparing an immunogen for the antibody of the present invention, and a method of producing the antibody are described.

(1) Preparation of antigen

As the antigen used to prepare the antibody of the present invention, any of the above-described RAIG2 (or RAIG3) protein or a partial peptide thereof (hereinafter, in the description of antibodies, these are comprehensively simply referred to as "RAIG2 (or RAIG3)" unless otherwise stated), or a (synthetic) peptide having 1 kind or 2 kinds or more of the same antigen determinant thereas and the like, can be used (hereinafter, these are sometimes simply referred to as the antigen of the present invention).

RAIG2 (or RAIG3), as described above, is produced by, for example, (a) preparing the same from a mammalian tissue or cells using a publicly known method or a method based thereon, (b) chemically synthesizing the same by a publicly known method of peptide synthesis using a peptide synthesizer and the like, (c) culturing a transformant containing a DNA that encodes RAIG2 (or RAIG3), or (d) biochemically synthesizing the same using a cell-free transcription/translation system with a nucleic acid that encodes RAIG2 (or RAIG3) as the template.

(a) When RAIG2 (or RAIG3) is prepared from a mammalian tissue or cells, the tissue or cells may be homogenized, and then the crude fraction (e.g., membrane fraction, soluble fraction) can also be used as is as the antigen. Alternatively, the same can also be purified and isolated by performing extraction with an acid, surfactant or alcohol and the like, and applying the extract to a combination of salting-out, dialysis, gel filtration, and chromatographies such as reversed-phase chromatography, ion exchange chromatography, and affinity chromatography. The obtained RAIG2 (or RAIG3) obtained can be used as the immunogen as is, and can also be used as the immunogen in the form of a partial peptide prepared by limited degradation using a peptidase and the like.

(b) When the antigen of the present invention is prepared chemically, examples of the synthetic peptide include one having the same structure as RAIG2 (or RAIG3) purified from a natural material using the method described in (a) above; to be specific, a peptide comprising 1 or 2 or more kinds of the same amino acid sequence as the amino acid sequence at an optionally chosen portion consisting of at least 3 or more, preferably 6 or more, amino acids in the amino acid sequence of RAIG2 (or RAIG3) and the like are used.

(c) When the antigen of the present invention is produced using a transformant containing a DNA, the DNA can be prepared according to a known cloning method [e.g., the method described in Molecular Cloning (2nd ed.; J. Sambrook et al., Cold Spring Harbor Lab. Press, 1989) and the like]. Examples of the cloning method include (i) a method comprising isolating a DNA that encodes the antigen from a human cDNA library by the hybridization method using DNA probes designed on the basis of the gene sequence that encodes RAIG2 (or RAIG3), (2) a method comprising preparing a DNA that encodes the antigen by a PCR method using a DNA primer designed on the basis of the gene sequence that encodes RAIG2 (or RAIG3) with a mammal-derived cDNA as the template, and inserting the DNA into an expression vector compatible with the host and the like. By culturing a transformant obtained by transforming a host with the expression vector in an appropriate medium, a desired antigen can be obtained.

(d) When a cell-free transcription/translation system is utilized, a method for synthesizing an mRNA by using an expression vector incorporating a DNA that encodes the antigen (e.g., an expression vector wherein the DNA is placed under the control of the T7 or SP6 promoter etc., and the like) as the template, that was prepared by the same method as (c) above, a transcription reaction mixture comprising an RNA polymerase matching the promoter, and its substrates (NTPs); and thereafter performing a translation reaction with the mRNA as the template using a known cell-free translation system (e.g., *E. coli,* rabbit reticulocytes, extract from wheat germ etc.), and the like can be mentioned. By adjusting the salt concentration and the like appropriately, the transcription reaction and the translation reaction can also be carried out in the same reaction mixture at one time.

As the immunogen, a whole RAIG2 (or RAIG3) protein molecule or a peptide having a partial amino acid sequence can be used. As examples of the partial amino acid sequence, those comprising 3 or more continuous amino acid residues, preferably those comprising 4 or more, more preferably 5 or more, still more preferably 6 or more continuous amino acid residues, can be mentioned. Alternatively, as examples of the amino acid sequence, those comprising 20 or less continuous amino acid residues, preferably those comprising 18 or less, more preferably 15 or less, still more preferably 12 or less continuous amino acid residues, can be mentioned. A portion of these amino acid residues (e.g., 1 to several residues) may be substituted with a substituent group (e.g., Cys, hydroxyl group, etc.). The peptide used as the immunogen has an amino acid sequence comprising one to several such partial amino acid sequences.

Alternatively, a mammalian cell that expresses RAIG2 (or RAIG3) per se can be used directly as the antigen of the present invention. As the mammalian cell, natural cells as described in the term (a) above, cells transformed by a method as described in the term (c) above and the like can be used. The host used for the transformation may be any cell, as long as it is collected from a human, monkey, rat, mouse, hamster, chicken and the like; HEK293, COS7, CHO-K1, NIH3T3, Balb3T3, FM3A, L929, SP2/0, P3U1, B16, or P388 and the like are preferably used. Natural mammalian cells or transformed warm-blooded animal cells that express RAIG2 (or RAIG3) can be injected to the immunized animal in suspension in a tissue culture medium (e.g.,: RPMI1640) or buffer solution (e.g.,: Hanks' Balanced Salt Solution). The method of immunization may be any method, as long as it is capable of promoting antibody production; intravenous injection, intraperitoneal injection, intramuscular injection or subcutaneous injection and the like are preferably used.

The antigen of the present invention permit direct use for immunization in an insolubilized form, as long as it has immunogenicity; when an antigen of low molecular weight (for example, molecular weight about 3,000 or less) having only one to several antigenic determinants in the molecule thereof (i.e., a partial peptide of RAIG2 (or RAIG3)) is used, it can be used for immunization in the form of a complex bound or adsorbed to a suitable carrier because these antigens are normally hapten molecules of low immunogenicity. As the carrier, a naturally occurring or synthetic polymer can be used. As examples of the naturally occurring polymer, serum albumin of a mammal such as bovine, rabbit, or human, thyroglobulin of a mammal such as bovine or rabbit, ovalbumin of chicken, hemoglobin of a mammal such as bovine, rabbit, human, or sheep, keyhole limpet hemocyanin (KLH) and the like can be used. As examples of the synthetic polymer, various latexes of polymers or copolymers of polyamino acids, polystyrenes, polyacryls, polyvinyls, polypropylenes and the like, and the like can be mentioned.

Regarding the mixing ratio of the carrier and hapten, any combination in any ratio can be bound or adsorbed, as long as an antibody against the antigen bound or adsorbed to the carrier is produced efficiently; usually, one wherein the above-described naturally occurring or synthetic polymer carrier in common use in preparing an antibody against hapten is bound or adsorbed in a ratio by weight of 0.1 to 100 to 1 of hapten can be used.

Various condensing agents can be used for coupling the hapten and carrier. For example, diazonium compounds such as bisdiazotized benzidine, which crosslink tyrosine, histidine, and tryptophan; dialdehyde compounds such as glutaraldehyde, which crosslink amino groups together; diisocyanate compounds such as toluene-2,4-diisocyanate; dimaleimide compounds such as N,N'-o-phenylenedimaleimide, which crosslink thiol groups together; maleimide activated ester compounds, which crosslink amino groups and thiol groups; carbodiimide compounds, which crosslink amino groups and carboxyl groups; and the like can be used advantageously. When amino groups are crosslinked together, it is also possible to react one amino group with an activated ester reagent having a dithiopyridyl group (e.g., N-succinimidyl 3-(2-pyridyldithio)propionate (SPDP) etc.), followed by reduction, to introduce the thiol group, and to introduce a maleimide group into the other amino group using a maleimide activated ester reagent, followed by a reaction of both.

(2) Preparation of monoclonal antibody

(a) Preparation of monoclonal antibody-producing cells

An antigen is administered as is, or along with a carrier or a diluent, to a warm-blooded animal at a site enabling antibody production by the methods such as intraperitoneal injection, intravenous injection, subcutaneous injection, intradermal injection and the like. In order to increase antibody productivity upon the administration, Freund's complete adjuvant or Freund's incomplete adjuvant may be administered. Dosing is normally performed about two to 10 times in total every 1 to 6 weeks. As examples of the warm-blooded animal to be used, monkeys, rabbits, dogs, guinea pigs, mice, rats, sheep, goats, donkeys and chickens can be mentioned. Although it is preferable to use a mammal of the same species as the recipient in order to avoid the problem of anti-Ig antibody production, mice and rats are generally preferably used for generating a monoclonal antibody.

Because artificial immunization to humans is ethically difficult, it is preferable, when the antibody of the present invention targets a human for administration, (i) to obtain a human antibody by immunizing a human antibody-producing animal (e.g., mouse) produced according to a method described below, (ii) to produce a chimeric antibody, humanized antibody or fully human antibody according to a method described below, or (iii) to obtain a human antibody using in combination the *in vitro* immunization method and cell immortalization with virus, human-human (or -mouse) hybridoma production technique, phage display method and the like. Note that the in vitro immunization method can also be used preferably as a method for obtaining an antibody against an antigen that is unstable and difficult to prepare in large amounts for the purpose of preparing a non-human animal-derived antibody, because there is the possibility of obtaining an antibody against an antigen for which antibody production is suppressed by ordinary immunization, because it is possible to obtain an antibody with an amount of antiqen on the nanogram to microgram order, because immunization completes in several days, and for other reasons.

As the animal cells used in the in vitro immunization method, lymphocytes, preferably B-lymphocytes and the like, isolated from peripheral blood, spleen, lymph node and the like of a human and the above-described warm-blooded animals (preferably mouse or rat) can be mentioned. For example, in the case of mouse or rat cells, the spleen is extirpated from an about 4- to 12-week-old animal, and splenocytes are separated and rinsed with a appropriate medium [e.g., Dulbecco's modified Eagle medium (DMEM), RPMI1640 medium, Ham's F12 medium and the like], after which the splenocytes are suspended in an antigen-containing medium supplemented with fetal calf serum (FCS; about 5 to 20%) and cultured using a $CO_2$ incubator and the like for about 4 to 10 days. Examples of the antigen concentration include, but are not limited to, 0.05 to 5 μg. It is preferable to prepare a culture supernatant of thymocytes of an animal of the same strain (preferably at about 1 to 2 weeks of age) according to a conventional method, and to add the supernatant to the medium.

Because it is difficult to obtain a thymocyte culture supernatant in in vitro immunization of human cells, it is preferable to perform immunization by adding, to the medium, several kinds of cytokines such as IL-2, IL-4, IL-5, and IL-6 and the like, and if necessary, an adjuvant substance (e.g., muramyldipeptide and the like) along with the antigen.

In preparing a monoclonal antibody, it is possible to establish an antibody-producing hybridoma by selecting an individual or cell population showing an increased antibody titer from among antigen-immunized warm-blooded animals (e.g., mice, rats) or animal cells (e.g., human, mouse, rat), respectively; collecting spleens or lymph nodes at 2 to 5 days after the final immunization or collecting the cells after 4 to 10 days of cultivation after *in vitro* immunization to isolate antibody-producing cells; and fusing the isolated cells with myeloma cells. A measurement of serum antibody titer can be performed by, for example, reacting a labeled antigen and an antiserum, and thereafter determining the activity of the label bound to the antibody.

Although the myeloma cells are not subject to limitation, as long as they are capable of producing a hybridoma that secretes a large amount of antibody, those that do not produce or secrete the antibody *per se* are preferable, with greater preference given to those of high cell fusion efficiency. To facilitate hybridoma selection, it is preferable to use a cell line that is susceptible to HAT (hypoxanthine, aminopterin, thymidine). As examples of the mouse myeloma cells, NS-1, P3U1, SP2/0, AP-1 and the like can be mentioned; as examples of the rat myeloma cells, R210.RCY3, Y3-Ag 1.2.3 and the like can be mentioned; as examples of the human myeloma cells, SKO-007, GM 1500-6TG-2, LICR-LON-HMy2, UC729-6 and the like can be mentioned.

Fusion operation can be performed according to a known method, for example, the method of Koehler and Milstein [*Nature*, 256, 495 (1975)]. As a fusion promoter, polyethylene glycol (PEG), Sendai virus and the like can be mentioned, and PEG and the like are preferably used. Although the molecular weight of PEG is not subject to limitation, PEG1000 to PEG6000, which are of low toxicity and relatively low viscosity, are preferable. As examples of the PEG concentration, about 10 to 80%, preferably about 30 to 50%, can be mentioned. As the solution for diluting PEG, various buffers such as serum-free medium (e.g., RPMI1640), complete medium comprising about 5 to 20% serum, phosphate buffered saline (PBS), and Tris buffer can be used. DMSO (e.g., about 10 to 20%) can also be added as desired. As examples of the pH of the fusion solution, about 4 to 10, preferably about 6 to 8 can be mentioned.
The ratio by number of antibody-producing cells (splenocytes) and myeloma cells is preferably about 1:1 to 20:1, and the cell fusion can be efficiently performed by incubation normally at 20 to 40°C, preferably at 30 to 37°C, normally for 1 to 10 minutes.

An antibody-producing cell line can also be obtained by infecting antibody-producing cells with a virus capable of transforming lymphocytes to immortalize the cells. As such viruses, for example, Epstein-Barr (EB) virus and the like can be mentioned. Although the majority of persons have immunity because they have ever been infected with this virus in an asymptomatic infection of infectious mononucleosis, virion is also produced when the ordinary EB virus is used; therefore, appropriate purification must be performed. As an EB system free from the possibility of viral contamination, it is also preferable to use a recombinant EB virus that retains the capability of immortalizing B lymphocytes but lacks the capability of replicating virion (for example, deficiency of the switch gene for transition from latent infection state to lytic infection state and the like).

Because marmoset-derived B95-8 cells secrete EB virus, B lymphocytes can be easily transformed by using a culture supernatant thereof. An antibody-producing B cell line can be obtained by, for example, culturing these cells using a medium supplemented with serum and penicillin/streptomycin (P/S) (e.g., RPMI1640) or a serum-free medium supplemented with a cell growth factor, thereafter separating the culture supernatant by filtration or centrifugation and the like, suspending therein antibody-producing B lymphocytes at a suitable concentration (e.g., about $10^7$ cells/mL), and incubating the suspension normally at 20 to 40°C, preferably at 30 to 37°C, normally for about 0.5 to 2 hours. When human antibody-producing cells are provided as mixed lymphocytes, it is preferable to previously remove T lymphocytes by allowing them to form an E rosette with, for example, sheep erythrocytes and the like, to increase transformation frequency of EB virus, because the majority of persons have T lymphocytes which exhibit cytotoxicity to cells infected with EB virus. It is also possible to select lymphocytes specific for the target antigen by mixing sheep erythrocytes, previously bound with a soluble antigen, with antibody-producing B lymphocytes, and separating the rosette using a density gradient of percoll and the like. Furthermore, because antigen-specific B lymphocytes are capped by adding the antigen in large excess so that they no longer present IgG to the surface, mixing with sheep erythrocytes bound with anti-IgG antibody results in the formation of rosette only by antigen-nonspecific B lymphocytes. Therefore, by collecting a layer of cells that don't form rosette from this mixture using a density gradient of percoll and the like, it is possible to select antigen-specific B lymphocytes.

Human antibody-secreting cells having acquired the capability of proliferating indefinitely by the transformation can be back fused with mouse or human myeloma cells in order to stably sustain the antibody-secreting ability. As the myeloma cells, the same as those described above can be used.

Hybridoma screening and breeding are normally performed using a medium for animal cells (e.g., RPMI1640) containing 5 to 20% FCS or a serum-free medium supplemented with cell growth factors, with the addition of HAT (hypoxanthine, aminopterin, thymidine). As examples of the concentrations of hypoxanthine, aminopterin and thymidine, about 0.1 mM, about 0.4 μM and about 0.016 mM and the like, respectively, can be mentioned. For selecting a human-mouse hybridoma, ouabain resistance can be used. Because human cell lines are more susceptible to ouabain than mouse cell lines, it is possible to eliminate unfused human cells by adding ouabain at about $10^{-7}$ to $10^{-3}$ M to the medium.

In selecting a hybridoma, it is preferable to use feeder cells or culture supernatants of certain cells. As the feeder cells, an allogenic cell species having a lifetime limited so that it dies after helping the emergence of hybridoma, cells capable of producing large amounts of a growth factor useful for the emergence of hybridoma with their proliferation potency reduced by irradiation and the like, and the like are used. For example, as the mouse feeder cells, splenocytes, macrophage, blood, thymocytes and the like can be mentioned; as the human feeder cells, peripheral blood mononuclear cells and the like can be mentioned. As examples of the cell culture supernatant, primary culture supernatants of the above-described various cells and culture supernatants of various established cell lines can be mentioned.
Moreover, a hybridoma can also be selected by reacting a fluorescein-labeled antigen with fusion cells, and thereafter separating the cells that bind to the antigen using a fluorescence-activated cell sorter (FACS). In this case, efforts for cloning can be lessened significantly because a hybridoma that produces an antibody against the target antigen can be directly selected.

For cloning a hybridoma that produces a monoclonal antibody against the target antigen, various methods can be used.
It is preferable to remove aminopterin as soon as possible because it inhibits many cell functions. In the case of mice and rats, aminopterin can be removed 2 weeks after fusion and beyond because most myeloma cells die within 10 to 14 days. However, a human hybridoma is normally maintained in a medium supplemented with aminopterin for about 4 to 6 weeks after fusion. It is desirable that hypoxanthine and thymidine be removed more than one week after the removal of aminopterin. That is, in the case of mouse cells, for example, a complete medium (e.g., RPMI1640 supplemented with 10% FCS) supplemented with hypoxanthine and thymidine (HT) is added or exchanged 7 to 10 days after fusion. About 8 to 14 days after fusion, visible clones emerge. Provided that the diameter of clone has reached about 1 mm, the amount of antibody in the culture supernatant can be measured.

A measurement of the amount of antibody can be performed by, for example, a method comprising adding the hybridoma culture supernatant to a solid phase (e.g., microplate) to which the target antigen or a derivative thereof or partial peptide thereof (including the partial amino acid sequence used as the epitope) is adsorbed directly or with a carrier, subsequently adding an anti-immunoglobulin (IgG) antibody (an antibody against IgG derived from an animal of the same species as the animal from which the original antibody-producing cells are derived is used) or protein A, which had been labeled with a radioactive substance (e.g., $^{125}I$, $^{131}I$, $^{3}H$, $^{14}C$), enzyme (e.g., β-galactosidase, β-glucosidase, alkaline phosphatase, peroxidase, malate dehydrogenase), fluorescent substance (e.g., fluorescamine, fluorescein isothiocyanate), luminescent substance (e.g., luminol, luminol derivative, luciferin, lucigenin) and the like, and detecting the antibody against the target antigen (epitope) bound to the solid phase, a method comprising adding the hybridoma culture supernatant to a solid phase to which an anti-IgG antibody or protein A is adsorbed, adding the target antigen, a derivative thereof, or a partial peptide thereof labeled with the same labeling reagent as described above, and detecting the antibody against the target antigen (epitope) bound to the solid phase and the like.

Although limiting dilution is normally used as the cloning method, cloning using soft agar and cloning using FACS (described above) are also possible. Cloning by limiting dilution can be performed by, for example, the following procedures, which, however, are not to be construed as limiting.
The amount of antibody is measured as described above, and positive wells are selected. Selected suitable feeder cells are previously added to a well plate. Cells are collected from the antibody-positive wells and suspended in complete medium (e.g., RMPI1640 supplemented with 10% FCS and P/S) to obtain a density of 30 cells/mL; 0.1 mL (3 cells/well) of this suspension is added to the 96-well plate with feeder cells added thereto; a portion of the remaining cell suspension is diluted to 10 cells/mL and sown to other wells (1 cell/well)in the same way; the still remaining cell suspension is diluted to 3 cells/mL and sown to other wells (0.3 cells/well). The cells are cultured for about 2 to 3 weeks until a visible clone appears, when the amount of antibody is measured to select positive wells, and the selected cells are recloned in the same way. In the case of human cells, cloning is relatively difficult, so that a plate in which cells are seeded at 10 cells/well is also prepared. Although a monoclonal antibody-producing hybridoma can be obtained normally by two times of subcloning, it is desirable to repeat recloning regularly for several more months to confirm the stability thereof.

Hybridomas can be cultured *in vitro* or in vivo.
As a method of *in vitro* culture, a method comprising gradually scaling up a monoclonal antibody-producing hybridoma obtained as described above, from a well plate, while keeping the cell density at, for example, about $10^5$ to $10^6$ cells/mL, and gradually lowering the FCS concentration, can be mentioned.
As a method of in vivo culture, for example, a method comprising an intraperitoneal injection of a mineral oil to a mouse (a mouse that is histocompatible with the parent strain of the hybridoma) to induce plasmacytoma (MOPC) 5 to 10 days later, to which intraperitoneally injecting about $10^6$ to $10^7$ cells of hybridoma, and collectinq ascites fluid under anesthesia 2 to 5 weeks later, can be mentioned.

(b) Purification of the monoclonal antibody

Separation and purification of the monoclonal antibody are performed according to a method known per se, such as immunoglobulin separation and purification [e.g., salting-out, alcohol precipitation, isoelectric point precipitation, electrophoresis, adsorption-desorption with an ion exchanger (e.g., DEAE, QEAE), ultracentrifugation, gel filtration, specific purification comprising selectively collecting the antibody by means of an antigen-coupled solid phase or an active adsorbent such as protein A or protein G, and dissociating the linkage to obtain the antibody, and the like].
As described above, a monoclonal antibody can be produced by culturing a hybridoma in or outside the living body of a warm-blooded animal, and harvesting an antibody from the body fluid or culture thereof.
In a preferred mode of embodiment, because the antibody of the present invention is used as a pharmaceutical product having humans as the subject of administration thereof, the antibody of the present invention (preferably a monoclonal antibody) is an antibody whose risk of showing antigenicity when administered to a human has been reduced; to be specific, the antibody is a fully human antibody, a humanized antibody, a mouse-human chimeric antibody and the like, particularly preferably a fully human antibody. A humanized antibody and a chimeric antibody can be prepared by genetic engineering technology according to the method described below. Although a fully human antibody can also be produced from the above-described human-human (or - mouse) hybridoma, it is desirable to produce it using a human antibody-producing animal described below (e.g., mouse) or the phage display method in order to stably supply the antibody in large amounts at low costs.

(i) Preparation of chimeric antibody

As used herein, "a chimeric antibody" means an antibody wherein the sequences of the variable regions of the H chain and L chain ($V_H$ and $V_L$) thereof are derived from a mammalian species, and wherein the sequences of the constant regions ($C_H$ and $C_L$) are derived from another mammalian species. The sequences of the variable regions are preferably derived from, for example, an animal species permitting easy preparation of a hybridoma, such as mouse, and the sequences of the constant regions are preferably derived from the recipient mammalian species.
As examples of the method of preparing a chimeric antibody, the method described in US Patent No. 6,331,415 or a partially modified method thereof and the like can be mentioned. To be specific, first, mRNA or total RNA is prepared from a monoclonal antibody-producing hybridoma (for example, mouse-mouse hybridoma) obtained as described above, according to a conventional method, to synthesize cDNA. DNAs that encode $V_H$ and $V_L$ are amplified and purified by PCR according to a conventional method with the cDNA as the template, using appropriate primers [for example, oligo DNAs comprising the base sequences that encode the N-terminal sequences of $V_H$ and $V_L$, respectively, as the sense primers, and oligo DNAs that hybridize to the base sequences that encode the terminal sequences of $C_H$ and $C_L$, respectively, as the antisense primer (see, for example, Bio/Technology, 9: 88-89, 1991)]. In the same manner, DNAs that encode $C_H$ and $C_L$ are amplified and purified from an RNA prepared from lymphocytes and the like of another mammal (e.g., human) by RT-PCR. $V_H$ and $C_H$, and $V_L$ and $C_L$, are ligated together, respectively, using a conventional method, and the chimeric H chain DNA and chimeric L chain DNA obtained are inserted into respective appropriate expression vectors [for example, vectors comprising promoters that have transcription activity in CHO cells, COS cells, mouse myeloma cells and the like (e.g., CMV promoter, SV40 promoter and the like)]. The DNAs that encode the two chains may be inserted into separate vectors, and may be inserted into a single vector in tandem. Host cells are transformed with the chimeric H chain and chimeric L chain expression vector(s) obtained. As the host cells, animal cells, for example, Chinese hamster ovary (CHO) cells, monkey-derived COS-7 cells, Vero cells, rat-derived GHS cells and the like, in addition to the above-described mouse myeloma cells, can be mentioned. For the transformation, any method applicable to animal cells can be used, with preference given to electroporation method and the like. It is possible to isolate a chimeric monoclonal antibody by culturing the host cells in a medium suitable thereto for a given period, and thereafter recovering the culture supernatant and purifying it in the same manner as described above. Alternatively, it is also possible to obtain a chimeric monoclonal antibody easily and in large amounts from milk or eggs of transgenic animals which are produced by a conventional method using germ line cells of an animal such as bovine, goat, or chicken as the host cells, for which a transgenic technique has been established and a know-how of mass propagation as a domestic animal (domestic fowl) has been compiled. Furthermore, it is also possible to obtain a chimeric monoclonal antibody in large amounts from the seeds, leaves and the like of a transgenic plant, produced by using microinjection and electroporation into protoplast, the particle gun method and Ti-vector method for intact cells and the like, with cells of a plant such as corn, rice, wheat, soybean, or tobacco as the host cells, for which a transgenic technique has been established, and which is cultured in large amounts as a major crop.
When the chimeric monoclonal antibody obtained is digested with papain, Fab is obtained; when the same is digested with pepsin, $F(ab')_2$ is obtained.
It is also possible to reformat into scFv by ligating DNAs that encode mouse $V_H$ and $V_L$ via a suitable linker, for example, a DNA that encodes a peptide consisting of 1 to 40 amino acids, preferably 3 to 30 amino acids, more preferably 5 to 20 amino acids [e.g., [Ser-(Gly)m]n or [(Gly)m-Ser]n (m is an integer from 0 to 10, n is an integer from 1 to 5) and the like]. Furthermore, it is possible to reformat into a minibody by ligating a DNA that encodes $C_{H3}$ via a suitable linker thereto, or reformat into a scFv-Fc by ligating a DNA that encodes $C_H$ full length via a suitable linker thereto. The DNA encoding such an antibody molecule modified (coupled) by genetic engineering can be expressed in a microorganism such as *E. coli* or yeast under the control of a suitable promoter, to produce the antibody molecule in large amounts.
When DNAs encoding mouse $V_H$ and $V_L$ are inserted into the downstream of one promoter in tandem and introduced into E. *coli,* a dimer named as Fv is formed by monocistronic gene expression. When an appropriate amino acid in the FRs of $V_H$ and $V_L$ is substituted with Cys using molecule modeling, a dimer named as dsFv is formed via the intermolecular disulfide bond between the two chains.

(ii) Humanized antibody

As used herein, "a humanized antibody" means an antibody wherein the sequences of all regions present in the variable region, other than the complementality determining region (CDR), [i.e., framework region (FR) in constant region and variable region] are derived from a human, and wherein only the sequence of CDR is derived from another mammalian species. The other mammalian species is preferably an animal species, for example, mouse and the like, with which production of hybridomas can be easily performed.
As examples of the method of preparing a humanized antibody, the methods described in US Patent Nos. 5,225,539, 5,585,089, 5,693,761 and 5,693,762 or partially modified methods therefrom and the like can be mentioned. To be specific, DNAs that encode $V_H$ and $V_L$ derived from a non-human mammalian species (e.g., mouse) are isolated in the same manner as with the above-described chimeric antibody, after which sequencing is performed by a conventional method using an automated DNA sequencer (e.g., manufactured by Applied Biosystems Company and the like), and the base sequences obtained or deduced amino acid sequences therefrom are analyzed using a known antibody sequence database [for example, Kabat database (see Kabat et al., "Sequences of Proteins of Immunological Interest", edited by NIH, US Department of Health and Human Services, Public Health Service, 5th edition, 1991) and the like] to determine the CDR and FR of the two chains. A base sequence wherein the CDR encoding region of a base sequence that encodes the L chain and H chain of a human antibody having an FR sequence similar to the determined FR sequence [e.g., human κ type L chain subgroup I and human H chain subgroup II or III (see Kabat et al., 1991 (*supra*))] is substituted with the determined base sequence that encodes the CDR of another animal species, is designed, and the base sequence is divided into fragments of about 20 to 40 bases, and a sequence complementary to the base sequence is divided into fragments of about 20 to 40 bases so that they alternatively overlap with the aforementioned fragments. It is possible to construct DNAs that encode $V_H$ and $V_L$ having human-derived FR and a CDR derived from another mammalian species by synthesizing individual fragments using a DNA synthesizer, and hybridizing and ligating them in accordance with conventional methods. In order to transfer a CDR derived from another mammalian species into human-derived $V_H$ and $V_L$ more quickly and more efficiently, it is preferable to use PCR-based site directed mutagenesis. As examples of such a method, the sequential CDR grafting method described in Japanese Patent Unexamined Publication No. HEI-5-227970 and the like can be mentioned. It is possible to obtain cells or transgenic animal/plant that produces a humanized antibody by ligating the thus-obtained DNAs that encode $V_H$ and $V_L$ to DNAs that encode human-derived $C_H$ and $C_L$, respectively, in the same manner as with the above-described chimeric antibody, and introducing the ligated product into suitable host cells.
A humanized antibody, like a chimeric antibody, can be modified to scFv, scFv-Fc, minibody, dsFv, Fv and the like by using genetic engineering techniques; and they can be produced in a microorganism such as E. *coli* or yeast by using a suitable promoter.
The technology for preparing a humanized antibody can also be applied to, for example, preparing a monoclonal antibody that can be preferably administered to another animal species for which no hybridoma production technology has been established. For example, animals widely propagated as domestic animals (domestic fowls) such as bovine, swine, sheep, goat, and chicken, and pet animals such as dogs and cats, and the like can be mentioned as the subject animal species.

(iii) Preparation of fully human antibody using human antibody-producing animal

Provided that a functional human Ig gene is introduced into a non-human warm-blooded animal having the endogenous immunoglobulin (Ig) gene knocked out (KO) therein, and that this animal is immunized with an antigen, a human antibody is produced in place of the antibody derived from the animal. Therefore, provided that an animal such as mice, for which a technique for producing a hybridoma has been established, is used, it is possible to acquire a fully human monoclonal antibody by the same method as the conventional method used to prepare a mouse monoclonal antibody. First, some of the human monoclonal antibodies, that were generated by using a human antibody-producing mouse obtained by crossing a mouse transfected with minigenes of the human Ig H chain and L chain using an ordinary transgenic (Tg) technique with a mouse wherein the endogenous mouse Ig gene has been inactivated using an ordinary KO technique, are already in clinical stage, and to date production of anti-human Ig human antibody (HAHA) has not been reported.

Later, Abgenix Inc. [trade name: XenoMouse (see Nat. Genet., 15: 146-156, 1997; US Patent No. 5,939,598 and the like)] and Medarex Inc. [trade name: Hu-Mab Mouse (see Nat. Biotechnol., 14: 845-851, 1996; US Patent No. 5,545,806 and the like)] established Tg mice transfected with even a larger human Ig gene using a yeast artificial chromosome (YAC) vector, thus enabling the production of human antibodies of richer repertoire. However, because the human Ig gene, for example, in the case of the H chain, exhibits its diversity as the VDJ exon, which is a variable combination of about 80 kinds of V fragments, about 30 kinds of D fragments and 6 kinds of J fragments, encodes the antigen binding site, the full length thereof is as large as about 1.5 Mb (14th chromosome) for the H chain, about 2 Mb (2nd chromosome) for the κL chain, and about 1 Mb (22nd chromosome) for the λL chain. To reproduce the diverse antibody repertoire in human in another animal species, it is desirable to introduce the full length of each Ig gene. However, a DNA that is insertable into a conventional transfection vector (plasmid, cosmid, BAC, YAC and the like) is normally several kb to several hundred kb in length, and it has been difficult to introduce the full length of Ig genes by the conventional technique for establishing a transgenic animal, which comprises inserting a cloned DNA into a fertilized egg.

Tomizuka et al. (Nat. Genet., 16: 133-143, 1997) prepared a mouse having the full-length human Ig gene by introducing a natural fragment of a human chromosome harboring the Ig gene (hCF) into a mouse [transchromosomic (TC) mouse]. That is, first, a human-mouse hybrid cell having human chromosomes in which the 14th chromosome comprisinq the H chain gene and the 2nd chromosome comprising the κL chain gene, both labeled with, for example, a drug-resistance marker and the like, is treated with a spindle formation inhibitor (e.g., colcemid) for about 48 hours to prepare a microcell wherein one to several chromosomes or fragments thereof are enveloped in nuclear membrane, and the chromosomes are introduced into a mouse ES cell by the micronuclear fusion method. A hybrid ES cell retaining the chromosomes having the human Ig gene or fragments thereof is selected using a medium containing a drug, and the cell is microinjected into a mouse embryo in the same manner as with the preparation of an ordinary KO mouse. A germ line chimera is selected among the chimeric mice obtained, with coat color as the index, and the like, to establish a TC mouse strain carrying the human 14th chromosome fragment (TC(hCF14)) and a TC mouse strain carrying the human 2nd chromosome fragment (TC(hCF2)). After establishing mouse strains wherein the endogenous H chain gene and κM chain gene are knocked out, respectively [KO (IgH) and KO (Igκ)] by a conventional method, it is possible to establish a mouse strain having all the four kinds of gene modifications (double TC/KO) by repeating the crossing of these four strains.

Provided that the same method as that for producing an ordinary mouse monoclonal antibody is applied to a double TC/KO mouse established as described above, it is possible to obtain an antigen-specific human monoclonal antibody-producing hybridoma. However, there is the drawback of a lower efficiency to obtain hybridomas than that with the ordinary mouse, because hCF2 containing the κL chain gene is unstable in the mouse cells.

On the other hand, because the aforementioned Hu-Mab mouse has a structure wherein the variable region cluster are doubled although it has about 50% of the κL chain gene, it exhibits a κ chain diversity equivalent to that with full length (on the other hand, HuMab mouse exhibits a low H chain diversity and inadequate response to antigen because it carries only about 10% of the H chain gene). And the κ chain is stably retained in the mouse cells because it is inserted in mouse chromosome via a YAC vector (Igκ-YAC). Making use of this advantage, it is possible to get the efficiency for obtaining hybridomas and affinity to antigen affinity of antibody that are equivalent to those with the ordinary mouse, by crossing a TC(hCF14) mouse with a Hu-Mab mouse to establish a mouse that stably retains both hCF14 and Igκ-YAC (trade name: KM mouse).

Furthermore, it is also possible to establish a human antibody-producing animal in which the λL chain gene is further transfected to reconstruct the diverse human antibody repertoire more completely. Such an animal can also be obtained by producing a TC mouse in which the human 22nd chromosome or a fragment thereof harboring the λL chain gene is introduced in the same manner as described above[TC(hCF22)], and crossing the mouse with the above-described double TC/KO mouse or KM mouse, or can also be obtained by, for example, constructing a human artificial chromosome (HAC) comprising both the H chain locus and the λL chain locus, and introducing it into a mouse cell (Nat.

Biotechnol., 18: 1086-1090, 2000).

When the antibody of the present invention is utilized as a pharmaceutical product, it is desirably a monoclonal antibody, but may be a polyclonal antibody. When the antibody of the present invention is a polyclonal antibody, it is not necessary to use hybridomas; therefore, provided that a human antibody-producing animal is produced in the same manner as described above using an animal species for which no technique for preparing a hybridoma has been established but a transgenic technique has been established, preferably an ungulate such as bovine, it is also possible to produce a human antibody in larger amounts at low costs (see, for example, Nat. Biotechnol., 20: 889-894, 2002). The human polyclonal antibody thus obtained can be purified by collecting blood, ascites fluid, milk, egg and the like, preferably milk or egg, of the human antibody-producing animal, in combination with the same purification techniques as described above.

(iv) Preparation of fully human antibody using phage display human antibody library

Another approach to produce a fully human antibody is a method using phage display. This method sometimes encounters cases in which a mutation due to PCR is introduced into a site other than CDRs; for this reason, a few reports of cases of HAHA production in clinical stage are available. On the other hand, however, the method has advantages such as no risk of cross-species viral infection derived from the host animal and the indefinite specificity of the antibody (antibodies against forbidden clone, sugar chain and the like can also be easily prepared).

The method of preparing a phage display human antibody library include, but are not limited to, for example, the methods described below.

Although a phage used is not subject to limitation, filamentous phage (Ff bacteriophage) is normally preferably used. As the method of presenting a foreign protein on the phage surface, a method comprising expressing and presenting the foreign protein as a fusion protein with any of the coat proteins g3p, and g6p to g9p on the coat protein can be mentioned; and a method comprising fusing the foreign protein to the N-terminal side of g3p or g8p is often used. As the phage display vector, besides 1) one in which the foreign gene is introduced in the form of fusion gene with the coat protein gene of the phage genome, to allow all the coat proteins presented on the phage surface to be presented as a fusion protein with the foreign protein, 2) one in which the gene encoding the fusion protein is inserted separately from the wild-type coat protein gene to allow the fusion protein and the wild-type coat protein to be expressed simultaneously, and 3) an *E. coli* having a phagemid vector harboring the gene that encodes the fusion protein is infected with a helper phage having the wild-type coat protein gene to produce phage particles that express the fusion protein and the wild-type coat protein simultaneously, and the like can be mentioned. However, a phage display vector of the type 2) or 3) is used for the preparation of an antibody library, because in the case of 1), the capability of infection is lost when a large foreign protein is fused.

As a specific vector, those described by Holt et al. (Curr. Opin. Biotechnol., 11: 445-449, 2000) can be mentioned as examples. For example, pCES1 (see J. Biol. Chem., 274: 18218-18230, 1999) is an Fab-expressing phagemid vector wherein a DNA encoding the κL chain constant region allocated to downstream of the g3p signal peptide, and a DNA encoding CH3, His-tag, c-myc tag, and the amber stop codon (TAG) followed by the g3p coding sequence, allocated to downstream of the g3p signal peptide, are arranged under the control of one lactose promoter. When this is introduced to an *E. coli* having an amber mutation, Fab is presented onto the g3p coat protein, but when it is expressed in the HB2151 strain and the like, which do not have an amber mutation, a soluble Fab antibody is produced. And as the scFv-expressing phagemid vector, for example, pHEN1 (J. Mol. Biol., 222: 581-597, 1991) and the like are used.

Meanwhile as examples of the helper phage, M13-KO7, VCSM13 and the like can be mentioned.

And as another phage display vector, a vector that is designed as a DNA sequence comprising the cysteine-encoding codon is linked to each of the 3' end of the antibody gene and the 5' end of the coat protein gene to express the two genes simultaneously and separately (not in the form of a fusion protein), and to present the antibody onto the coat protein on the phage surface via S-S bonds between the introduced cysteine residues (CysDisplay™ technology of Morphosys Company) and the like, can be mentioned.

As the kind of human antibody library, a naive/non-immunized library, a synthetic library, an immunized library and the like can be mentioned.

The naive/non-immunized library is a library obtained by acquiring the $V_H$ and $V_L$ genes retained by a normal human by RT-PCR, and randomly cloning them into the above-described phage display vector. Normally, mRNA derived from lymphocytes of peripheral blood, bone marrow, tonsil and the like of a normal human, and the like are used as the template. A library prepared by selectively amplifying IgM-derived mRNA in which a class switch due to antigen sensitization is not undergoing, to avoid V gene biases such as clinical history, is particularly called a naive library. Representatively, the library of Cambridge Antibody Technology (see J. Mol. Biol., 222: 581-597, 1991; Nat. Biotechnol., 14: 309-314, 1996), the library of Medical Research Council (see Annu. Rev. Immunol., 12: 433-455, 1994), the library of Dyax Corp. (see J. Biol. Chem., 1999 (supra*);* Proc. Natl. Acad. Sci. USA, 14: 7969-7974, 2000) and the like can be mentioned.

A synthetic library is obtained by selecting a functional particular antibody gene in human B cells, and substituting a portion of antigen-binding region in a V gene fragment, for example, CDR3 and the like, with DNAs encoding a random amino acid sequence of appropriate length, to construct a library. It is recognized to be excellent in antibody expression efficiency and stability because the library can be constructed with the combination of the $V_H$ and $V_L$ genes, which produce functional scFv and Fab, since the beginning. Representatively, the HuCAL library of Morphosys AG (see J. Mol. Biol., 296: 57-86, 2000), the library of BioInvent (see Nat. Biotechnol., 18: 852, 2000), the library of Crucell (see Proc. Natl. Acad. Sci. USA, 92: 3938, 1995; J. Immunol. Methods, 272: 219-233, 2003) and the like can be mentioned. An immunized library is a library obtained by preparing an mRNA from lymphocytes collected from a human having an increased blood antibody titer against the target antigen, or from human lymphocytes and the like which are artificially immunized with the target antigen by the above-described *in vitro* immunization method, in the same manner as with the above-described naive/non-immunized library, and amplifying the $V_H$ and $V_L$ genes by RT-PCR, to construct a library. It is possible to obtain the desired antibody even from such libraries of relatively small size because the desired antibody gene is contained in the library already at the beginning.

The wider the diversity of the library is, the better; actually, however, an appropriate library size is about $10^8$ to $10^{11}$ clones, taking into consideration of the number of phages handlable in the following panning operation ($10^{11}$ to $10^{13}$ phages) and the number of phages necessary to isolate and amplify clones in ordinary panning (100 to 1,000 phages/clone),; it is possible to screen for an antibody normally having a Kd value on the order of $10^{-9}$ with a library of about $10^8$ clones.

The process for selecting an antibody against the target antigen by the phage display method is referred to as panning. To be specific, for example, a phage presenting an antigen-specific antibody is concentrated by repeating a series of operations of bringing an antigen-immobilized carrier and a phage library into contact with each other, washing out the unbound phage, thereafter eluting the bound phage from the carrier, and infecting the phage to *E. coli* to proliferate it, about 3 to 5 times. As the carrier for immobilizing the antigen, various carriers used in ordinary antigen-antibody reactions or affinity chromatography, for example, insoluble polysaccharides such as agarose, dextran, and cellulose, synthetic resins such as polystyrene, polyacrylamide, and silicon, or microplates, tubes, membranes, columns, beads and the like comprising glass, metal and the like, and surface plasmon resonance (SPR) sensor chips, and the like can be mentioned. For the antigen immobilization, physical adsorption may be used, and a method using a chemical bond used to insolubilize and immobilize a protein or enzyme and the like is also acceptable. For example, a biotin-(strept) avidin system and the like are preferably used. When the RAIG2 (or RAIG3), that is a target antigen, is a small molecule such as an oligopeptide, it is necessary to pay special attention to prevent masking of the portion used as the epitope by conjugating with the carrier. For washing the unbound phage, a blocking solution such as BSA solution (once or twice), a PBS containing a surfactant such as Tween (3 to 5 times) and the like can be used. There is also a report mentioning that the use of citrate buffer (pH 5) and the like is preferable for the washing. For elution of the specific phage, an acid (e.g., 0.1 M hydrochloric acid and the like) is normally used; cleavage with a specific protease (for example, a gene sequence that encodes the trypsin cleavage site can be introduced into the linkage site between the antibody gene and the coat protein gene. In this case, *E. coli* infection and proliferation are possible even if all the coat protein is expressed in the form of a fusion protein because the wild-type coat protein is presented on the surface of the eluted phage), competitive elution with a soluble antigen, or elution by reduction of S-S bond (for example, in the aforementioned CysDisplay™, the antigen-specific phage can be recovered by dissociating the antibody and the coat protein by using a suitable reducing agent after performing panning.) is also possible. When elution has been performed with an acid, the eluate is neutralized with Tris and the like, and the eluted phage is then infected to E. *coli,* which is cultured; after which the phage is recovered by a conventional method.

After the phage presenting the antigen-specific antibody is concentrated by panning, the phage is infected to *E. coli* and the cells are sown onto a plate to perform cell cloning. The phage is again collected from the each clone, and the antigen binding activity is confirmed by the above-described antibody titer assay (e.g., ELISA, RIA, FIA and the like) or a measurement utilizing FACS or SPR.

Isolation and purification of the antibody from the selected phage clone that presents the antigen-specific antibody can be performed by, for example, when using a vector incorporating an amber stop codon at the linker site of the antibody gene and the coat protein gene as the phage display vector, infecting the phage to an E. *coli* that does not have amber mutation (e.g., HB2151 strain) to produce and secrete soluble antibody molecules in periplasm or the medium, lysing the cell wall with lysozyme and the like, collecting the extracellular fraction, and purifying using the same purification technique as described above. Provided that the His-tag or c-myc tag has been introduced in advance, the antibody can easily be purified by using IMAC, an anti-c-myc antibody column and the like. When cleavage with a specific protease is utilized in panning, the antibody molecule is separated from the phage surface by an action with the protease, so that the desired antibody can be purified by performing the same purification operation as above mentioned.

The technology for producing a fully human antibody using a human antibody-producing animal and a phage display human antibody library can also be applied to the production of a monoclonal antibody derived from another animal species. For example, animals widely propagated as domestic animals (domestic fowls) such as bovine, swine, sheep, goat, and chicken, and pet animals such as dogs and cats, and the like can be mentioned as the subject animal species. In non-human animals, the utilization of an immunized library is more effective because there are fewer ethical problems concerning artificial immunization with the target antigen.

(3) Preparation of polyclonal antibody

The polyclonal antibody of the present invention can be produced according to a method known per se or a method based thereon. For example, the polyclonal antibody of the present invention can be produced by forming a complex of an immunoantigen (a protein or a peptide antigen) itself, or an immunoantigen and a carrier protein, immunizing a warm-blooded animal with the complex in the same manner as the above-described method of monoclonal antibody production, collecting a product containing an antibody against RAIG2 (or RAIG3) from the immunized animal, and separating and purifying the antibody.

Regarding the complex of immunoantigen and carrier protein used to immunize a warm-blooded animal, the kind of carrier protein and the mixing ratio of carrier protein and hapten may be any ones whatever they are, as long as an antibody against the immunizing hapten crosslinked to the carrier protein is efficiently produced; for example, a method that comprises coupling bovine serum albumin, bovine thyroglobulin, hemocyanin or the like to the hapten in a ratio by weight of about 0.1 to about 20, preferably about 1 to about 5, to 1 of hapten, can be used.

Various condensing agents can be used for the coupling of hapten and carrier protein; glutaraldehyde, carbodiimide, maleimide activated ester, activated ester reagents comprising the thiol group or the dithiopyridyl group, and the like can be used.

The condensation product is administered as is, or along with a carrier or a diluent, to a warm-blooded animal at a site enabling antibody production by its administration. In order to increase antibody productivity upon the administration, Freund's complete adjuvant or Freund's incomplete adjuvant may be administered. Dosing is normally performed about 2 to about 10 times in total every 1 to 6 weeks.

The polyclonal antibody can be collected from blood, ascites fluid and the like, preferably blood, of a warm-blooded animal immunized by the above-described method.

Polyclonal antibody titer in antiserum can be determined in the same manner as the above-described determination of antibody titer in antiserum. Separation and purification of the polyclonal antibody can be performed according to a method of immunoglobulin separation and purification, as in the above-described separation and purification of monoclonal antibody.

RAIG2 (or RAIG3) functions as a receptor of glucose or 1,5-AG to control the secretion of hormones such as insulin and ACTH in response to blood glucose concentrations. Therefore, RAIG2 (or RAIG3) or a partial peptide thereof, a nucleic acid that encodes the same or a portion thereof, an antibody against RAIG2 (or RAIG3) (hereinafter sometimes referred to as "the antibody of the present invention"), and a nucleic acid that encodes an antisense nucleic acid for RAIG2 (or RAIG3) (hereinafter sometimes referred to as "the antisense nucleic acid of the present invention") have the uses shown below.

[1] Sugar/lipid metabolism regulator and the like

Because RAIG2 (or RAIG3) senses glucose and 1,5-AG concentrations in the blood and promotes insulin secretion in the pancreas, and promotes ACTH secretion in the pituitary at low glucose concentrations/suppresses the same at high glucose concentrations, a) RAIG2 (or RAIG3) or a partial peptide thereof, and b) a DNA that encodes RAIG2 (or RAIG3) or a partial peptide thereof can be used as 1) a regulator of sugar/lipid metabolism, 2) a regulator of pancreas and/or pituitary function, 3) an insulin secretion promoter and/or an agent for reducing blood glucose level and/or ACTH secretion regulator, 4) a prophylactic/therapeutic agent for a disease involved by a sugar/lipid metabolism abnormality, a pancreas and/or pituitary function abnormality, or an insulin secretion and/or blood glucose level and/or ACTH secretion abnormality and the like.

For example, for a patient in whom the physiological action of ligand glucose or 1,5-AG is unexpectable because of a reduction in RAIG2 (or RAIG3) in vivo (i.e., RAIG2 (or RAIG3) deficiency), it is possible to increase the amount of RAIG2 (or RAIG3) in the patient's body to allow glucose or 1,5-AG to fully exhibit the action thereof, a) by administering RAIG2 (or RAIG3) or a partial peptide thereof to the patient to supplement for the amount of RAIG2 (or RAIG3), or b) (i) by administering a nucleic acid (preferably DNA) that encodes RAIG2 (or RAIG3) or a partial peptide thereof to the patient to express the nucleic acid, or (ii) by introducing to the target cell a nucleic acid (preferably DNA) that encodes RAIG2 (or RAIG3) or a partial peptide thereof to express the nucleic acid, and then transplanting the cell to the patient, and the like.

Examples of diseases involved by a sugar/lipid metabolism abnormality, a pancreas and/or pituitary function abnormality, or an insulin secretion and/or blood glucose level and/or ACTH secretion abnormality include, but are not limited to, diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder and the like.

RAIG2 (or RAIG3) or a partial peptide thereof and a DNA that encodes the same (hereinafter sometimes referred to as "then DNA of the present invention") can be used as the above-described pharmaceutical after being blended with a pharmacologically acceptable carrier to form a pharmaceutical compound as required.

As the pharmacologically acceptable carrier, various organic or inorganic carrier substances in common use as pharmaceutical materials can be used, which are formulated as excipients, lubricants, binders, and disintegrants in solid preparations; as solvents, solubilizers, suspending agents, isotonizing agents, buffers, soothing agents and the like in liquid preparations. Pharmaceutical additives such as antiseptics, antioxidants, colorants, and sweeteners can also be used as necessary.

As examples of preferable excipients, lactose, sucrose, D-mannitol, D-sorbitol, starch, α-starch, dextrin, crystalline cellulose, low-substituted hydroxypropylcellulose, carboxymethylcellulose sodium, gum arabic, pullulan, light silicic anhydride, synthetic aluminum silicate, magnesium metasilicate aluminate and the like can be mentioned.

As examples of preferable lubricants, magnesium stearate, calcium stearate, talc, colloidal silica and the like can be mentioned.

As examples of preferable binders, α-starch, cane sugar, gelatin, gum arabic, methylcellulose, carboxymethylcellulose, carboxymethylcellulose sodium, crystalline cellulose, sucrose, D-mannitol, trehalose, dextrin, pullulan, hydroxypropylcellulose, hydroxypropylmethylcellulose, polyvinylpyrrolidone and the like can be mentioned.

As examples of preferable disintegrants, lactose, sucrose, starch, carboxymethylcellulose, carboxymethylcellulose calcium, crosslinked carmellose sodium, carboxymethylstarch sodium, light silicic anhydride, low- substituted hydroxypropylcellulose and the like can be mentioned.

As examples of preferable solvents, water for injection, physiological saline, Ringer's solution, alcohol, propylene glycol, polyethylene glycol, sesame oil, corn oil, olive oil, cottonseed oil and the like can be mentioned.

As examples of preferable solubilizers, polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like can be mentioned.

As examples of preferable suspending agents, surfactants such as stearyltriethanolamine, sodium lauryl sulfate, laurylaminopropionic acid, lecithin, benzalkonium chloride, benzethonium chloride, and glyceryl monostearate; hydrophilic polymers such as polyvinyl alcohol, polyvinylpyrrolidone, carboxymethylcellulose sodium, methylcellulose, hydroxymethylcellulose, hydroxyethylcellulose, and hydroxypropylcellulose; polysorbates, polyoxyethylene hydrogenated castor oil and the like can be mentioned.

As examples of preferable isotonizing agents, sodium chloride, glycerin, D-mannitol, D-sorbitol, glucose and the like can be mentioned.

As examples of preferable buffers, buffer solutions such as of phosphates, acetates, carbonates and citrates, and the like can be mentioned.

As examples of preferable soothing agents, benzyl alcohol and the like can be mentioned.

As examples of preferable antiseptic agents, paraoxybenzoic acid esters, chlorobutanol, benzyl alcohol, phenethyl alcohol, dehydroacetic acid, sorbic acid and the like can be mentioned.

As examples of preferable antioxidants, sulfites, ascorbic acid salts and the like can be mentioned.

As examples of preferable colorants, water-soluble food tar colors (e.g., food colors such as Food Color Red No.2 and No.3, Food Color Yellow No. 4 and No.5, and Food Color Blue No. 1 and No.2), water-insoluble lake colors (e.g., aluminum salts of the aforementioned water-soluble food tar colors, and the like) , natural colors (e.g., β-carotene, chlorophyll, red iron oxide and the like) and the like can be mentioned.

As examples of preferable sweeteners, saccharin sodium, dipotassium glycyrrhizinate, aspartame, stevia and the like can be mentioned.

As examples of dosage forms for the aforementioned pharmaceutical composition, oral preparations such as tablets, capsules (including soft capsules and microcapsules), granules, powders, syrups, emulsions, and suspensions; and non-oral preparations such as injections (e.g., subcutaneous injections, intravenous injections, intramuscular injections, intraperitoneal injections and the like), external preparations (e.g., nasal preparations, transdermal preparations, ointments and the like), suppositories (e.g., rectal suppositories, vaginal suppositories and the like), pellets, drip infusions, and sustained-release preparations (e.g., sustained-release microcapsules and the like) can be mentioned.

These pharmaceutical compositions can be produced by a method in common use in the field of drug formulation technology, for example, methods described in the Japanese Pharmacopoeia and the like. Specific methods of preparing preparations are described in detail below. The active ingredient content in the pharmaceutical composition varies depending on dosage form, active ingredient dose and the like, and is, for example, about 0.1% to 100% by weight.

For example, an oral preparation is produced by adding an excipient (e.g., lactose, sucrose, starch, D-mannitol and the like), a disintegrant (e.g., carboxymethylcellulose calcium and the like), a binder (e.g., α-starch, gum arabic, carboxymethylcellulose, hydroxypropylcellulose, polyvinylpyrrolidone and the like), a lubricant (e.g., talc, magnesium stearate, polyethylene glycol 6000 and the like) and the like, to active ingredients, and compressing them, with coating performed, if necessary, using a coating base for the purpose of taste masking, enteric dissolution or sustained release, by a method known per se.

As examples of the coating base, sugar-coating bases, water-soluble film-coating bases, enteric film-coating bases, sustained-release film-coating bases and the like can be mentioned.

As the sugar-coating base, sucrose is used, which may be used in combination with one or two or more kinds selected from among talc, precipitated calcium carbonate, gelatin, gum arabic, pullulan, carnauba wax and the like.

As examples of the water-soluble film-coating base, cellulose polymers such as hydroxypropylcellulose, hydroxypropylmethylcellulose, hydroxyethylcellulose and methylhydroxyethylcellulose; synthetic polymers such as polyvinylacetal diethylaminoacetate, aminoalkylmethacrylate copolymer E [Eudragit E (trade name), Roehm Pharma GmbH], and polyvinylpyrrolidone; polysaccharides such as pullulan; and the like can be mentioned.

As examples of the enteric film-coating base, cellulose polymers such as hydroxypropylmethylcellulose phthalate, hydroxypropylmethylcellulose acetate succinate, carboxymethylethylcellulose, and cellulose acetate phthalate; acrylate polymers such as methacrylate copolymer L [Eudragit L (trade name), Roehm Pharma GmbH], methacrylate copolymer LD [Eudragit L-30D55 (trade name), Roehm Pharma GmbH], and methacrylate copolymer S [Eudragit S (trade name), Roehm Pharma GmbH]; naturally occurring substances such as shellac; and the like can be mentioned. As examples of the sustained-release film-coating base, cellulose polymers such as ethylcellulose; acrylate polymers such as aminoalkylmethacrylate copolymer RS [Eudragit RS (trade name), Roehm Pharma GmbH] and ethyl acrylate/methyl methacrylate copolymer suspension [Eudragit NE (trade name), Roehm Pharma GmbH]; and the like can be mentioned.

The above-described coating bases may be used in a suitable mixture of two or more kinds thereof. Also, a light-blocking agent such as titanium oxide or iron sesquioxide may be used during coating.

For example, an injection is produced by dissolving, suspending or emulsifying active ingredients, along with a dispersing agent (e.g., polysorbate 80, polyoxyethylene hydrogenated castor oil 60, polyethylene glycol, carboxymethylcellulose, sodium alginate and the like), a preservative (e.g., methylparaben, propylparaben, benzyl alcohol, chlorobutanol, phenol and the like), an isotonizing agent (e.g., sodium chloride, glycerin, D-mannitol, D-sorbitol, glucose and the like) and the like, in an aqueous solvent (e.g., distilled water, physiological saline, Ringer's solution and the like) or an oily solvent (e.g., vegetable oils such as olive oil, sesame oil, cottonseed oil and corn oil, propylene glycol and the like). If desired, additives such as a solubilizer (e.g., sodium salicylate, sodium acetate and the like), a stabilizer (e.g., human serum albumin and the like), and a soothing agent (e.g., benzyl alcohol and the like) may be used. The injection is subjected, usually filled in a suitable ampoule.

Because the preparation thus obtained is safe and less toxic, it can be administered to, for example, mammals (for example, humans, rats, rabbits, sheep, swine, cattle, cats, dogs, monkeys and the like).

When the DNA of the present invention is used as the above-described prophylactic/therapeutic agent, the DNA of the present invention can be administered alone, or after being inserted into an appropriate expression vector such as a retrovirus vector, adenovirus vector, or adenovirus-associated virus vector, according to a conventional method. The DNA of the present invention can also be administered as is, or along with an auxiliary for promoting its ingestion, using a gene gun or a catheter such as a hydrogel catheter.

The dose of RAIG2 (or RAIG3) or a partial peptide thereof varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, a diabetic patient (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, a diabetic patient (assuming a 60 kg body weight). In cases where the subject of administration is other than a human, a dose corresponding to a human dose per 60 kg body weight can be administered.

The dose of the DNA of the present invention varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, a diabetic patient (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, a diabetic patient (assuming a 60 kg body weight). In cases where the subject of administration is other than a human, a dose corresponding to a human dose per 60 kg body weight can be administered.

Meanwhile, a neutralizing antibody against RAIG2 (or RAIG3) (the neutralizing antibody of the present invention) is capable of inactivating (i.e., neutralizing) a signal transduction function involved by RAIG2 (or RAIG3), for example, regulatory activity on secretion of hormones such as insulin and ACTH, by inhibiting the interaction between glucose or 1,5-AG and RAIG2 (or RAIG3). Meanwhile, a nucleic acid containing a base sequence complementary to the base sequence that encodes RAIG2 (or RAIG3) or a portion thereof (including ribozyme and siRNA as described above; the antisense nucleic acid of the present invention) is capable of inhibiting the expression of RAIG2 (or RAIG3) by blocking RAIG2 (or RAIG3) gene transcription, transcription product processing and/or translation from mRNA. Therefore, (a) the neutralizing antibody of the present invention or (b) the antisense nucleic acid of the present invention can be used in the direction reverse to the above-described RAIG2 (or RAIG3) or the DNA of the present invention as 1) a regulator of sugar/lipid metabolism, 2) regulator of pancreas and/or pituitary function, 3) an insulin secretion suppressor and/or an agent for increasing blood glucose level and/or ACTH secretion regulator, 4) a prophylactic/therapeutic agent for a disease involved by a sugar/lipid metabolism abnormality, a pancreas and/or pituitary function abnormality, or an insulin secretion and/or blood glucose level and/or ACTH secretion abnormality and the like. Examples of the disease include, but are not limited to, obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity, cancers and the like.

The antibody of the present invention and the antisense nucleic acid of the present invention can be prepared as pharmaceutical preparations in the same manner as with RAIG2 (or RAIG3) or a partial peptide thereof and the DNA of the present invention above, respectively. The antisense nucleic acid can also be administered as is, using a gene gun or a catheter such as a hydrogel catheter.

The dose of the antibody of the present invention varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, an obese patient (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, an obese patient (assuming a 60 kg body weight). In cases where the subject of administration is other than a human, a dose corresponding to a human dose per 60 kg body weight can be administered.

The dose of the antisense nucleic acid of the present invention varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, an obese patient (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, an obese patient (assuming a 60 kg body weight). In cases where the subject of administration is other than a human, a dose corresponding to a human dose per 60 kg body weight can be administered.

[2] Genetic diagnostic reagent

A nucleic acid that encodes RAIG2 (or RAIG3) or a portion thereof (hereinafter sometimes referred to as "the sense nucleic acid of the present invention") and the antisense nucleic acid of the present invention, when used as probes or primers, are capable of detecting an abnormality of a DNA or mRNA that encodes RAIG2 (or RAIG3) (gene abnormality) in humans or mammals (for example, rats, mice, rabbits, sheep, pigs, bovines, cats, dogs, monkeys and the like), and are therefore useful as, for example, genetic diagnostic reagents for damages, mutations or decreased expression of the DNA or mRNA, increases or overexpression of the DNA or mRNA and the like.

The above-described genetic diagnosis using the sense or antisense nucleic acid of the present invention can be performed by, for example, methods known per se, such as Northern hybridization and the PCR-SSCP method (Genomics, Vol. 5, pp. 874-879 (1989), Proceedings of the National Academy of Sciences of the United States of America, Vol. 86, pp. 2766-2770 (1989)).

For example, if decreased expression of RAIG2 (or RAIG3) expression is detected by Northern hybridization and the like, it can be judged, for example, that the subject is likely to be suffering from a disease involved by RAIG2 (or RAIG3) dysfunction, or is likely to suffer from the same in the future. Examples of diseases involved by RAIG2 (or RAIG3) dysfunction include diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder and the like.

If overexpression of RAIG2 (or RAIG3) is detected by Northern hybridization and the like, it can be judged, for example, that the subject is likely to be suffering from a disease involved by RAIG2 (or RAIG3) overexpression, or likely to suffer from the same in the future. Examples of diseases involved by overexpression of RAIG2 (or RAIG3) include obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity, cancers and the like.

[3] Screening for compound that alters the amount of RAIG2 (or RAIG3) expressed

The sense and antisense nucleic acids of the present invention can be used as probes or primers for screening for a compound that alters the amount of RAIG2 (or RAIG3) expressed.
Accordingly, the present invention provides a method of screening a compound that changes the expression amount of RAIG2 (or RAIG3), which comprises measuring the amount of RAIG2 (or RAIG3) mRNA contained in, for example, (i) (1) blood, (2) a particular organ, (3) a tissue or cells isolated from an organ, of a non-human mammal, or (ii) a transformant or the like.
Specifically, the amount of RAIG2 (or RAIG3) mRNA is measured as described below.

(i) A drug (for example, anti-obesity drugs, anti-diabetic drugs, antihypertensive drugs, vasoactive drugs, antidepressant agents and the like) or a physical stress (for example, soaking stress, electric shock, brightness/darkness, low temperatures and the like) or the like is given to a normal or disease model non-human mammal (for example, mice, rats, rabbits, sheep, swine, cattle, cats, dogs, monkeys and the like, more specifically, obese mice, diabetic mice, hypertensive rats, arteriosclerotic rabbits, depressive rats and the like); after a given time has elapsed, blood or a particular organ (for example, pancreas, pituitary and the like), a tissue (for example, pancreatic islet, pituitary gland tissue and the like) or cells (for example, pancreatic β cells, pituitary gland cells and the like) are obtained.
The RAIG2 (or RAIG3) mRNA contained in the cells and the like obtained can be quantified by, for example, extracting the mRNA from the cells and the like by an ordinary method, and using a technique such as TaqMan PCR, and can also be analyzed by performing Northern blot by a means known per se.
(ii) A transformant expressing RAIG2 (or RAIG3) or a partial peptide thereof can be prepared according to the method described above, and the mRNA of the RAIG2 (or RAIG3) or a partial peptide thereof contained in the transformant can be quantified and analyzed in the same manner as described above.

Screening of a compound that changes the expression amount of RAIG2 (or RAIG3) can be performed by:

(i) administering a test compound to a normal or disease model non-human mammal at a given time before (before 30 minutes to before 24 hours, preferably before 30 minutes to before 12 hours, more preferably before 1 hour to before 6 hours) or after (after 30 minutes to after 3 days, preferably after 1 hour to after 2 days, more preferably after 1 hour to after 24 hours) giving a drug, a physical stress or the like, or at the same time as the drug or physical stress, and quantifying and analyzing the amount of RAIG2 (or RAIG3) mRNA contained in the cells after a given time has elapsed after administration (after 30 minutes to after 3 days, preferably after 1 hour to after 2 days, more preferably after 1 hour to after 24 hours), and can also be preformed by: (ii) mixing a test compound in the medium at the start of cultivation of the transformant according to a conventional method, and quantifying and analyzing the amount of mRNA of RAIG2 (or RAIG3) or a partial peptide thereof contained in the transformant after cultivation for a given time (after 1 day to after 7 days, preferably after 1 day to after 3 days, more preferably after 2 days to after 3 days).

The compound obtained using the above-described screening method or a salt thereof is a compound having an action to alter the amount of RAIG2 (or RAIG3) expressed; specifically, the compound is (a) a compound that enhances a cell stimulatory activity via the interaction between RAIG2 (or RAIG3) and glucose or 1,5-AG (for example, intracellular cAMP production regulation, inositol phosphate production regulation, intracellular $Ca^{2+}$ uptake, cGMP production regulation, insulin secretion promotion, blood glucose reduction, ACTH secretion regulation, appetite regulation and the like) by increasing the amount of RAIG2 (or RAIG3) expressed, or (b) a compound that weakens the cell stimulatory activity by reducing the amount of RAIG2 (or RAIG3) expressed.
As the compound used, peptides, proteins, non-peptide compounds, synthetic compounds, fermentation products and the like can be mentioned; these compounds may be novel compounds or known compounds.
A compound that enhances the cell stimulation activity is useful as a safe and less toxic pharmaceutical for enhancing a physiological activity of RAIG2 (or RAIG3).
A compound that weakens the cell stimulation activity is useful as a safe and less toxic pharmaceutical for reducing a physiological activity of RAIG2 (or RAIG3).
Because RAIG2 (or RAIG3), as described above, senses blood glucose concentrations, promotes insulin secretion from the pancreas to decrease blood glucose, and controls ACTH secretion by CRF (corticotropin release factor) according to blood glucose concentration, a compound that alters the amount of RAIG2 (or RAIG3) expressed can be used as 1) a regulator of sugar/lipid metabolism, 2) a regulator of pancreas and/or pituitary function, 3) a regulator of insulin secretion and/or blood glucose and/or ACTH secretion, 4) a prophylactic/therapeutic agent for a disease involved by a sugar/lipid metabolism abnormality, a pancreas and/or pituitary function abnormality, or an insulin secretion and/or blood glucose level and/or ACTH secretion abnormality and the like.

More specifically, a compound that increases the amount of RAIG2 (or RAIG3) expressed can be used as a prophylactic/therapeutic agent for diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder and the like. Meanwhile, a compound that decreases the amount of RAIG2 (or RAIG3) expressed can be used as a prophylactic/therapeutic agent for obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers and the like.
When the compound is used as the above-described pharmaceutical, it can be prepared in the same manner as with RAIG2 (or RAIG3) above.
Because the preparation thus obtained is safe and less toxic, it can be administered to, for example, a mammal (for example, humans, rats, rabbits, sheep, swine, cattle, cats, dogs, monkeys and the like).
The dose of the compound or a salt thereof varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, a diabetic patient (assuming a 60 kg body weight). In parenteral administration, the single dose varies depending on the subject of administration, target organ, symptoms, method of administration and the like, for example, in the form of an injection, the dose is normally about 0.01 to 30 mg, preferably about 0.1 to 20 mg, and more preferably about 0.1 to 10 mg, per day, in, for example, a diabetic patient (assuming a 60 kg body weight). In cases where the subject of administration is other than a human, a dose corresponding to a human dose per 60 kg body weight can be administered.

[4] Quantitation of glucose and 1,5-AG

Because RAIG2 (or RAIG3) has the capability of binding to glucose and 1,5-AG, it allows quantitation of the concentrations of these sugars in vivo at high sensitivity.
The assay method of the present invention can, for example, in combination with a competitive method. Specifically, by bringing a sample into contact with RAIG2 (or RAIG3) or a partial peptide thereof, it is possible to determine the glucose or 1,5-AG concentration in the sample. Specifically, for example, the method of quantitation of the present invention can be used according to a method described in (1) or (2) below and the like or a method based thereon.

(1) Hiroshi Irie, ed., "Radioimmunoassay" (Kodansha Ltd., published in 1974)
(2) Hiroshi Irie, ed., "Sequel to the Radioimmunoassay" (Kodansha Ltd., 1979)

[5] Screening for a compound that alters the bindability of RAIG2 (or RAIG3) and glucose or 1,5-AG (agonist, antagonist and the like)
By either using RAIG2 (or RAIG3) or a partial peptide thereof, or constructing an expression system for recombinant RAIG2 (or RAIG3), and using an affinity assay system incorporating the expression system, it is possible to efficiently screen for a compound that alters the bindability of RAIG2 (or RAIG3) and a ligand (for example, peptide, protein, non-peptide compound, synthetic compound, fermentation product and the like) or a salt thereof.
Such compounds include (a) a compound having a cell stimulatory activity (for example, GTP-GDP exchange reaction promotion in coupled G$\alpha$, intracellular cAMP production regulation, inositol phosphate production regulation, intracellular $Ca^{2+}$ uptake, cGMP production regulation, insulin secretion promotion, blood glucose reduction, ACTH secretion regulation, appetite regulation and the like) via RAIG2 (or RAIG3) (agonist), (b) a compound having no cell stimulatory activity (antagonist), (c) a compound that enhances the binding force of RAIG2 (or RAIG3) and a ligand, or (d) a compound that decreases the binding force of RAIG2 (or RAIG3) and masculine and the like.
Accordingly, the present invention provides a screening method for a compound that alters the bindability of RAIG2 (or RAIG3) and glucose (or 1,5-AG) or a salt thereof, comprising comparing the values obtained (i) when RAIG2 (or RAIG3) and glucose (or 1,5-AG) are brought into contact with each other, and (ii) when RAIG2 (or RAIG3) and glucose (or 1,5-AG) and a test compound are brought into contact with each other.
In the above-described screening method, the amount of glucose (or 1,5-AG) bound to RAIG2 (or RAIG3), a cell stimulatory activity and the like are measured in cases (i) and (ii), and the values obtained are compared.
More specifically, the present invention provides:

(1) a screening method for a compound that alters the bindability of RAIG2 (or RAIG3) and glucose (or 1,5-AG) or a salt thereof, comprising measuring and comparing the amounts of labeled glucose (or 1,5-AG) bound to RAIG2 (or RAIG3), obtained when the labeled glucose (or 1,5-AG) is brought into contact with RAIG2 (or RAIG3) and when the labeled glucose (or 1,5-AG) and a test compound are brought into contact with RAIG2 (or RAIG3),

(2) a screening method for a compound that alters the bindability of RAIG2 (or RAIG3) and glucose (or 1,5-AG) or a salt thereof, comprising measuring and comparing the amounts of labeled glucose (or 1,5-AG) bound to a cell that produces RAIG2 (or RAIG3) or a membrane fraction thereof, obtained when the labeled glucose (or 1,5-AG) is brought into contact with the cell or membrane fraction thereof and when the labeled glucose (or 1,5-AG) and a test compound are brought into contact with the cell that produces RAIG2 (or RAIG3) or membrane fraction thereof,

(3) a screening method for a compound that alters the bindability of RAIG2 (or RAIG3) and glucose (or 1,5-AG) or a salt thereof, comprising measuring and comparing the amounts of labeled glucose (or 1,5-AG) to RAIG2 (or RAIG3), obtained when the labeled glucose (or 1,5-AG) is brought into contact with RAIG2 (or RAIG3) expressed on the cell membrane by culturing a transformant comprising the DNA of the present invention, and when the labeled glucose (or 1,5-AG) and a test compound are brought into contact with RAIG2 (or RAIG3) expressed on the cell membrane by culturing the transformant comprising the DNA of the present invention,

(4) a screening method for a compound that alters the bindability of RAIG2 (or RAIG3) and glucose (or 1,5-AG) or a salt thereof, comprising measuring and comparing a cell stimulatory activity (for example, GTP-GDP exchange reaction promotion in coupled Gα, intracellular cAMP production regulation, inositol phosphate production regulation, intracellular $Ca^{2+}$ uptake, cGMP production regulation, insulin secretion promotion, blood glucose reduction, ACTH secretion regulation, appetite regulation and the like) via RAIG2 (or RAIG3), obtained when the glucose (or 1,5-AG) is brought into contact with a cell expressing RAIG2 (or RAIG3) on the cell membrane, and when the glucose (or 1,5-AG) and a test compound are brought into contact with the cell expressing RAIG2 (or RAIG3) on the cell membrane, and

(5) a screening method for a compound that alters the bindability of RAIG2 (or RAIG3) and glucose (or 1,5-AG) or a salt thereof, comprising measuring and comparing a cell stimulatory activity (for example, GTP-GDP exchange reaction promotion in coupled Gα, intracellular cAMP production regulation, inositol phosphate production regulation, intracellular $Ca^{2+}$ uptake, cGMP production regulation, insulin secretion promotion, blood glucose reduction, ACTH secretion regulation, appetite regulation and the like) via RAIG2 (or RAIG3), obtained when the glucose (or 1,5-AG) is brought into contact with RAIG2 (or RAIG3) expressed on the cell membrane by culturing a transformant comprising the DNA of the present invention, and when the glucose (or 1,5-AG) and a test compound are brought into contact with RAIG2 (or RAIG3) expressed on the cell membrane by culturing the transformant comprising the DNA of the present invention.

The screening method of the present invention is hereinafter described specifically.
First, the RAIG2 (or RAIG3) used in the screening method of the present invention may be any one comprising the above-described RAIG2 (or RAIG3) or a partial peptide thereof, and the cell membrane fraction of an organ of an SS169-producing mammal is preferred. However, because human-derived organs, in particular, are extremely difficult to obtain, the RAIG2 (or RAIG3) used in the screening method of the present invention is preferably a human-derived RAIG2 (or RAIG3) expressed in a large amount using a transformant.
RAIG2 (or RAIG3) is produced using the method described above, which is preferably performed by expressing the DNA of the present invention in mammalian cells or insect cells. A cDNA is normally used as the DNA fragment encoding the desired portion of the protein, but is not construed as limiting. For example, a gene fragment or a synthetic DNA may also be used. For introducing a DNA fragment encoding RAIG2 (or RAIG3) or a partial peptide thereof into host animal (or insect) cells and efficiently expressing the same, it is preferable to insert the DNA fragment downstream of an SV40-derived promoter, a retrovirus promoter, a metallothionein promoter, a human heat shock promoter, a cytomegalovirus promoter, an SRα promoter, the polyhedrin promoter of nuclear polyhedrosis virus (NPV), which is a baculovirus having insect hosts, or the like.
Therefore, the RAIG2 (or RAIG3) used in the screening method of the present invention may be RAIG2 (or RAIG3) purified according to a method known per se, or may be used as cells producing the RAIG2 (or RAIG3) or a cell membrane fraction thereof.
In the above-described screening method, when using cells producing RAIG2 (or RAIG3), the cells may be fixed with glutaraldehyde, formalin and the like. This fixation can be performed according to a method known per se.
Cells producing RAIG2 (or RAIG3) refer to host cells expressing the RAIG2 (or RAIG3); as the host cells, yeasts, insect cells, animal cells and the like can be used.
The aforementioned cell membrane fraction refers to a fraction rich in cell membrane obtained by a method known per se after cell disruption. As the method of cell disruption, cell squashing using a Potter-Elvehjem homogenizer, disruption using a Waring blender or Polytron (manufactured by Kinematica Inc.), disruption by ultrasonication, disruption by cell spraying through a thin nozzle under an increased pressure using a French press or the like, and the like can be mentioned. For cell membrane fractionation, fractionation by centrifugal forces, such as fractional centrifugation or density gradient centrifugation, is mainly used. For example, a cell disruption fluid is centrifuged at a low speed (500 rpm to 3000 rpm) for a short time (normally about 1 to 10 minutes), the supernatant is centrifuged at a higher speed (15000 rpm to 30000 rpm) normally for 30 minutes to 2 hours, and the precipitate obtained is used as the membrane fraction. The membrane fraction is rich in the RAIG2 (or RAIG3) expressed and cell-derived membrane components such as phospholipids and membrane proteins.

The amount of RAIG2 (or RAIG3) in the cells producing the RAIG2 (or RAIG3) and the membrane fraction thereof is preferably $10^3$ to $10^8$ molecules per cell, more preferably $10^5$ to $10^7$ molecules. Note that as the expression amount increases, glucose (or 1,5 AG)-binding activity per unit of membrane fraction (specific activity) increases so that it is possible to construct a highly sensitive screening system, and to assay a large number of samples in the same lot.

To perform methods (1) to (3) above for screening a compound that changes the bindability between RAIG2 (or RAIG3) and glucose (or 1,5 AG), for example, an appropriate RAIG2 (or RAIG3)-containing membrane fraction and labeled glucose (or 1,5 AG) are necessary.

As the RAIG2 (or RAIG3)-containing membrane fraction, a naturally occurring RAIG2 (or RAIG3)-containing membrane fraction or a recombinant RAIG2 (or RAIG3)-containing membrane fraction having an activity equivalent to that thereof, or the like is desirable. As used herein, equivalent activity means equivalent glucose (or 1,5 AG)-binding activity, signal transmission activity or the like.

Examples of labeled glucose (or 1,5-AG) used include those labeled with, for example, [$^3$H], [$^{125}$I], [$^{14}$C], and the like by a conventional method.

Specifically, to perform screening of a compound that changes the bindability between RAIG2 (or RAIG3) and glucose (or 1,5 AG), cells producing RAIG2 (or RAIG3) or a membrane fraction thereof is first suspended in a buffer suitable for the screening to prepare a standard preparation of RAIG2 (or RAIG3). The buffer may be any buffer that does not interfere with the binding of RAIG2 (or RAIG3) and glucose (or 1,5 AG), such as a phosphate buffer or Tris-hydrochloride buffer having a pH value of 4 to 10 (desirably a pH value of 6 to 8). To reduce non-specific binding, a surfactant such as CHAPS, Tween-80™ (Kao-Atlas Co.), digitonin or deoxycholate may be added to the buffer. Furthermore, to suppress the degradation of receptors and ligands by proteases, a protease inhibitor such as PMSF, leupeptin, E-64 (manufactured by Peptide Research Laboratory, Co.), or pepstatin may be added. A given amount (5,000 cpm to 500,000 cpm) of a labeled glucose (or 1,5 AG) is added to 0.01 ml to 10 ml of a suspension of RAIG2 (or RAIG3), in the presence of a $10^{-4}$M to $10^{-10}$M test compound. To determine non-specific binding (NSB), a reaction tube containing the unlabeled ligand in large excess is also provided. The reaction is performed at about 0°C to 50°C, desirably about 4°C to 37°C, for about 20 minutes to 24 hours, desirably about 30 minutes to 3 hours. After the reaction, the reaction mixture is filtered through glass fiber filter paper and the like, and washed with an appropriate amount of the same buffer; thereafter, the residual radioactivity on the glass fiber filter paper is counted using a liquid scintillation counter or a $\gamma$-counter. A test compound showing a specific binding (B-NSB) of, for example, not more than 50%, as the percent ratio to the count ($B_0$-NSB) calculated by subtracting non-specific binding (NSB) from the count in the absence of antagonizing substance ($B_0$), can be selected as a candidate substance capable of inhibiting the antagonism.

For embodying the above-described methods (4) to (5) of screening for a compound that alters the bindability of RAIG2 (or RAIG3) and glucose (or 1,5-AG), for example, a cell stimulatory activity via RAIG2 (or RAIG3) (for example, GTP-GDP exchange reaction promotion in coupled G$\alpha$, intracellular cAMP production regulation, inositol phosphate production regulation, intracellular $Ca^{2+}$ uptake, cGMP production regulation, insulin secretion promotion, blood glucose reduction, ACTH secretion regulation, appetite regulation and the like) can be measured using a publicly known method or a commercially available measuring kit.

Specifically, first, cells producing RAIG2 (or RAIG3) are cultured on a multi-well plate or the like. In performing the screening, the medium is replaced with a fresh medium or an appropriate non-cytotoxic buffer in advance, and a test compound and the like are added, followed by incubation for a given time; thereafter, the cells are extracted or the supernatant is recovered, and the resulting product is quantified by each method. If it is difficult to detect the production of an indicator substance for cell stimulation activity due to a degrading enzyme contained in the cells, the assay may be performed with the addition of an inhibitor of the degrading enzyme. Activities such as cAMP production suppression can be detected as suppressive effects on the production of cells having baseline production previously increased with forskolin or the like.

To perform the screening with a measurement of a cell stimulation activity, appropriate cells expressing RAIG2 (or RAIG3) on the membrane thereof are necessary. As the cells expressing RAIG2 (or RAIG3), cell lines producing naturally occurring RAIG2 (or RAIG3), cell lines expressing the aforementioned recombinant RAIG2 (or RAIG3) and the like are desirable.

As examples of the test compound used, peptides, proteins, non-peptide compounds, synthetic compounds, fermentation products, cell extracts, plant extracts, animal tissue extracts and the like can be mentioned; these compounds may be novel compounds or known compounds.

The screening kit for a compound that alters the bindability of RAIG2 (or RAIG3) and glucose (or 1,5-AG) or a salt thereof comprises RAIG2 (or RAIG3), cells that produce RAIG2 (or RAIG3) or a membrane fraction thereof and the like. As examples of the screening kit of the present invention, the following can be mentioned.

1. Screening reagents

(1) Assay buffer solution and wash buffer solution

Hanks' balanced salt solution (manufactured by Gibco Co.) supplemented with 0.05% bovine serum albumin (manufactured by Sigma Co.).
The solution is sterilized by filtration through a filter of 0.45 μm pore size and stored at 4°C, or may be prepared freshly just before use.

(2) Standard preparation of RAIG2 (or RAIG3)

CHO cells expressing RAIG2 (or RAIG3) are subcultured on a 12-well plate at a density of 5 x $10^5$ cells/well and cultured at 37°C under 5% $CO_2$ and 95% air for 2 days.

(3) Labeled glucose (or 1,5 AG)

A commercially available glucose (or 1,5 AG) labeled with [$^3$H], [$^{125}$I], [$^{14}$C] or the like.
An aqueous solution of the glucose (or 1,5 AG) is stored at 4°C or -20°C and diluted to 1 μM with the assay buffer solution just before use.

(4) Standard solution of glucose (or 1,5 AG)

Musclin is dissolved in PBS containing 0.1% bovine serum albumin (manufactured by Sigma Co.) to obtain a concentration of 1 mM, and stored at -20°C.
2. Assay method

(1) After RAIG2 (or RAIG3)-expressing CHO cells cultured on a 12-well tissue culture plate are washed twice with 1 ml of the assay buffer solution, 490 μl of the assay buffer solution is added to each well.
(2) After 5 μl of $10^{-3}$ to $10^{-10}$M test compound solution is added, 5 μl of a labeled musclin is added and the reaction is performed at room temperature for 1 hour. To determine non-specific binding, 5 μl of $10^{-3}$M standard musclin solution, instead of the test compound, is added in advance.
(3) The reaction liquid is removed and the wells are washed 3 times with 1 ml of the wash buffer solution. The labeled musclin bound to the cells (or plate) is dissolved in 0.2N NaOH-1% SDS, and mixed with 4 ml of liquid scintillator A (manufactured by Wako Pure Chemical Industries, Ltd.).
(4) Radioactivity is measured using a liquid scintillation counter (manufactured by Beckman Co.), and percent maximum binding (PMB) is calculated using the following equation.

[Equation 1]

$$PMB = [(B - NSB)/(B_0 - NSB)] \times 100$$

PMB: percent maximum binding
B: value obtained in the presence of test compound
NSB: non-specific binding
$B_0$: maximum binding
In another preferred embodiment, the cell that expresses RAIG2 (or RAIG3) on the cell membrane further expresses coupled Gα. The Gα family that couples with RAIG2 (or RAIG3), respectively, can be identified by determining whether or not the signal transduction action of RAIG2 (or RAIG3) is activated when the ligand glucose (or 1,5-AG) is allowed to act thereon. Preferably, as the cell that expresses RAIG2 (or RAIG3) and coupled Gα thereof, a transformant cell co-transfected with the DNA of the present invention and a DNA that encodes the coupled Gα can be mentioned.
When glucose (or 1,5-AG) binds to RAIG2 (or RAIG3), the Gα activation domain of the receptor and the receptor binding region of the coupled Gα interact to cause a conformational change in the Gα, resulting in the dissociation of GDP from the GTP/GDP binding region and immediate binding of GTP. Gα-GTP acts on an effector to promote or suppress the activity thereof. Meanwhile, when an antagonist binds, no conformational change in the receptor occurs so that the Gα activation domain remains in the inactive state; therefore, the activated Gα-GTP level decreases, and the action on an effector is inhibited. Here, provided that a GTP analogue that does not undergo hydrolysis by the GTPase activity of $G\alpha$, such as $^{35}$S-labeled GTP$\gamma$S, for example, has been added to the system, in place of GTP, it is possible to evaluate the effect of a test compound on the GDP-GTP exchange reaction in $G\alpha$, and to screen for a substance that possesses RAIG2 (or RAIG3) agonist/antagonist activity in the presence and absence of the test compound, by measuring and comparing membrane-bound radioactivity levels. Hence, if the radioactivity increases in the presence of a test compound, the test compound has agonist activity for RAIG2 (or RAIG3), and if the radioactivity decreases, the test compound has antagonist activity for RAIG2 (or RAIG3).

In another preferred embodiment, provided is a screening method comprising comparing the activity levels of an effector that interacts with coupled $G\alpha$ in the presence and absence of a test compound, in a co-expression system for RAIG2 (or RAIG3) and the coupled $G\alpha$. Therefore, a cell that expresses RAIG2 (or RAIG3) and coupled $G\alpha$ thereof further expresses an effector that accepts signals from the $G\alpha$. $G\alpha$ ($G\alpha_s$), belonging to the $G_s$ family, promotes the activity of adenylate cyclase as the effector, and is exemplified by $G\alpha_{s-1}$ - $G\alpha_{s-4}$ and $G_{olf}$. $G\alpha$ ($G\alpha_i$), belonging to the $G_i$ family, suppresses the activity of adenylate cyclase as the effector, and is exemplified by $Ga_{i-1}$ - $Ga_{i-3}$ and $G_z$. $G\alpha$ ($G\alpha_q$), belonging to the Gq family, promotes the activity of phospholipase C as the effector, and is exemplified by Gq and $G_{16}$.

In a screening system comprising adenylate cyclase (hereinafter also referred to as AC) as the effector, the action of $G\alpha$ on the effector can be evaluated by directly determining the AC activity. The AC activity can be determined using any commonly known technique; examples of useful methods include, but are not limited to, a method comprising adding ATP to an AC-containing membrane fraction, and determining the resulting cAMP content by competitive immunoassay using an anti-cAMP antibody in the presence of cAMP labeled with a RI (e.g., $^{125}$I), an enzyme (e.g., alkaline phosphatase, peroxidase, etc.), a fluorescent substance (e.g., FITC, rhodamine, etc.), or the like, and a method comprising adding [$\alpha$-$^{32}$P] ATP to an AC-containing membrane, separating the resulting [$^{32}$P] cAMP using an alumina column etc., and subsequently determining the radioactivity thereof. When the coupled $G\alpha$ is $G\alpha_s$, AC activity levels are measured and compared in the presence and absence of a test compound; if the AC activity increases in the presence of the test compound, the test compound has agonist activity for RAIG2 (or RAIG3), and conversely, if the AC activity decreases, the test compound has antagonist activity for RAIG2 (or RAIG3). Meanwhile, when the coupled $G\alpha$ is $G\alpha_i$, if the AC activity increases in the presence of a test compound, the test compound has antagonist activity for RAIG2 (or RAIG3), and conversely, if the AC activity decreases, the test compound has agonist activity for RAIG2 (or RAIG3).

When using an intact eukaryotic cell as the screening system, the action of $G\alpha$ on AC can also be evaluated by determining the intracellular cAMP content, or labeling the cell with [$^3$H] adenine, and determining the radioactivity of resulting [$^3$H] cAMP. Although the intracellular cAMP content can be determined by incubating cells in the presence and absence of the test compound for an appropriate time, subsequently disrupting the cells, and subjecting the thus-obtained extract by the aforementioned competitive immunoassay, any other commonly known method can be used.

In another embodiment, the cAMP content may be evaluated by determining the amount of expression of reporter gene under the control of the cAMP-response element (CRE). In other words, the intracellular cAMP content is determined by culturing a eukaryotic cell incorporating a vector containing an expression cassette with a DNA that encodes the reporter protein (e.g., luciferase, GFP, peroxidase, alkaline phosphatase, etc.) linked downstream of a CRE-containing promoter, in the presence and absence of the test compound for an comparing the expression of the reporter gene in the thus-obtained extract using a commonly known technique.

Therefore, when the coupled $G\alpha$ is Gas, if the intracellular cAMP content (or the amount of reporter gene under the control of CRE expressed) increases in the presence of a test compound, the test compound has agonist activity for RAIG2 (or RAIG3), and conversely, if the cAMP content (or the amount of reporter gene expressed) decreases, the test compound has antagonist activity for RAIG2 (or RAIG3). Meanwhile, when the coupled $G\alpha$ is $G\alpha_i$, if the intracellular cAMP content (or the amount of reporter gene under the control of CRE expressed) increases in the presence of a test compound, the test compound has antagonist activity for RAIG2 (or RAIG3), and conversely, if the cAMP content (or the amount of reporter gene expressed) decreases, the test compound has agonist activity for RAIG2 (or RAIG3) .

On the other hand, in a screening system containing phospholipase C (hereinafter also referred to as PLC) as the effector (i.e., a case wherein the coupled $G\alpha$ polypeptide is $G\alpha_q$), the action of $G\alpha$ on the effector can be evaluated by directly determining the PLC activity. The PLC activity can, for example, be evaluated by adding $^3$H-labeled phosphatidylinositol 4,5-diphosphate to a PLC-containing membrane fraction, and determining the amount of inositol phosphate produced using a commonly known technique. PLC activity levels are measured and compared in the presence and absence of a test compound; if the PLC activity increases in the presence of the test compound, the test compound has agonist activity for RAIG2 (or RAIG3), and conversely, if the PLC activity decreases, the test compound has antagonist activity for RAIG2 (or RAIG3).

When using an intact eukaryotic cell as the screening system, the action of $G\alpha$ on PLC can also be evaluated by adding [$^3$H] labeled inositol to the cell and determining the radioactivity of the resulting [$^3$H] labeled inositol phosphate, or determining the intracellular $Ca^{2+}$ content. Although the intracellular $Ca^{2+}$ content can be determined by spectroscopy using a fluorescent probe (fura-2,indo-1,fluor-3,Calcium-Green I etc.) or by using a calcium-sensitive luminescent protein such as aequorin after the cells are incubated for a given time in the presence and absence of the test compound, any other commonly known method can be used. As an apparatus suitable for spectroscopy using a fluorescent probe, there may be mentioned the FLIPR (Molecular Devices Company) system.

In another embodiment, the $Ca^{2+}$ content may be evaluated by determining the amount of expression of reporter gene under the control of $Ca^{2+}$-upregulated TPA (12-O-tetradecanoylphorbol-13-acetate)-responsive element (TRE). In other words, the intracellular $Ca^{2+}$ content is determined by culturing a eukaryotic cell incorporating a vector containing an expression cassette with a DNA that encodes the reporter protein (e.g., luciferase, GFP, peroxidase, alkaline phosphatase etc.) linked downstream of a TRE-containing promoter, in the presence and absence of the test compound for an appropriate time, disrupting the cells, and measuring and comparing the expression of the reporter gene in the thus-obtained extract using a commonly known technique.
Therefore, if the intracellular $Ca^{2+}$ content (or the amount of reporter gene under the control of TRE expressed) increases in the presence of a test compound, the test compound has agonist activity for RAIG2 (or RAIG3), and conversely, if the intracellular $Ca^{2+}$ content (or the amount of reporter gene expressed) decreases, the test compound has antagonist activity for RAIG2 (or RAIG3).

By allowing a chimeric G$\alpha$ wherein the region that interacts with the AC of the G$\alpha_s$ and G$\alpha_i$ polypeptides (about 5 amino acids at the C-terminus) is replaced with the sequence of G$\alpha_q$ to be expressed in a eukaryotic cell, whatever the coupled G$\alpha$ of RAIG2 (or RAIG3) is, it is possible to screen for RAIG2 (or RAIG3) agonists/antagonists using phospholipase C as the effector.

In another preferred embodiment, as described in the aforementioned patent document 1, by allowing a fusion protein wherein RAIG2 (or RAIG3) has coupled G$\alpha$ joined to the C-terminal side thereof to be expressed in a host eukaryotic cell such as a mammalian cell or insect cell, RAIG2 (or RAIG3) can be activated permanently. In this case, it is possible to screen for agonists and inverse agonists without using a ligand such as glucose or 1,5-AG.

In still another preferred embodiment, yeast (for example, baker's yeast and the like) is used as the host cell. Yeast has STE2, a receptor of conjugation pheromone $\alpha$ factor, as the only GPCR. Upon stimulation by a ligand, STE2 activates GPA1 being a coupled G$\alpha$ and dissociates from G$\beta$/G$\gamma$. FUS1 expression is induced by the kinase cascade signal from the dissociated G$\beta$/G$\gamma$, and conjugation occurs. Here, if STE2 is destroyed, and RAIG2 (or RAIG3) is expressed in yeast instead, and RAIG2 (or RAIG3) coupled G$\alpha$ is co-expressed under the control of the GPA1 promoter, RAIG2 (or RAIG3), upon stimulation by glucose (or 1,5-AG), mediates intracellular signal transduction just like STE2. Here, if a G$\alpha$ that couples with RATG2 (nr BAIG3) is co-expressed under the control of the GPA1 promoter, and fused with a gene that confers an auxotrophic mutation to FUS1 or a reporter gene (for example, LacZ) and the like, signal activation can easily be detected. Details of this method are given in, for example, Yeast, 16: 11-22 (2000) and the like.

As described above, RAIG2 (or RAIG3) senses glucose (or 1,5-AG) concentrations, promotes insulin secretion from pancreatic $\beta$ cells, and controls CRF-stimulated ACTH secretion from pituitary cells. Therefore, by comparing insulin secretion or ACTH secretion levels obtained when insulin-secreting cells or ACTH-secreting cells having RAIG2 (or RAIG3) expressed forcibly therein are cultured in the presence of glucose (or 1,5-AG) and when the same are cultured in the presence of both glucose (or 1,5-AG) and a test compound, it is possible to screen for agonists and antagonists of RAIG2 (or RAIG3).
If insulin secretion is promoted in the presence of a test compound, the test compound can be selected as an agonist of RAIG2 (or RAIG3); conversely, if insulin secretion is inhibited, the test compound can be selected as an antagonist of RAIG2 (or RAIG3). Meanwhile, at low glucose (or 1,5-AG) concentrations, if ACTH secretion is promoted in the presence of the test compound, the test compound can be selected as an agonist of RAIG2 (or RAIG3); conversely, if ACTH secretion is inhibited, the test compound can be selected as an antagonist of RAIG2 (or RAIG3). At high glucose (or 1,5-AG) concentrations, if ACTH secretion is inhibited in the presence of the test compound, the test compound can be selected as an agonist of RAIG2 (or RAIG3); conversely, if ACTH secretion is promoted, the test compound can be selected as an antagonist of RAIG2 (or RAIG3).

The compound obtained using the above-described screening method or screening kit or a salt thereof is a compound having an action to alter the bindability of RAIG2 (or RAIG3) and glucose (or 1,5-AG); specifically, the compound is (a) a compound having a cell stimulatory activity (for example, GTP-GDP exchange reaction promotion in coupled G$\alpha$, intracellular cAMP production regulation, inositol phosphate production regulation, intracellular $Ca^{2+}$ uptake, cGMP production regulation, insulin secretion promotion, blood glucose reduction, ACTH secretion regulation, appetite regulation and the like) via glucose (or 1,5-AG)-RAIG2 (or RAIG3) interaction (agonist), (b) a compound having no cell stimulatory activity (antagonist), (c) a compound that enhances the binding force of RAIG2 (or RAIG3) and glucose (or 1,5-AG), or (d) a compound that decreases the binding force of RAIG2 (or RAIG3) and glucose (or 1,5-AG).
As the compound used, peptides, proteins, non-peptide compounds, synthetic compounds, fermentation products and the like can be mentioned; these compounds may be novel compounds or known compounds.
An agonist for RAIG2 (or RAIG3) is useful as a safe pharmaceutical of low toxicity that enhances glucose (or 1,5-AG) activity because it has the same action as the physiological activity of glucose (or 1,5-AG) on RAIG2 (or RAIG3). An antagonist for RAIG2 (or RAIG3) is useful as a safe pharmaceutical of low toxicity that suppresses glucose (or 1,5-AG) activity because it is capable of suppressing the physiological activity of glucose (or 1,5-AG) on RAIG2 (or RAIG3). A compound that enhances the binding force of RAIG2 (or RAIG3) and glucose (or 1,5-AG) is useful as a safe pharmaceutical of low toxicity that enhances the physiological activity of glucose (or 1,5-AG) on RAIG2 (or RAIG3). A compound that weakens the bindability of RAIG2 (or RAIG3) and glucose (or 1,5-AG) is useful as a safe pharmaceutical of low toxicity that decreases the physiological activity possessed by glucose (or 1,5-AG) on RAIG2 (or RAIG3).

[6] Prophylactic/therapeutic agents for various diseases, comprising a compound that alters the bindability of RAIG2 (or RAIG3) and glucose (or 1,5-AG) (agonist, antagonist)

RAIG2 (or RAIG3), as described above, functions as a receptor of glucose (or 1,5-AG) to sense blood glucose concentrations, promote insulin secretion in the pancreas and regulate CRF-stimulated ACTH secretion in the pituitary according to glucose concentration. Therefore, a compound that alters the bindability of RAIG2 (or RAIG3) and glucose (or 1,5-AG) (agonist, antagonist) can be used as 1) a regulator of sugar/lipid metabolism, 2) a regulator of pancreas and/or pituitary function, 3) a regulator of insulin secretion and/or blood glucose and/or ACTH secretion, 4) a prophylactic/therapeutic agent for a disease involved by a sugar/lipid metabolism abnormality, a pancreas and/or pituitary function abnormality, or an insulin secretion and/or blood glucose level and/or ACTH secretion abnormality and the like.

More specifically, an agonist of RAIG2 (or RAIG3) can be used as a prophylactic/therapeutic agent for diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder and the like. Meanwhile, an antagonist of RAIG2 (or RAIG3) can be used as a prophylactic/therapeutic agent for obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers and the like.

Because the preparation thus obtained is safe and less toxic, it can be administered to, for example, mammals (for example, humans, rats, rabbits, sheep, swine, cattle, cats, dogs, monkeys and the like).

The dose of agonist of RAIG 2 (or RAIG 3) varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, a diabetic patient (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, a diabetic patient (assuming a 60 kg body weight). In cases where the subject of administration is other than a human, a dose corresponding to a human dose per 60 kg body weight can be administered.

The dose of antagonist of RAIG 2 (or RAIG 3) varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, an obese patient (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, an obese patient (assuming a 60 kg body weight). In cases where the subject of administration is other than a human, a dose corresponding to a human dose per 60 kg body weight can be administered.

[7] Quantitation of RAIG 2 (or RAIG 3)

Because the antibody of the present invention is capable of specifically recognizing RAIG 2 (or RAIG 3), it can be used for quantitation of RAIG 2 (or RAIG 3) in a test fluid, particularly quantitation by sandwich immunoassay, and the like. Accordingly, the present invention provides, for example, (i) a method of quantifying RAIG 2 (or RAIG 3) in a test fluid, which comprises competitively reacting the antibody of the present invention and the test fluid, a labeled protein and the like, and measuring the ratio of labeled protein and the like bound to the antibody, and (ii) a method of quantifying RAIG 2 (or RAIG 3) in a test fluid, which comprises simultaneously or sequentially reacting the test fluid, the antibody of the present invention as immobilized on a carrier, and the antibody of the present invention as labeled, and measuring the activity of labeling agent on the insolubilizing carrier.

In (ii) above, the insolubilized antibody and the labeled antibody preferably have antigen recognition sites such that they do not mutually interfere with their binding to RAIG 2 (or RAIG 3) (for example, one antibody recognizes the N-terminus of the RAIG 2 (or RAIG 3), and the other antibody reacts with the C-terminus of the RAIG 2 (or RAIG 3), and the like).

Using a monoclonal antibody against RAIG 2 (or RAIG 3) (hereinafter sometimes referred to as the monoclonal antibody of the present invention), the RAIG 2 (or RAIG 3) can be measured, and can also be detected by tissue staining and the like. For these purposes, the antibody molecule itself may be used, and the F(ab')2, Fab' or Fab fraction of the antibody molecule may also be used. The assay method using an antibody against RAIG 2 (or RAIG 3) is not to be subject to limitation; any assay method can be used, as long as the amount of antibody, antigen, or antibody-antigen complex corresponding to the amount of antigen (for example, the amount of RAIG 2 (or RAIG 3)) in a test fluid is detected by a chemical or physical means and calculated from a standard curve generated using standard solutions containing known amounts of antigen. For example, nephelometry, the competitive method, immunometric method, and sandwich method are advantageously used, with preference given to the sandwich method described below, in terms of sensitivity and specificity.

As examples of the labeling agent used for the assay method using a labeled substance, radioisotopes, enzymes, fluorescent substances, luminescent substances and the like can be mentioned. As examples of the radioisotopes used, [$^{125}$I], [$^{131}$I], [$^{3}$H], [$^{14}$C] and the like can be mentioned. As examples of the enzymes, stable enzymes of high specific activity are preferred; for example, β-galactosidase, β-glucosidase, alkaline phosphatase, peroxidase, malate dehydrogenase and the like can be mentioned. As examples of the fluorescent substances, fluorescamine, fluorescein isothiocyanate and the like can be mentioned. As examples of the luminescent substances, luminol, luminol derivatives, luciferin, lucigenin and the like can be mentioned. Furthermore, the biotin-avidin system may also be used for the binding of an antibody or antigen and the labeling agent.

For insolubilization of the antigen or antibody, physical adsorption may be used, and methods based on chemical binding, which are normally used to insolubilize or immobilize proteins or enzymes and the like, may also be used. As examples of the carrier, insoluble polysaccharides such as agarose and dextran; synthetic resins such as polystyrene, polyacrylamide and silicone; glass and the like can be used.

In the sandwich method, the monoclonal antibody of the present invention as insolubilized is reacted with a test fluid (primary reaction), and further reacted with the monoclonal antibody of the present invention as labeled (secondary reaction), and the activity of the labeling agent on the insolubilizing carrier is then measured, whereby the amount of RAIG 2 (or RAIG 3) in the test fluid can be quantified. The primary and secondary reactions may be performed in the reverse order, and may be performed simultaneously or at a time interval. The labeling agent and the method of insolubilization may be the same as those described above.

In immunoassays by the sandwich method, the antibody used for an immobilized antibody or a labeled antibody need not always be one kind; a mixture of two or more kinds of antibodies may be used to increase assay sensitivity.

In assaying RAIG 2 (or RAIG 3) by the sandwich method, the monoclonal antibodies of the present invention used for the primary and secondary reactions are preferably antibodies having mutually different sites for binding with the RAIG 2 (or RAIG 3). That is, regarding the antibodies used for the primary and secondary reactions, for example, when the antibody used for the secondary reaction recognizes the C-terminus region of the RAIG 2 (or RAIG 3), the antibody used for the primary reaction is preferably an antibody that recognizes a region other than the C-terminus region, for example, the N-terminus region.

The monoclonal antibody of the present invention can be used for assay systems other than the sandwich method, for example, the competitive method, immunometric method, nephelometry and the like. In the competitive method, an antigen in a test fluid and a labeled antigen are competitively reacted with an antibody, then the unreacted labeled antigen (F) and the labeled antigen bound to the antibody (B) are separated (B/F separation); the amount of labeled antigen in B or F is measured to quantify the amount of antigen in the test fluid. For this reaction, the liquid phase method, which uses a soluble antibody and polyethylene glycol and a secondary antibody to the above-described antibody and the like for B/F separation, and the immobilization method, which uses a soluble primary antibody and a solid-immobilized secondary antibody, are available.

In the immunometric method, an antigen in a test fluid and an immobilized antigen are competitively reacted with a given amount of labeled antibody, and thereafter the solid phase is separated from the liquid phase, or an antigen in a test fluid is reacted with an excess amount of labeled antibody, an immobilized antigen is then added to bind the unreacted labeled antibody to the solid phase, and the solid phase is separated from the liquid phase. Subsequently, the amount of labeled antibody in either phase is measured to quantify the amount of antigen in the test fluid.

In nephelometry, the amount of insoluble precipitate from the antigen-antibody reaction within the gel or in the solution is measured. Even when the amount of antigen in the test fluid is small and only a small amount of precipitate is obtained, laser nephelometry, which is based on laser scattering, and the like can be used advantageously.

In applying these individual immunoassays to the quantification of RAIG 2 (or RAIG 3), no special conditions, procedures or the like need to be set forth. The assay system for the RAIG 2 (or RAIG 3) may be constructed with ordinary technical considerations of those skilled in the art on ordinary conditions and procedures in the respective methods. For details of these general techniques, reference can be made to reviews, texts and the like [see, for example, Hiroshi Irie, ed., "Radioimmunoassay" (Kodansha Ltd., published in 1974), Hiroshi Irie, ed., "Sequel to the Radioimmunoassay" (Kodansha Ltd., published in 1979), Eiji Ishikawa et al., ed., "Enzyme Immonoassay" (Igakushoin, published in 1978), Eiji Ishikawa et al., ed., "Enzyme Immonoassay" (2nd ed.) (Igakushoin, published in 1982), Eiji Ishikawa et al., ed., "Enzyme Immonoassay" (3rd ed.) (Igakushoin, published in 1987), Methods in ENZYMOLOGY, Vol. 70 (Immunochemical Techniques (Part A)), ibidem, Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press Publishing) and the like].
As described above, RAIG 2 (or RAIG 3) can be quantified highly sensitively by using the antibody of the present invention. Furthermore, various diseases associated with RAIG 2 (or RAIG 3) hypofunction or hyperfunction can be diagnosed by quantifying RAIG 2 (or RAIG 3) in vivo using the antibody of the present invention.
The antibody of the present invention can also be used to specifically detect RAIG 2 (or RAIG 3) present in a test sample such as body fluid or tissue. The antibody of the present invention can also be used to prepare an antibody column for purification of RAIG 2 (or RAIG 3), to detect RAIG 2 (or RAIG 3) in each fraction during purification, to analyze the behavior of RAIG 2 (or RAIG 3) in subject cells, and the like.

[8] Screening a compound that changes the amount of RAIG 2 (or RAIG 3) in the cell membrane

Because the antibody of the present invention is capable of specifically recognizing RAIG 2 (or RAIG 3), it can be used for screening a compound that changes the amount of RAIG 2 (or RAIG 3) in the cell membrane.
Accordingly, the present invention provides, for example:

(i) a method of screening a compound that changes the amount of RAIG 2 (or RAIG 3) in the cell membrane, which comprises disrupting (1) blood, (2) a particular organ, or (3) a tissue, cells or the like isolated from an organ, of a non-human mammal, then isolating a cell membrane fraction, and quantifying the RAIG 2 (or RAIG 3) contained the cell membrane fraction,
(ii) a method of screening a compound that changes the amount of RAIG 2 (or RAIG 3) in the cell membrane, which comprises disrupting a transformant expressing RAIG 2 (or RAIG 3) or a partial peptide thereof, or the like, then isolating a cell membrane fraction, and quantifying the RAIG 2 (or RAIG 3) contained in the cell membrane fraction,
(iii) a method of screening a compound that changes the amount of RAIG 2 (or RAIG 3) in the cell membrane, which comprises identifying RAIG 2 (or RAIG 3) on the cell membrane by cutting into sections (1) blood, (2) a particular organ, or (3) a tissue, cells or the like isolated from an organ, of a non-human mammal, then quantifying the extent of staining of RAIG 2 (or RAIG 3) on the cell surface layer using an immunostaining technique, and
(iv) a method of screening a compound that changes the amount of RAIG 2 (or RAIG 3) species in the cell membrane, which comprises identifying the RAIG 2 (or RAIG 3) on the cell membrane by cutting into sections a transformant expressing RAIG 2 (or RAIG 3) or a partial peptide thereof, or the like, then quantifying the extent of staining of the RAIG 2 (or RAIG 3) on the cell surface layer using an immunostaining technique.

Quantitation of the RAIG 2 (or RAIG 3) contained in the cell membrane fraction is specifically performed as described below.

(i) A drug (for example, anti-obesity drugs, anti-diabetic drugs, antihypertensive drugs, vasoactive drugs, antidepressant agents and the like) or a physical stress (for example, soaking stress, electric shock, brightness/darkness, low temperatures and the like) or the like is given to a normal or disease model non-human mammal (for example, mice, rats, rabbits, sheep, swine, cattle, cats, dogs, monkeys and the like, more specifically, obese mice, diabetic mice, hypertensive rats, arteriosclerotic rabbits, depressive rats and the like); after a given time has elapsed, blood or a particular organ (for example, pancreas, pituitary and the like), a tissue (for example, pancreatic islet and the like) or cells (for example, pancreatic β cells, pituitary gland cells or the like) are obtained. The cells and the like obtained are suspended in an appropriate buffer solution (for example, Tris-HCl buffer solution, phosphate buffer solution, HEPES buffer solution and the like) and the like, and the cells or the like are disrupted using a surfactant (for example, Triton X100™, Tween 20™ and the like) and the like, and further treated using techniques such as centrifugation, filtration and column fractionation to yield a cell membrane fraction.

The cell membrane fraction refers to a fraction rich in cell membrane obtained by a method known per se after cell disruption. As the method of cell disruption, cell squashing using a Potter-Elvehjem homogenizer, disruption using a Waring blender or Polytron (manufactured by Kinematica Inc.), disruption by ultrasonication, disruption by cell spraying through a thin nozzle under an increased pressure using a French press or the like, and the like can be mentioned. For cell membrane fractionation, fractionation by centrifugal forces, such as fractional centrifugation or density gradient centrifugation, is mainly used. For example, a cell disruption fluid is centrifuged at a low speed (500 rpm to 3000 rpm) for a short time (normally about 1 to 10 minutes), the supernatant is centrifuged at a higher speed (15000 rpm to 30000 rpm) normally for 30 minutes to 2 hours, and the precipitate obtained is used as the membrane fraction. The membrane fraction is rich in RAIG 2 (or RAIG 3) and cell-derived membrane components such as phospholipids and membrane proteins.
The RAIG 2 (or RAIG 3) contained in the cell membrane fraction can be quantified by, for example, a sandwich immunoassay using the antibody of the present invention, Western blot analysis and the like.
The sandwich immunoassay can be performed in the same manner as the method described above, and Western blot can be performed by a means known per se.

(ii) A transformant expressing RAIG 2 (or RAIG 3) or a partial peptide thereof can be prepared according to the method described above, and the RAIG 2 (or RAIG 3) contained in the cell membrane fraction can be quantified.

Screening of a compound that changes the amount of RAIG 2 (or RAIG 3) in the cell membrane can be performed by:

(i) administering a test compound to a normal or disease model non-human mammal at a given time before (before 30 minutes to before 24 hours, preferably before 30 minutes to before 12 hours, more preferably before 1 hour to before 6 hours) or after (after 30 minutes to after 3 days, preferably after 1 hour to after 2 days, more preferably after 1 hour to after 24 hours) a drug, a physical stress or the like is given, or at the same time as the drug or physical stress, and quantifying the amount of RAIG 2 (or RAIG 3) in the cell membrane after a given time has elapsed after administration (after 30 minutes to after 3 days, preferably after 1 hour to after 2 days, more preferably after 1 hour to after 24 hours), and can also be preformed by:

(ii) mixing a test compound in a medium at the start of cultivation of a transformant according to a conventional method, and quantifying the amount of RAIG 2 (or RAIG 3) in the cell membrane after cultivation for a given time (after 1 day to after 7 days, preferably after 1 day to after 3 days, more preferably after 2 days to after 3 days).

Identification of the RAIG 2 (or RAIG 3) contained in the cell membrane fraction is specifically performed as described below.

(iii) A drug (for example, anti-obesity drugs, anti-diabetic drugs, antihypertensive drugs, vasoactive drugs, antidepressant agents and the like) or a physical stress (for example, soaking stress, electric shock, brightness/darkness, low temperatures and the like) or the like is given to a normal or disease model non-human mammal (for example, mice, rats, rabbits, sheep, swine, cattle, cats, dogs, monkeys and the like, more specifically, obese mice, diabetic mice, hypertensive rats, arteriosclerotic rabbits, depressive rats and the like); after a given time has elapsed, blood or a particular organ (for example, pancreas, pituitary and the like), a tissue (for example, pancreatic islet and the like) or cells (for example, pancreatic β cells, pituitary gland cells and the like) are obtained. The cells or the like obtained are cut into tissue sections according to a conventional method, and immunostaining is performed using the antibody of the present invention. The amount of RAIG 2 (or RAIG 3) in the cell membrane can be determined, quantitatively or qualitatively, by quantifying the extent of staining of RAIG 2 (or RAIG 3) on the cell surface layer to identify the RAIG 2 (or RAIG 3) on the cell membrane.

(iv) The RAIG 2 (or RAIG 3) contained in a cell membrane fraction can also be identified in a similar way using a transformant expressing SS169 or a partial peptide thereof and the like.

The compound obtained using the above-described screening method or a salt thereof is a compound having an action to alter the amount of RAIG2 (or RAIG3) in the cell membrane; specifically, the compound is (a) a compound that enhances a cell stimulatory activity via glucose (or 1,5-AG)-RAIG2 (or RAIG3) interaction (for example, GTP-GDP exchange reaction promotion in coupled Gα, intracellular cAMP production regulation, inositol phosphate production regulation, intracellular $Ca^{2+}$ uptake, cGMP production regulation, insulin secretion promotion, blood glucose reduction, ACTH secretion regulation, appetite regulation and the like) by increasing the amount of RAIG2 (or RAIG3) in the cell membrane, or (b) a compound that weakens the cell stimulatory activity by reducing the amount of RAIG2 (or RAIG3) in the cell membrane.
As the compound used, peptides, proteins, non-peptide compounds, synthetic compounds, fermentation products and the like can be mentioned; these compounds may be novel compounds or known compounds.
A compound that enhances the cell stimulation activity is useful as a safe and less toxic pharmaceutical for enhancing a physiological activity of RAIG 2 (or RAIG 3).
A compound that weakens the cell stimulation activity is useful as a safe and less toxic pharmaceutical for reducing a physiological activity of RAIG 2 (or RAIG 3).

[9] Prophylactic/therapeutic agent for various diseases, comprising a compound that alters the amount of RAIG2 (or RAIG3) in the cell membrane

As described above, RAIG2 (or RAIG3) functions as a receptor of glucose (or 1,5-AG) to sense blood glucose concentrations, promote insulin secretion in the pancreas and regulate CRF-stimulated ACTH secretion in the pituitary according to the glucose concentration. Therefore, a compound that alters the amount of RAIG2 (or RAIG3) in the cell membrane can be used as 1) a regulator of sugar/lipid metabolism, 2) a regulator of pancreas and/or pituitary function, 3) a regulator of insulin secretion and/or blood glucose and/or ACTH secretion, 4) a prophylactic/therapeutic agent for a disease involved by a sugar/lipid metabolism abnormality, a pancreas and/or pituitary function abnormality, or an insulin secretion and/or blood glucose level and/or ACTH secretion abnormality and the like.
More specifically, a compound that alters the amount of RAIG2 (or RAIG3) in the cell membrane can be used as a prophylactic/therapeutic agent for diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder and the like. Meanwhile, a compound that decreases the amount of RAIG2 (or RAIG3) in the cell membrane can be used as a prophylactic/therapeutic agent for obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers and the like.

Because the preparation thus obtained is safe and less toxic, it can be administered to, for example, mammals (for example, humans, rats, rabbits, sheep, swine, cattle, cats, dogs, monkeys and the like).
The dose of a compound that increases the amount of RAIG2 (or RAIG3) in the cell membrane varies depending on the subject of administration, target organ, symptoms, method of administration and the like; in oral administration, the dose is normally about 0.1 to 100 mg, preferably about 1.0 to 50 mg, more preferably about 1.0 to 20 mg, per day, in, for example, a diabetic patient (assuming a 60 kg body weight). In parenteral administration, the single dose varies depending on the subject of administration, target organ, symptoms, method of administration and the like; for example, in the form of an injection, it is advantageous to administer normally about 0.01 to 30 mg, preferably about 0.1 to 20 mg, more preferably about 0.1 to 10 mg, per day, in, for example, a diabetic patient (assuming a 60 kg body weight). In cases where the subject of administration is other than a human, a dose corresponding to a human dose per 60 kg body weight can be administered.
The dose of a compound that decreases the amount of RAIG2 (or RAIG3) in the cell membrane varies depending on the subject of administration, target organ, symptoms, method of administration and the like; in oral administration, the dose is normally about 0.1 to 100 mg, preferably about 1.0 to 50 mg, more preferably about 1.0 to 20 mg, per day, in, for example, an obese patient (assuming a 60 kg body weight. In parenteral administration, the single dose varies depending on the subject of administration, target organ, symptoms, method of administration and the like; for example, in the form of an injection, it is advantageous to administer normally about 0.01 to 30 mg, preferably about 0.1 to 20 mg, more preferably about 0.1 to 10 mg, per day, in, for example, an obese patient (assuming a 60 kg body weight). In cases where the subject of administration is other than a human, a dose corresponding to a human dose per 60 kg body weight can be administered.

[10] Preparation of non-human transgenic animal bearing a DNA encoding RAIG 2 (or RAIG 3)

The present invention provides a non-human mammal bearing a DNA encoding exogenous RAIG 2 (or RAIG 3) (hereinafter abbreviated as "the exogenous DNA of the present invention") or a variant DNA thereof (sometimes abbreviated as "the exogenous variant DNA of the present invention").
Accordingly, the present invention provides:

[1] a non-human mammal bearing the exogenous DNA of the present invention or a variant DNA thereof,
[2] the animal described in term [1], wherein the non-human mammal is a rodent,
[3] the animal described in term [2], wherein the rodent is a mouse or a rat, and
[4] a recombination vector comprising the exogenous DNA of the present invention or a variant DNA thereof, and expressible in a mammal.

A non-human mammal bearing the exogenous DNA of the present invention or a variant DNA thereof (hereinafter abbreviated as the DNA-transfected animal of the present invention) can be prepared by transferring a desired DNA into an unfertilized egg, a fertilized egg, a spermatozoon, a germinal cell (including its precursor cell) or the like, preferably in the embryogenic stage in the development of the non-human mammal (more preferably in the single-cell or fertilized-egg stage and generally before the 8-cell phase), by the calcium phosphate method, the electric pulse method, the lipofection method, the aggregation method, the microinjection method, the particle gun method, the DEAE-dextran method or the like. Also, it is possible to transfer the exogenous DNA of the present invention into a somatic cell, a living organ, a tissue cell or the like by the DNA transfer method, and utilize them for cell culture, tissue culture and the like, and these cells may be fused with the above-described germinal cells by a method of cell fusion known per se to create the DNA transgenic animal of the present invention.
As examples of the non-human mammal used, cattle, swine, sheep, goat, rabbits, dogs, cats, guinea pigs, hamsters, mice, rats and the like can be mentioned. In particular, preferred from the viewpoint of creating a pathologic animal model system are rodents, especially mice (for example, C57BL/6 line, DBA2 line and the like, which are pure lines,

$B6C3F_1$ line, $BDF_1$ line, $B6D2F_1$ line, BALB/c line, ICR line and the like, which are cross lines) or rats (for example, Wistar, SD and the like), since they have relatively short ontogeny and lifecycles and are easy to breed.
As "the mammal" in a recombination vector expressible in a mammal, humans and the like can be mentioned, in addition to the above-described non-human mammals.

The exogenous DNA of the present invention refers to the DNA of the present invention once isolated/extracted from a mammal, rather than the DNA of the present invention inherently possessed by a non-human mammal.
As the variant DNA of the present invention, those resulting from a variation (for example, mutations and the like) in the base sequence of the original DNA of the present invention, specifically DNAs having a base addition, deletion, substitution with another base or the like, and the like can be mentioned, and abnormal DNAs are included.
The abnormal DNA means a DNA that allows the expression of abnormal RAIG 2 (or RAIG 3), and is exemplified by a DNA that allows the expression of a protein that suppresses the function of normal RAIG 2 (or RAIG 3), and the like.
The exogenous DNA of the present invention may be derived from a mammal of any of the same species as, or a different species from, the subject animal. In transferring the DNA of the present invention into the subject animal, it is generally advantageous to use the DNA of the present invention as a DNA construct having the DNA bound downstream of a promoter capable of allowing the expression of the DNA in animal cells. For example, when the human DNA of the present invention is transferred, a DNA-transfected mammal that highly expresses the DNA of the present invention can be prepared by microinjecting a DNA construct (e.g., vector and the like) having the human DNA of the present invention ligated downstream of various promoters allowing the expression of a DNA derived from various mammals (for example, rabbits, dogs, cats, guinea pigs, hamsters, rats, mice and the like) bearing the DNA of the present invention, which is highly homologous to the human DNA, into a fertilized egg of the subject mammal, for example, a mouse fertilized egg.
As the expression vector for carrying the DNA of the present invention, *Escherichia* coli-derived plasmids, Bacillus subtilis-derived plasmids, yeast-derived plasmids, bacteriophages such as λ phage, retroviruses such as Moloney leukemia virus, animal viruses such as vaccinia virus and baculovirus, and the like can be used. In particular, *Escherichia coli*-derived plasmids, *Bacillus subtilis*-derived plasmids, yeast-derived plasmids and the like are preferably used.
As examples of the promoter that regulates the DNA expression, 1) promoters of DNA derived from viruses (e.g., simian virus, cytomegalovirus, Moloney mouse leukemia virus, JC virus, breast cancer virus, poliovirus and the like) and 2) promoters derived from various mammals (humans, rabbits, dogs, cats, guinea pigs, hamsters, rats, mice and the like), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase β I subunit, dystrophin, tartrate-resistant alkaline phosphatase, atrial natriuretic factor, endothelial receptor tyrosine kinase (generally abbreviated as Tie2), sodium-potassium ATPase (Na, K-ATPase), neurofilament light chain, metallothioneins I and IIA, metalloproteinase 1 tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin basic protein, thyroglobulin, Thy-1, immunoglobulins, H-chain variable region (VNP), serum amyloid P component, myoglobin, troponin C, smooth muscle α actin, preproencephalin A, vasopressin and the like can be used. Among them, the cytomegalovirus promoter, the human protein elongation factor 1α (EF-1α) promoter, the human and chicken β actin promoters and the like, which enable high expression in the whole body, are preferred.
The above-described vector preferably has a sequence that terminates the transcription of desired messenger RNA in the DNA-transfected animal (generally referred to as a terminator); for example, sequences of DNAs derived from viruses or various mammals can be used, with preference given to the simian virus SV40 terminator and the like.
In addition, for the purpose of increasing the expression of the desired exogenous DNA, the splicing signal and enhancer region of each DNA, a portion of the intron of an eukaryotic DNA and the like may also be joined upstream of the 5' end of the promoter region, between the promoter region and the translational region, or downstream of the 3' end of the translational region, depending on the purpose of use.
The translational region of normal RAIG 2 (or RAIG 3) can be acquired as the whole genomic DNA or a portion thereof from DNAs derived from the liver, kidney, adipose tissue, or muscle of various mammals (for example, humans, rabbits, dogs, cats, guinea pigs, hamsters, rats, mice and the like) and from various commercially available genomic DNA libraries, or using as the source material a cDNA prepared by a known method from RNA derived from the aforementioned organs or tissues. Also, an exogenous abnormal DNA can be obtained by mutating the translational region of normal RAIG 2 (or RAIG 3) obtained from the above-described cells or tissues by point mutagenesis.
The translational region can be prepared as a DNA construct expressible in the transgenic animal by an ordinary DNA engineering technique wherein the DNA is joined downstream of the aforementioned promoter (and, if desired, upstream of the transcription termination site).
The exogenous DNA of the present invention at the fertilized egg cell stage is transferred in a manner such that the DNA is certainly present in all the germinal cells and somatic cells of the subject mammal. The presence of the exogenous DNA of the present invention in the germinal cells of the animal prepared by DNA transfer means that all offspring of the animal prepared will carry the exogenous DNA of the present invention in all of the germinal cells and somatic cells thereof. The offspring of the animal of this species that have inherited the exogenous DNA of the present invention have the exogenous DNA of the present invention in all of the germinal cells and somatic cells thereof.
A non-human mammal transfected with the normal exogenous DNA of the present invention can be grown over generations as animals bearing that DNA in an ordinary breeding environment after being confirmed to stably retain the exogenous DNA by mating.
The exogenous DNA of the present invention at the fertilized egg cell stage is transferred in a manner such that the DNA is present in excess in all of the germinal cells and somatic cells of the subject mammal. The presence of the exogenous DNA of the present invention in excess in the germinal cells of the animal prepared by DNA transfer means that all offspring of the animal prepared will carry the exogenous DNA of the present invention in excess in all of the germinal cells and somatic cells thereof. The offspring of the animal of this species that have inherited the exogenous DNA of the present invention have the exogenous DNA of the present invention in excess in all of the germinal cells and somatic cells thereof.
By obtaining a homozygous animal having the transferred DNA in both the homologous chromosomes, and mating a male and female of this animal, the animal can be grown over generations so that all offspring thereof will retain the DNA in excess.

A non-human mammal having the normal DNA of the present invention highly expresses the normal DNA of the invention; the normal DNA may eventually cause RAIG 2 (or RAIG 3) hyperfunction by enhancing the function of the endogenous normal DNA, and the animal can be used as a pathologic model animal for the disease. For example, using an animal transfected with the normal DNA of the present invention, it is possible to elucidate the pathological mechanisms of RAIG 2 (or RAIG 3) hyperfunction and diseases associated with RAIG 2 (or RAIG 3), and to investigate therapeutic methods for these diseases.
Also, because a mammal transfected with the exogenous normal DNA of the present invention has a symptom of increased free RAIG 2 (or RAIG 3), it can be used for screening tests for therapeutic drugs for diseases associated with RAIG 2 (or RAIG 3).
On the other hand, a non-human mammal bearing the exogenous abnormal DNA of the present invention can be grown over generations as an animal bearing the DNA in an ordinary breeding environment after being confirmed to stably retain the exogenous DNA by mating. Furthermore, the desired exogenous DNA can be used as the source material as incorporated in the aforementioned plasmid. A DNA construct with a promoter can be prepared by an ordinary DNA engineering technique. The abnormal DNA of the present invention at the fertilized egg cell stage is transferred in a manner such that the DNA is present in all of the germinal cells and somatic cells of the subject mammal. The presence of the abnormal DNA of the present invention in the germinal cells of the animal prepared by DNA transfer means that all offspring of the animal prepared will carry the abnormal DNA of the present invention in all of the germinal cells and somatic cells thereof. The offspring of the animal of this species that have inherited the exogenous DNA of the present invention have the abnormal DNA of the present invention in all of the germinal cells and somatic cells thereof. By obtaining a homozygous animal having the introduced DNA in both the homologous chromosomes, and mating a male and female of this animal, the animal can be grown over generations so that all the offspring thereof will carry the DNA.

A non-human mammal bearing the abnormal DNA of the present invention highly expresses the abnormal DNA of the present invention; the DNA may finally cause functionally inactive adiaphoria to RAIG 2 (or RAIG 3) by inhibiting the function of endogenous normal DNA, and the animal can be utilized as a pathological model animal for the disease. For example, using an animal transfected with the abnormal DNA of the present invention, it is possible to elucidate the pathological mechanism of functionally inactive adiaphoria to RAIG 2 (or RAIG 3), and to investigate a therapeutic method for this disease.
Regarding specific applicability, an animal highly expressing the abnormal DNA of the present invention serves as a model for elucidating the functional impairment (dominant negative action) of normal RAIG 2 (or RAIG 3) due to abnormal RAIG 2 (or RAIG 3) in functionally inactive adiaphoria to RAIG 2 (or RAIG 3).
Because a mammal transfected with the exogenous abnormal DNA of the present invention has a symptom of increased free abnormal RAIG 2 (or RAIG 3), it can also be utilized for screening tests for a therapeutic drug for functionally inactive adiaphoria to RAIG 2 (or RAIG 3).
As examples of other potential applications of the above-described two kinds of DNA-transfected animal of the present invention, the following can be mentioned:

(1) use as cell sources for tissue culture,
(2) analysis of association with proteins specifically expressed or activated by RAIG 2 (or RAIG 3) and the like, by a direct analysis of DNA or RNA in a tissue of the DNA-transfected animal of the present invention, or by an analysis of the RAIG 2 (or RAIG 3) produced,
(3) research into the functions of cells generally difficult-to-culture tissues using cells of DNA-bearing tissues cultured by standard tissue culture technology,

(4) screening of a drug that enhances a function of cells using the cells described in (3) above,

(5) isolation and purification of variant RAIG 2 (or RAIG 3) and preparation of its antibody, and the like.

Furthermore, using the DNA-transfected animal of the present invention, it is possible to examine the clinical symptoms in diseases associated with RAIG 2 (or RAIG 3), including functionally inactive adiaphoria to RAIG 2 (or RAIG 3) and the like, and it is also possible to obtain more extensive pathological findings in various organs of a model of a disease associated with RAIG 2 (or RAIG 3), thus contributing to the development of new therapeutic methods, and to research and treatment for secondary diseases due to the disease.

It is also possible to excise each organ from the DNA-transfected animal of the present invention, shred the organ, then acquire liberated DNA-transfected cells using a protein-degrading enzyme such as trypsin, and culture the cells or establish a line of the cultured cells. Furthermore, it is possible to examine the associations with identification, apoptosis, differentiation or growth of RAIG 2 (or RAIG 3)-producing cells, or the signal transmission mechanisms involved therein, to examine abnormalities thereof, and the like, thus providing effective research materials for the elucidation of the action of RAIG 2 (or RAIG 3).

Furthermore, for the development of therapeutic drugs for diseases associated with RAIG 2 (or RAIG 3), including functionally inactive adiaphoria to RAIG 2 (or RAIG 3), using the DNA-transfected animal of the present invention, it is possible to provide an effective and rapid screening method for the therapeutic drugs for the diseases, using the above-described test method, quantification method and the like. Also, using the DNA-transfected animal of the present invention or the exogenous DNA expression vector of the present invention, it is possible to investigate and develop a DNA therapy for a disease associated with RAIG 2 (or RAIG 3).

[11] Preparation of non-human knockout animal having the RAIG 2 (or RAIG 3)-encoding gene inactivated therein

The present invention provides non-human mammalian embryonic stem cells having the DNA of the present invention inactivated therein and a non-human mammal deficient in the expression of the DNA of the present invention. Accordingly, the present invention provides:

[1] non-human mammalian embryonic stem cells having the DNA of the present invention inactivated therein,

[2] the embryonic stem cells described in term [1], wherein the DNA has been inactivated by introducing a reporter gene (e.g., the β-galactosidase gene derived from *Escherichia coli*),

[3] the embryonic stem cells described in term [1], which is resistant to neomycin,

[4] the embryonic stem cells described in term [1], wherein the non-human mammal is a rodent,

[5] the embryonic stem cells described in term [4], wherein the rodent is a mouse,

[6] a non-human mammal deficient in the expression of the DNA of the present invention, wherein the DNA has been inactivated,

[7] the non-human mammal described in term [6], wherein the DNA has been inactivated by introducing a reporter gene (e.g., the β-galactosidase gene derived from *Escherichia coli*), the reporter gene being expressible under the control of a promoter for the DNA of the present invention,

[8] the non-human mammal described in term [6], wherein the non-human mammal is a rodent,

[9] the non-human mammal described in term [8], wherein the rodent is a mouse, and

[10] a method of screening a compound that promotes or inhibits the promoter activity for the DNA of the present invention or a salt thereof, which comprises administering a test compound to the animal described in term [7], and detecting the expression of the reporter gene.

Non-human mammalian embryonic stem cells having the DNA of the present invention inactivated therein refer to embryonic stem cells (hereinafter abbreviated as ES cells) of a non-human mammal, wherein the DNA has substantially no capability of RAIG 2 (or RAIG 3) expression (hereinafter also referred to as the knockout DNA of the present invention) as a result of artificially mutating the DNA of the present invention possessed by the non-human mammal to suppress the capability of DNA expression, or to substantially deprive of the activity of the RAIG 2 (or RAIG 3) encoded by the DNA. The non-human mammal is exemplified by the same examples as those mentioned above.

The method of artificially mutating the DNA of the present invention can be performed by, for example, deleting a portion or whole of the DNA sequence, or inserting, or replacing with, another DNA, by a gene engineering technique. For example, by shifting the reading frame of codon or destroying the function of promoter or exon with these mutations, the knockout DNA of the present invention may be prepared.

Specifically, non-human mammalian embryonic stem cells having the DNA of the present invention inactivated therein (hereinafter abbreviated as the DNA-inactivated ES cells of the present invention or the knockout ES cells of the present invention) can be obtained by isolating the DNA of the present invention existing in the desired non-human mammal; constructing a DNA strand having a DNA sequence to destroy the gene by inserting a drug resistance gene represented by the neomycin resistance gene and the hygromycin resistance gene, or a reporter gene represented by lacZ β-galactosidase gene) and cat (chloramphenicol acetyltransferase gene) or the like into the exon moiety of the DNA of the present invention to destroy the exon function, or inserting a DNA sequence that terminates gene transcription (for example, poly A addition signal and the like) into the intron moiety between exons to prevent the synthesis of complete mRNA; transferring the DNA strand into a chromosome of the animal by, for example, homologous recombination; analyzing the thus-obtained ES cells by a Southern hybridization analysis using a DNA sequence on the DNA of the present invention or in the vicinity thereof as a probe, or by a PCR method using a DNA sequence on the targeting vector and a DNA sequence in the vicinity other than the DNA of the present invention used to prepare the targeting vector, as primers; and selecting the knockout ES cells of the present invention.

The starting ES cell to have the DNA of the present invention inactivated therein by homologous recombination and the like may be of an already established cell line as described above, or of a cell line newly established by the known method of Evans and Kaufman. For example, in the case of mouse ES cells, an ES cell of the 129 line is currently generally used as the starting cell; however, since the immunological background of the 129 line is unclear, an ES cell line established using, for example, C57BL/6 mice, or $BDF_1$ mice ($F_1$ between C57BL/6 and DBA/2), which have been improved by crossing with DBA/2 to increase the otherwise small number of collectable eggs of C57BL/6, and the like can, also be used advantageously, for the purpose of obtaining a pure line of ES cells of clear immunological genetic background, and the like. Because $BDF_1$ mice are backgrounded by C57BL/6 mice, in addition to being advantageous in that the number of collectable eggs is large and the eggs are tough, the ES cells obtained using a $BDF_1$ mouse, when used to create a pathological model mouse, can be used advantageously in that the genetic background thereof can be replaced with C57BL/6 mouse by back-crossing with C57BL/6 mouse.

For establishing a line of ES cells, blastocysts at day 3.5 after fertilization are normally used; in addition, by collecting 8-cell embryos and culturing them to the blastocyst stage, a large number of early embryos can be obtained efficiently. Although the ES cells used may be of either sex, male ES cells are normally more convenient in creating a germ line chimera. Also, to reduce operating time for painstaking cultivation, it is desirable to make sex identification as early as possible.

As an example of the method of sex identification of ES cells, a method that comprises amplifying and detecting a gene in the sex-determining region on Y chromosome by a PCR method can be mentioned. Using this method, a number of ES cells in one colony (about 50 cells) is enough for karyotype analysis, compared to the conventional method, which requires about $10^6$ cells for the same purpose; therefore, it is possible to perform primary selection of ES cells in the early stage of cultivation by sex identification, thus enabling a significant reduction in operating time in the early stage of cultivation because early selection of male cells has become possible.

Secondary selection can be performed by, for example, confirming the chromosome number by the G-binding method, and the like. Although the chromosome number of the ES cells obtained is desirably 100% of the normal number, it is desirable that a gene of the ES cells is knocked out and then re-cloned into normal cells (for example, cells having a chromosome number of 2n = 40 for mice) if a 100% number is difficult to achieve due to physical procedures during cell line establishment, and the like.

Although the embryonic stem cell line thus obtained generally shows very good growing capacity, it is likely to lose its capability of ontogeny, so that it must be subcultured carefully. For example, cells of this line are cultured using a method of cultivation on appropriate feeder cells such as STO fibroblasts in the presence of LIF (1 to 10000 U/ml) in a carbon dioxide incubator (preferably under 5% carbon dioxide and 95% air or under 5% oxygen, 5% carbon dioxide and 90% air) at about 37°C, or the like; they are subcultured using, for example, a method comprising a treatment with a trypsin/EDTA solution (normally 0.001% to 0.5% trypsin/0.1 to 5 mM EDTA, preferably about 0.1% trypsin/1 mM EDTA) to give single cells, and sowing them on newly prepared feeder cells, or the like. This subculture is normally performed every 1 to 3 days, during which it is desirable that the cells be examined, and any morphologically abnormal cells, if found, are desirably discarded.

ES cells can be differentiated into various types of cells, such as the parietal muscle, visceral muscles, and myocardium when subjected to monolayer culture to the extent of a high density, or to suspension culture to the extent of the formation of a cell mass, under appropriate conditions [M.J. Evans and M.H. Kaufman, Nature, Vol. 292, p. 154, 1981; G. R. Martin, Proceedings of the National Academy of Sciences of the USA (Proc. Natl. Acad. Sci. U.S.A.), Vol. 78, p. 7634, 1981; T.C. Doetschman et al., Journal of Embryology and Experimental Morphology (J. Embryol. Exp. Morphol.), Vol. 87, p. 27, 1985], and cells deficient in the expression of the DNA of the present invention obtained by differentiating the ES cells of the present invention are useful in cytobiological investigations of RAIG 2 (or RAIG 3) or RAIG 2 (or RAIG 3).

A non-human mammal deficient in the expression of the DNA of the present invention can be distinguished from a normal animal by measuring the amounts of mRNA in the animals, and indirectly comparing the expression amounts thereof.

The non-human mammal is exemplified by the same examples as those mentioned above.

A non-human mammal deficient in the expression of the DNA of the present invention can be produced by, for example, introducing a targeting vector prepared as described above into a mouse embryonic stem cell or mouse ovum to cause homologous recombination to replace the DNA sequence having the DNA of the present invention inactivated therein in the introduced targeting vector with the DNA of the present invention on a chromosome of the mouse embryonic stem cell or mouse ovum, whereby the DNA of the present invention can be knocked out. Since many recombinations in mammals are non-homologous, as examples of means of screening cells having undergone homologous recombination, a method that comprises inserting a drug resistance gene such as the neomycin resistance gene into the DNA of the present invention, constructing a targeting vector comprising the thymidine kinase (tk) gene in the vicinity of the DNA of the present invention, introducing the vector into an embryonic stem cell or ovum, and selecting cells that survive in the presence of a drug corresponding to the inserted drug resistance gene (for example, G418 and the like for the neomycin resistance gene) and ganciclovir, can be mentioned. That is, if the insertion variant DNA of the present invention is incorporated onto a chromosome by homologous recombination, the cells are resistant to ganciclovir because the tk gene is eliminated, whereas in the case of incorporation by non-homologous recombination, the cells are susceptible to ganciclovir because the tk gene is incorporated at the same time. If the diphtheria toxin gene or the like is used instead of the tk gene, cells undergoing random insertion will die due to the production of the toxin, so that selection with a single drug is possible.

The final confirmation of cells having the DNA of the present invention knocked out therein can be achieved by a Southern hybridization analysis using a DNA sequence on the DNA of the present invention or in the vicinity thereof as a probe, or by a PCR method using a DNA sequence on the targeting vector and a DNA sequence in the vicinity other than the mouse-derived DNA of the present invention used in the targeting vector, as primers.

When a non-human mammalian embryonic stem cell is used, a cell line having the DNA of the present invention inactivated therein by homologous gene recombination is cloned, its cell is injected into a non-human mammalian embryo or blastocyst at an appropriate stage, for example, the 8-cell stage, and the chimeric embryo prepared is transplanted into the uterus of a pseudopregnant female of the non-human mammal. The animal created is a chimeric animal consisting of both cells having the normal locus of the DNA of present invention and cells having an artificially mutated locus of the DNA of the present invention.

When some of the germ cells of the chimeric animal have a mutated locus of the DNA of the present invention, such a chimeric individual and a normal individual may be mated; from the animal population thus obtained, an individual all of whose tissues consist of cells having an artificially mutated locus of the DNA of the present invention can be selected by, for example, coat color judgment and the like. Because the individual thus obtained is normally a hetero-expression-deficient individual, such hetero-expression-deficient individuals may be mated to obtain an individual homo-deficient in the expression of the protein of the present invention out of the pups thereof.

When an ovum is used, for example, a transgenic non-human mammal incorporating a targeting vector in a chromosome can be obtained by injecting a DNA solution into the nucleus of the ovum by the microinjection method, and such an individual is obtained by selecting one having a mutation in the locus of the DNA of the present invention by homologous gene recombination from among these transgenic non-human mammals.

An individual having the DNA of the present invention thus knocked out therein can also be grown over generations in an ordinary breeding environment after animal individuals obtained by mating as well are confirmed to have the DNA knocked out therein.

Furthermore, establishment and maintenance of a germ line can be performed according to conventional methods. That is, homozygous animals having the inactivated DNA in both the homologous chromosomes can be obtained by mating a male and female animal bearing the inactivated DNA. These homozygous animals can be efficiently propagated by breeding in a ratio of one normal individual and a plurality of homozygous animals relative to the dam. By mating a male and female heterozygous animal, homozygous and heterozygous animals having the inactivated DNA are propagated over generations.

Non-human mammalian embryonic stem cells having the DNA of the present invention inactivated therein are highly useful in creating a non-human mammal deficient in the expression of the DNA of the present invention.

Also, because a non-human mammal deficient in the expression of the DNA of the present invention lacks various biological activities induced by RAIG 2 (or RAIG 3), it can serve as models of diseases caused by inactivation of biological activities of RAIG 2 (or RAIG 3), and is hence useful in elucidating the causes of these diseases and investigating therapies therefor.

[12a] Method of screening a compound having a therapeutic/prophylactic effect for a disease caused by deficiency, damage and the like of the DNA of the present invention.

A non-human mammal deficient in the expression of the DNA of the present invention can be used to screen a compound having a therapeutic/prophylactic effect for a disease caused by deficiency, damage and the like of the DNA of the present invention.

Accordingly, the present invention provides a method of screening a compound having a therapeutic/prophylactic effect for a disease caused by deficiency, damage and the like of the DNA of the present invention or a salt thereof, which comprises administering a test compound to a non-human mammal deficient in the expression of the DNA of the present invention, and observing and measuring changes in the animal.
As the non-human mammal deficient in the expression of the DNA of the present invention used for the screening method, the same examples as those mentioned above can be mentioned.
As examples of the test compound used, peptides, proteins, non-peptide compounds, synthetic compounds, fermentation products, cell extracts, plant extracts, animal tissue extracts, plasma and the like can be mentioned; these compounds may be novel compounds or known compounds.
Specifically, the therapeutic/prophylactic effect of a test compound can be tested by treating a non-human mammal deficient in the expression of the DNA of the present invention with the test compound, comparing the animal with a non-treated control animal, with the changes in the organs, tissues, disease symptoms and the like in the animals as indexes.
Examples of the method of treating a test animal with a test compound include oral administration, intravenous injection and the like, which can be selected as appropriate for the symptoms of the test animal, the properties of the test compound, and the like. A dose of the test compound can be selected as appropriate according to the method of administration, properties of the test compound and the like.

When a test compound is administered to a test animal in the screening method, the test compound can be selected as a compound having a therapeutic/prophylactic effect for a disease such as impaired glucose tolerance, if the blood glucose level in the test animal decreases by about 10% or more, preferably about 30% or more, and more preferably about 50% or more.
The compound obtained using the screening method is a compound selected from among the above-described test compounds, and can be used, for a disease caused by RAIG2 (or RAIG3) deficiency or damage and the like, as for example, 1) a regulator of sugar/lipid metabolism, 2) a regulator of pancreas and/or pituitary function, 3) a regulator of insulin secretion and/or blood glucose and/or ACTH secretion, 4) a prophylactic/therapeutic agent for a disease involved by a sugar/lipid metabolism abnormality, a pancreas and/or pituitary function abnormality, or an insulin secretion and/or blood glucose level and/or ACTH secretion abnormality and the like. Furthermore, compounds derived from the compound obtained in the above-described screening can be used in the same way.

A compound obtained by the screening method may have formed a salt; as examples of the salt of the compound, physiologically acceptable salts with acids (e.g., inorganic acids, organic acids and the like), bases (e.g., alkali metals and the like) and the like can be mentioned, with preference given to physiologically acceptable acid adduct salts. As examples of such salts, salts with inorganic acids (for example, hydrochloric acid, phosphoric acid, hydrobromic acid, sulfuric acid and the like), salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid and the like) and the like can be mentioned.

When a compound obtained by the screening method or a salt thereof is used as a pharmaceutical, it can be formulated in the same manner as with the aforementioned RAIG 2 (or RAIG 3).
Because the preparation thus obtained is safe and less toxic, it can be administered to, for example, mammals (for example, humans, rats, mice, guinea pigs, rabbits, sheep, swine, cattle, horses, cats, dogs, monkeys and the like).
The dose of the compound or a salt thereof varies depending on the subject of administration, target organ, symptoms, method of administration and the like; in oral administration, the dose is normally about 0.1 to 100 mg, preferably about 1.0 to 50 mg, more preferably about 1.0 to 20 mg, per day, in, for example, a diabetic patient (assuming a 60 kg body weight). In parenteral administration, the single dose varies depending on the subject of administration, target organ, symptoms, method of administration and the like; for example, in the form of an injection, it is advantageous to administer normally about 0.01 to 30 mg, preferably about 0.1 to 20 mg, more preferably about 0.1 to 10 mg, per day, in, for example, a diabetic patient (assuming a 60 kg body weight). In cases where the subject of administration is other than a human, a dose corresponding to a human dose per 60 kg body weight can be administered.

[12b] Method of screening a compound that promotes or inhibits the activity of a promoter for the DNA of the present invention

The present invention provides a method of screening a compound that promotes or inhibits the activity of a promoter for the DNA of the present invention or a salt thereof, which comprises administering a test compound to a non-human mammal deficient in the expression of the DNA of the present invention, and detecting the expression of a reporter gene.
In the above-described screening method, as the non-human mammal deficient in the expression of the DNA of the present invention, one having the DNA of the present invention inactivated therein by introducing a reporter gene, wherein the reporter gene is expressible under the control of a promoter for the DNA of the present invention, out of the aforementioned non-human mammals deficient in the expression of the DNA of the present invention, is used.
The test compound is exemplified by the same examples as those mentioned above.
As the reporter gene used, the same examples as those mentioned above can be mentioned, with preference given to the β-galactosidase gene (lacZ), the soluble alkaline phosphatase gene or the luciferase gene and the like.
In a non-human mammal deficient in the expression of the DNA of the present invention having the DNA of the present invention replaced with a reporter gene therein, the reporter gene occurs under the control of a promoter for the DNA of the present invention; therefore, promoter activity can be detected by tracing the expression of a substance encoded by the reporter gene.
For example, when a portion of the RAIG 2 (or RAIG 3)-encoding DNA region is replaced with the β-galactosidase gene (lacZ) derived from *Escherichia coli,* β-galactosidase is expressed, in place of RAIG 2 (or RAIG 3), in tissues where RAIG 2 (or RAIG 3) is otherwise expressed. Therefore, for example, by staining using a reagent that serves as a substrate for β-galactosidase, such as 5-bromo-4-chloro-3-indolyl-β-galactopyranoside (X-gal), the expression state of RATG 2 (or RAIG 3) in the animal body can be examined conveniently. Specifically, RAIG 2 (or RAIG 3)-deficient mouse or a tissue section thereof is fixed with glutaraldehyde and the like, washed with phosphate-buffered saline (PBS), then reacted with a staining solution containing X-gal at room temperature or around 37°C for about 30 minutes to 1 hour, thereafter the tissue specimen is washed with 1 mM EDTA/PBS solution to stop the β-galactosidase reaction, and the color developed is examined. The lacZ-encoding mRNA may be detected according to a conventional method.
A compound obtained using the above-described screening method or a salt thereof is a compound selected from among the above-described test compounds, and promoting or inhibiting the activity of a promoter for the DNA of the present invention.
A compound obtained by the screening method may have formed a salt; as examples of the salt of the compound, physiologically acceptable salts with acids (e.g., inorganic acids and the like), bases (e.g., organic acids and the like) and the like can be mentioned, with preference given to physiologically acceptable acid adduct salts. As examples of such salts, salts with inorganic acids (for example, hydrochloric acid, phosphoric acid, hydrobromic acid, sulfuric acid and the like), salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid and the like) and the like can be mentioned.

Because a compound that promotes the activity of a promoter for the DNA of the present invention or a salt thereof is capable of promoting the expression of SS169 and promoting the function of RAIG 2 (or RAIG 3), it is useful as, for example, a pharmaceutical such as a prophylactic/therapeutic drug for a disease associated with RAIG 2 (or RAIG 3) dysfunction and the like.
Because a compound that inhibits the activity of a promoter for the DNA of the present invention or a salt thereof is capable of inhibiting the expression of SS169 and inhibiting the function of RAIG 2 (or RAIG 3), it is useful as, for example, a pharmaceutical such as a prophylactic/therapeutic drug for a disease associated with RAIG 2 (or RAIG 3) overexpression and the like.
Examples of diseases associated with RAIG2 (or RAIG3) dysfunction include diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder and the like. Meanwhile, examples of diseases associated with RAIG2 (or RAIG3) overexpression include obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers and the like.
Furthermore, compounds derived from a compound obtained by the above-described screening can also be used in the same manner.

When a compound obtained by the screening method or a salt thereof is used as a pharmaceutical, it can be formulated in the same manner as with the aforementioned "prophylactic/therapeutic agent for a disease associated with RAIG 2 (or RAIG 3) dysfunction".
Because the preparation thus obtained is safe and less toxic, it can be administered to, for example, mammals (for example, humans, rats, mice, guinea pigs, rabbits, sheep, swine; cattle, horses, cats, dogs, monkeys and the like).
The dose of the compound or a salt thereof varies depending on the subject of administration, target organ, symptoms, method of administration and the like; in oral administration, the dose is normally about 0.1 to 100 mg, preferably about 1.0 to 50 mg, more preferably about 1.0 to 20 mg, per day, in, for example, a diabetic patient (assuming a 60 kg body weight). In parenteral administration, the single dose varies depending on the subject of administration, target organ, symptoms, method of administration and the like; for example, in the form of an injection, it is advantageous to administer normally about 0.01 to 30 mg, preferably about 0.1 to 20 mg, more preferably about 0.1 to 10 mg, per day, in, for example, a diabetic patient (assuming a 60 kg body weight). In cases where the subject of administration is other than a human, a dose corresponding to a human dose per 60 kg body weight can be administered.

As stated above, a non-human mammal deficient in the expression of the DNA of the present invention is highly useful in screening a compound that promotes or inhibits the activity of a promoter for the DNA of the present invention or a salt thereof, and can contribute significantly to the elucidation of the causes of various diseases due to deficiency of the expression of the DNA of the present invention or the development of a prophylactic/therapeutic drug.
Also, provided that a gene encoding one of various proteins is joined downstream of a DNA comprising a RAIG 2 (or RAIG 3) promoter region, and this DNA construct is injected into an animal ovum to prepare what is called a transgenic animal, it is also possible to allow the animal to specifically synthesize the protein, and investigate its action in vivo. Furthermore, provided that an appropriate reporter gene is bound to the above-described promoter portion, and a cell line that expresses the gene is established, the cell line can be used as a screening system for a low-molecular compound that acts to specifically promote or suppress the in vivo productivity of RAIG 2 (or RAIG 3) itself.

Being a drosophila homologue of RAIG2, BOSS is known to have the N-terminal extracellular domain thereof involved in the synthesis of the compound eye, but the physiological function of the 7-pass transmembrane region thereof, which has a homology to RAIG2, had not been known at all. As shown in an Example below, as a result of a comparison of the blood glucose levels between a boss gene-deficient mutant and transgenic flies having the gene expressed forcibly therein, an increase of blood glucose level was observed in the former compared to the wild type, and this increase was suppressed in the latter. This strongly suggests that BOSS, like RAIG2 and RAIG3, may also sense blood glucose concentrations and regulate insulin signals. Therefore, systems in the drosophila such as the boss gene-deficient mutant, flies having the boss gene knocked out, and transgenic flies having the boss gene expressed forcibly therein can serve as useful tools in basic research into sugar/lipid metabolism and diseases such as diabetes mellitus in mammals such as humans.

In the description and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA : | deoxyribonucleic acid |
| cDNA : | complementary deoxyribonucleic acid |
| A : | adenine |
| T : | thymine |
| G : | guanine |
| C : | cytosine |
| RNA : | ribonucleic acid |
| RNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| EDTA : | ethylenediamine tetraacetic acid |
| SDS : | sodium dodecyl sulfate |

| | |
|---|---|
| Gly : | glycine |
| Ala : | alanine |
| Val | : saline |
| Leu : | leucine |
| Ile : | isoleucine |
| Ser : | serine |
| Thr : | threonine |
| Cys : | cysteine |
| Met | : methionine |
| Glu : | glutamic acid |
| Asp : | aspartic acid |
| Lys : | lysine |
| Arg : | arginine |
| His : | histidine |
| Phe : | phenylalanine |
| Tyr : | tyrosine |
| Trp : | tryptophan |
| Pro | : proline |
| Asn : | asparagine |

Gln : glutamine
pGlu : pyroglutamic acid
Me : methyl group
Et : ethyl group
Bu : butyl group
Ph : phenol group
TC : thiazolidin-4(R)-carboxamide group

Furthermore, substituents, protecting groups and reagents often used in the present description are denoted using the following symbols.

Tos : p-toluenesulfonyl
CHO : formyl
Bzl : benzyl
$Cl_2Bzl$ : 2,6-dichlorobenzyl
Bom : benzyloxymethyl
Z : benzyloxycarbonyl
Cl-Z : 2-chlorobenzyloxycarbonyl
Br-Z : 2-bromobenzyloxycarbonyl
Boc : t-butoxycarbonyl
DNP : dinitrophenol
Trt : trityl
Bum : t-butoxymethyl
Fmoc : N-9-fluorenyl methoxycarbonyl
HOBt : 1-hydroxybenztriazole
HOOBt : 3,4-dihydro-3-hydroxy-4-oxo-1,2,3-benztriazine
HONB : 1- hydroxy-5-norbornene-2,3-dicarboximide
DCC : N,N'-dicyclohexylcarbodiimide

The sequence identification numbers in the sequence listing herein show the following sequences.
[SEQ ID NO:1] shows the base sequence of human RAIG2 cDNA coding sequence.
[SEQ ID NO:2] shows the amino acid sequence of human RAIG2.
[SEQ ID NO:3] shows the base sequence of human RAIG3 cDNA coding sequence.
[SEQ ID NO:4] shows the amino acid sequence of human RAIG3.

The present invention is hereinafter described in further detail by means of the following examples, which, however, are merely for exemplification, and never limit the scope of the present invention.

**Examples**

Example 1

Mouse insulinoma MIN6 cells (supplied by Dr. Junichi Miyazaki at the Division of Stem Cell Regulation Research, Department of Molecular Therapeutics, G6 Division of Pathophysiology and Therapeutics, Osaka University Graduate School of Medicine) were cultured in a 24-well plate containing a DMEM comprising 15% fetal calf serum (inactivated by treatment at 56°C for 30 minutes), 5 μl/l β-mercaptoethanol and 4.5 g/l glucose, in an atmosphere of 5% $CO_2$/95% air, at 37°C and low density. cDNAs of RAIG2 and RAIG3 were obtained from a human brain or liver cDNA library by a conventional method of PCR. This was inserted into pIRES2-EGFP (CLONTECH). After 24 hours, the above-described cells were transfected with an expression vector containing the RAIG2 or RAIG3 cDNA. The transfection was performed using Lipofectamin 2000 (Invitrogen), as directed in the manufacturer's manual. After 48 hours of further cultivation, the cells were retained in a KRBB solution [129 mM NaCl, 4.8 mM KCl, 1.2 mM $MgSO_4$, 1.2 mM $KH_2PO_4$, 4.8 mM $NaHCO_3$, 10 mM HEPES, 2.5 mM $CaCl_2$, 0.2% bovine serum albumin (pH adjusted to 7.4)] containing 2.8 mM glucose at 37°C for 15 minutes, and then washed. After this was performed twice, 0.5 ml of a KRBB solution containing each concentration of glucose or 1,5-AG (purchased from Wako Pure Chemical Industries) was added, and the cells were retained at 37°C for 1 hour. Each 10 μl portion of the culture supernatant was taken, and the insulin content was measured by ELISA. For the measurement, Rat/mouse Insulin ELISA Kit (produced by LINCO Research, purchased from SCETI Co., Ltd.) was used. As a result, insulin release increased with 20 mM glucose compared to 2.8 mM glucose, and increased further in the cells having RAIG3 expressed forcibly therein (FIG. 1). With 10 mM glucose, the amount of insulin released was indifferent from that with 2.8 mM glucose in control cells, but in the cells having RAIG3 expressed forcibly therein, the amount released increased significantly (FIG. 1). In the cells having RAIG2 or RAIG3 expressed forcibly therein, by adding 0.6 mM 1,5-AG to 2.8 mM glucose, insulin release equivalent to that with 20 mM glucose was caused (FIGs. 2a and b).

Example 2

Mouse pituitary tumor AtT-20/D16v-F2 cells (purchased from ATCC) secrete ACTH in response to CRF. This occurs in low glucose (2.8 mM) solution, and is suppressed by high glucose (20 mM). These cells were cultured in a 24-well plate containing an MEM containing 10% fetal calf serum (inactivated by treatment at 56°C for 30 minutes) and 1 g/l glucose, in an atmosphere of 10% $CO_2$/95% air, at 37°C. After 1 hour, the cells were transfected with siRNA of RAIG2 or RAIG3. Three kinds of siRNAs for mouse RAIG2 and RAIG3, designed by Ambion, were purchased and used. The transfection was performed using HiPerFect Transfection Reagent (produced by QIAGEN) as directed in the manufacturer's manual. After each treatment, the cells were cultured for 48 hours, and then retained in a KRBB solution containing 2.8 mM glucose at 37°C for 15 minutes, and washed. After this was performed twice, a KRBB solution containing each concentration of glucose was added, 0.5 ml of 10 nM CRF (purchased from Peptide Institute, Inc.; a 100 μM solution was prepared using 0.1 M acetic acid, and diluted with water 10 fold, and this dilution was added to the KRBB solution at 1/1000) was added, and the cells were retained at 37°C for 1 hour. Each 50 μl portion of the culture supernatant was taken, and the amount of ACTH was measured by ELISA. For the measurement, the ACTH (Rat) EIA Kit (produced by Peninsula Laboratories, purchased from SCETI Co., Ltd.) was used. When the expression of RAIG2 was suppressed, ACTH release at low glucose was suppressed (FIG. 3). Meanwhile, when the expression of RAIG3 was suppressed, the suppression of ACTH release by high glucose was modified (FIG. 3). From these results, it was thought that RAIG2 and RAIG3 sensed glucose concentrations and controlled the amount of ACTH released by CRF.

Example 3

Third-stage larvae of the wild type drosophila, boss-deficient mutant drosophila, and a transgenic drosophila having boss expressed forcibly therein (4-5 larvae for each case) were washed with water and dried on filter paper, after which 0.5 μl of Hemolympho (celomic fluid = corresponding to human blood and lymph) was recovered. Thereto, 14.5 μl of PBS was added; this was centrifuged at 13,000xg, and the supernatant was recovered. 100 μl of Infinity Glucose Reagent (ThermoDMA) was added to 1 μl of each Hemolympho (in the case of trehalose measurement, 0.2 μl of Pig kidney trehalase (Sigma) was added), and the cells were incubated at 37°C for 12 hours, after which glucose and trehalose concentrations were measured. As a result, in the boss-deficient mutant, the blood glucose level was increased compared to the wild type (1.3 times higher), in the transgenic with forcible expression, this increase was suppressed (FIG. 4).

Example 4

Male flies at 3 days after eclosion were given starving stress (reared on 0.8% agar/PBS at 25°C), and their survival rate was determined. It was found that without an energy source, the boss-deficient mutant died earlier than the wild type.
Regarding the 48-hour survival rate, 62% of the wild-type flies survived, whereas the survival rate for the boss-deficient mutant decreased to 29%. Conversely, in the transgenic flies with forcible expression, the survival rate increased to 96%. In the boss-deficient mutant, fat reserve in the body decreased, and this is thought to be the cause. The blood glucose level increase observed in the boss-deficient mutant, and the survival rate reduction under the starving conditions agree with the phenomenon observed in the drosophila having insulin signaling suppressed therein. Hence, BOSS, like mouse RAIG2 and RAIG3, is also thought to control suqar metabolism by sensing glucose and 1,5-AG concentrations and regulate insulin signaling.

**Industrial Applicability**

According to the present invention, provided are a regulator of sugar/lipid metabolism or pancreas and/or pituitary function, a regulator of insulin secretion and/or blood glucose and/or ACTH secretion, a prophylactic/therapeutic agent and diagnostic reagent for a disease involved by a sugar/lipid metabolism abnormality or a pancreas and/or pituitary function abnormality, and a tool for screening for a pharmaceutical or animal drug candidate compound that is effective in the prophylaxis/treatment of the disease.
This application is based on a patent application No. 2006-101057 filed in Japan (filing date: March 31, 2006), the contents of which are incorporated in full herein by this reference.

## Claims

1. A pharmaceutical or animal drug comprising RAIG2 or a partial peptide thereof and/or RAIG3 or a partial peptide thereof.

2. The pharmaceutical or animal drug of claim 1, which is a regulator of sugar/lipid metabolism.

3. The pharmaceutical or animal drug of claim 1, which is a regulator of pancreas and/or pituitary function.

4. The pharmaceutical or animal drug of claim 1, which is an insulin secretion promoter and/or an agent for reducing blood glucose level and/or an adrenocorticotropic hormone secretion regulator.

5. The pharmaceutical or animal drug of claim 1, which is a prophylactic/therapeutic agent for a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia and memory and learning disorder.

6. A pharmaceutical or animal drug comprising a nucleic acid that encodes RAIG2 or a partial peptide thereof and/or a nucleic acid that encodes RAIG3 or a partial peptide thereof.

7. The pharmaceutical or animal drug of claim 6, which is a regulator of sugar/lipid metabolism.

8. The pharmaceutical or animal drug of claim 6, which is a regulator of pancreas and/or pituitary function.

9. The pharmaceutical or animal drug of claim 6, which is an insulin secretion promoter and/or an agent for reducing blood glucose level and/or an adrenocorticotropic hormone secretion regulator.

10. The pharmaceutical or animal drug of claim 6, which is a prophylactic/therapeutic agent for a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia and memory and learning disorder.

11. A diagnostic reagent comprising a nucleic acid containing a base sequence that encodes RAIG2 or a portion thereof and/or a nucleic acid containing a base sequence that encodes RAIG3 or a portion thereof.

12. The reagent of claim 11, which is for diagnosing a sugar/lipid metabolism abnormality.

13. The reagent of claim 11, which is for diagnosing a pancreas and/or pituitary function abnormality.

14. The reagent of claim 11, which is for diagnosing an insulin secretion and/or blood glucose abnormality, and/or an adrenocorticotropic hormone secretion abnormality.

15. The reagent of claim 11, which is for diagnosing a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder, obesity, hypoglycemia, hypertension, edema, insulin resistance syndrome, brittle diabetes, fatty atrophy, insulin allergy, insulinoma, fat toxicity and cancers.

16. A diagnostic reagent comprising an antibody against RAIG2 and/or an antibody against RAIG3.

17. The reagent of claim 16, which is for diagnosing a sugar/lipid metabolism abnormality.

18. The reagent of claim 16, which is for diagnosing a pancreas and/or pituitary function abnormality.

19. The reagent of claim 16, which is for diagnosing an insulin secretion and/or blood glucose abnormality, and/or an adrenocorticotropic hormone secretion abnormality.

20. The reagent of claim 16, which is for diagnosing a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder, obesity, hypoglycemia, hypertension, edema, insulin resistance syndrome, brittle diabetes, fatty atrophy, insulin allergy, insulinoma, fat toxicity and cancers.

21. A pharmaceutical or animal drug comprising a neutralizing antibody against RAIG2 and/or a neutralizing antibody against RAIG3.

22. The pharmaceutical or animal drug of claim 21, which is a regulator of sugar/lipid metabolism.

23. The pharmaceutical or animal drug of claim 21, which is a regulator of pancreas and/or pituitary function.

**24.** The pharmaceutical or animal drug of claim 21, which is an insulin secretion suppressor and/or an agent for increasing blood glucose level and/or an adrenocorticotropic hormone secretion regulator.

**25.** The pharmaceutical or animal drug of claim 21, which is a prophylactic/therapeutic agent for a disease selected from the group consisting of obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers.

**26.** A pharmaceutical or animal drug comprising a nucleic acid containing a complementary strand sequence of a base sequence that encodes RAIG2 or a portion thereof and/or a nucleic acid containing a complementary strand sequence of a base sequence that encodes RAIG3 or a portion thereof.

**27.** The pharmaceutical or animal drug of claim 26, which is a regulator of sugar/lipid metabolism.

**28.** The pharmaceutical or animal drug of claim 26, which is a regulator of pancreas and/or pituitary function.

**29.** The pharmaceutical or animal drug of claim 26, which is an insulin secretion suppressor and/or an agent for increasing blood glucose level and/or an adrenocorticotropic hormone secretion regulator.

**30.** The pharmaceutical or animal drug of claim 26, which is a prophylactic/therapeutic agent for a disease selected from the group consisting of obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers.

**31.** A screening method for a substance that regulates sugar/lipid metabolism, comprising using RAIG2 or a partial peptide thereof or RAIG3 or a partial peptide thereof.

**32.** A screening method for a substance that regulates pancreas and/or pituitary function, comprising using RAIG2 or a partial peptide thereof or RAIG3 or a partial peptide thereof.

**33.** A screening method for a substance that regulates insulin secretion and/or blood glucose and/or adrenocorticotropic hormone secretion, comprising using RAIG2 or a partial peptide thereof or RAIG3 or a partial peptide thereof.

**34.** A screening method for a prophylactic/therapeutic substance for a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia, memory and learning disorder, obesity, hypoglycemia, hypertension, edema, insulin resistance syndrome, brittle diabetes, fatty atrophy, insulin allergy, insulinoma, fat toxicity and cancers, comprising using RAIG2 or a partial peptide thereof or RAIG3 or a partial peptide thereof.

**35.** The method of any one of claims 31 to 34, wherein RAIG2 or a partial peptide thereof or RAIG3 or a partial peptide thereof is supplied in the form of a eukaryotic cell that produces the same.

**36.** The method of claim 35, comprising further using a nucleic acid containing a base sequence that encodes RAIG2 or a portion thereof or a nucleic acid containing a base sequence that encodes RAIG3 or a portion thereof, or an antibody against RAIG2 or an antibody against RAIG3.

**37.** The method of claim 35, wherein secretion of insulin or adrenocorticotropic hormone serves as an index.

**38.** The method of claim 35, wherein the intracellular $Ca^{2+}$ concentration or cAMP production serves as an index.

**39.** The method of claim 35, wherein the expression of a reporter or selection marker gene serves as an index.

**40.** A screening method for a substance that alters the bindability of RAIG2 or RAIG3 and glucose or 1,5-anhydroglucitol, comprising using RAIG2 or a partial peptide thereof or RAIG3 or a partial peptide thereof, and glucose or 1,5-anhydroglucitol.

**41.** The method of claim 40, wherein RAIG2 or a partial peptide thereof or RAIG3 or a partial peptide thereof is supplied in the form of a cell that produces the same.

**42.** The method of claim 41, wherein secretion of insulin or adrenocorticotropic hormone serves as an index.

**43.** The method of claim 41, wherein the intracellular $Ca^{2+}$ concentration or cAMP production serves as an index.

**44.** The method of claim 41, wherein the expression of a reporter or selection marker gene serves as an index.

**45.** A pharmaceutical or animal drug comprising a substance that enhances the expression or activity of RAIG2 or RAIG3.

**46.** The pharmaceutical or animal drug of claim 45, wherein the substance that enhances the expression or activity of RAIG2 or RAIG3 is an agonist of RAIG2 or RAIG3.

**47.** The pharmaceutical or animal drug of claim 45 or 46, which is a regulator of sugar/lipid metabolism.

**48.** The pharmaceutical or animal drug of claim 45 or 46, which is a regulator of pancreas and/or pituitary function.

**49.** The pharmaceutical or animal drug of claim 45 or 46, which is an insulin secretion promoter and/or an agent for reducing blood glucose level and/or an adrenocorticotropic hormone secretion regulator.

**50.** The pharmaceutical or animal drug of claim 45 or 46, which is a prophylactic/therapeutic agent for a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia and memory and learning disorder.

**51.** A pharmaceutical or animal drug comprising a substance that inhibits the expression or activity of RAIG2 or RAIG3.

**52.** The pharmaceutical or animal drug of claim 51, wherein the substance that inhibits the expression or activity of RAIG2 or RAIG3 is an antagonist of RAIG2 or RAIG3.

**53.** The pharmaceutical or animal drug of claim 51 or 52, which is a regulator of sugar/lipid metabolism.

**54.** The pharmaceutical or animal drug of claim 51 or 52, which is a regulator of pancreas and/or pituitary function.

**55.** The pharmaceutical or animal drug of claim 51 or 52, which is an insulin secretion suppressor and/or an agent for increasing blood glucose level and/or an adrenocorticotropic hormone secretion regulator.

**56.** The pharmaceutical or animal drug of claim 51 or 52, which is a prophylactic/therapeutic agent for a disease selected from the group consisting of obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers.

**57.** A method of regulating any one selected from the group consisting of sugar/lipid metabolism, pancreatic function, pituitary function, insulin secretion, blood glucose and adrenocorticotropic hormone secretion in a mammal, comprising regulating the expression or activity of RAIG2 and/or RAIG3 in the animal.

**58.** A prophylactic/therapeutic method for a disease selected from the group consisting of diabetes mellitus, impaired glucose tolerance, ketosis, acidosis, diabetic neuropathy, diabetic nephropathy, diabetic retinopathy, hyperlipemia, eating disorder, depression, Cushing's disease, gonadal dysfunction, dermatopathies, arthropathies, osteopenia, arteriosclerosis, thrombotic disease, dyspepsia and memory and learning disorder in a mammal, comprising administering an effective amount of a substance that enhances the expression or activity of RAIG2 and/or a substance that enhances the expression or activity of RAIG3 to the mammal.

**59.** A prophylactic/therapeutic method for a disease selected from the group consisting of obesity, hyperlipemia, type 2 diabetes mellitus, hypoglycemia, hypertension, diabetic neuropathy, diabetic nephropathy, diabetic retinopathy, edema, insulin resistance syndrome, brittle diabetes, fatty atrophy, insulin allergy, insulinoma, arteriosclerosis, eating disorder, depression, Cushing's disease, thrombotic disease, fat toxicity and cancers in the mammal, comprising administering an effective amount of a substance that inhibits the expression or activity of RAIG2 and/or a substance that inhibits the expression or activity of RAIG3 to a mammal.

FIG. 1

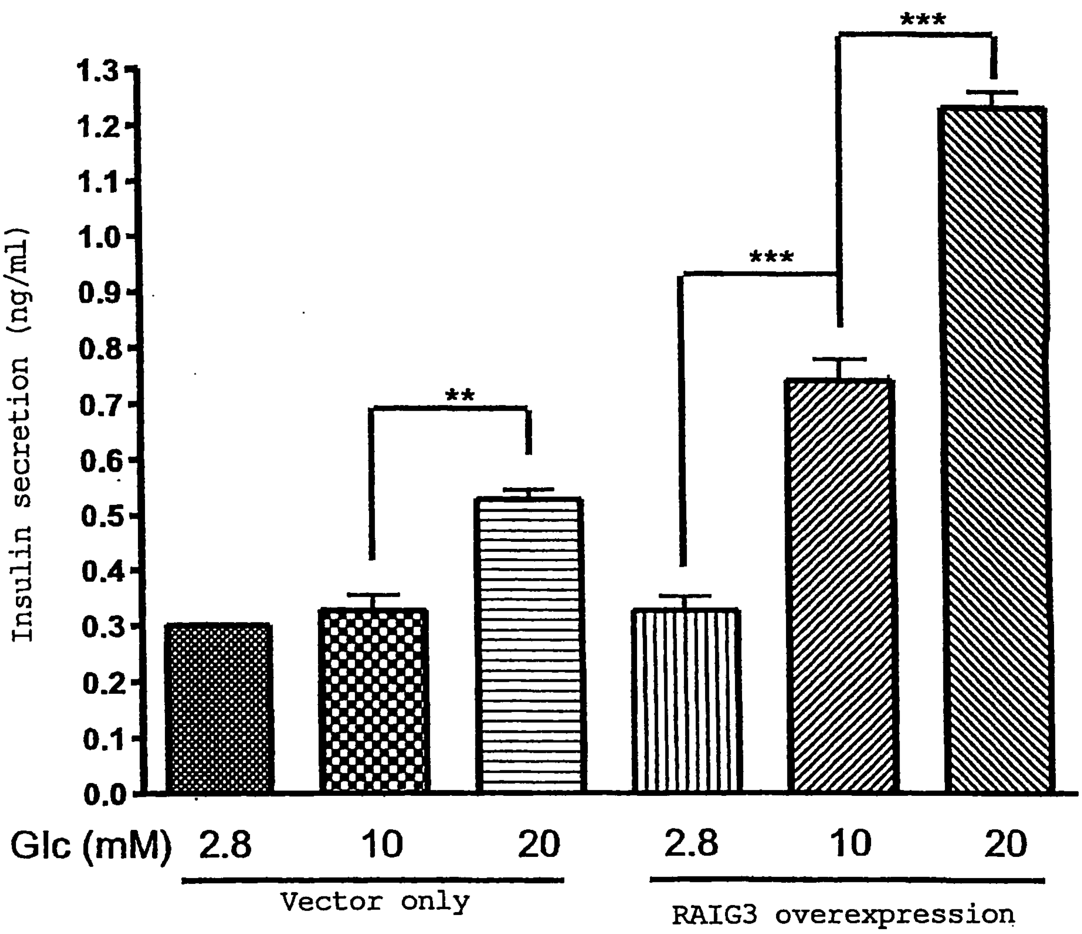
Insulin secretion (ng/ml)
1.3
1.2
1.1
1.0
0.9
0.8
0.7
0.6
0.5
0.4
0.3
0.2
0.1
0.0
**
***
***
Glc (mM) 2.8 10 20 2.8 10 20
Vector only
RAIG3 overexpression

FIG. 2 (a)

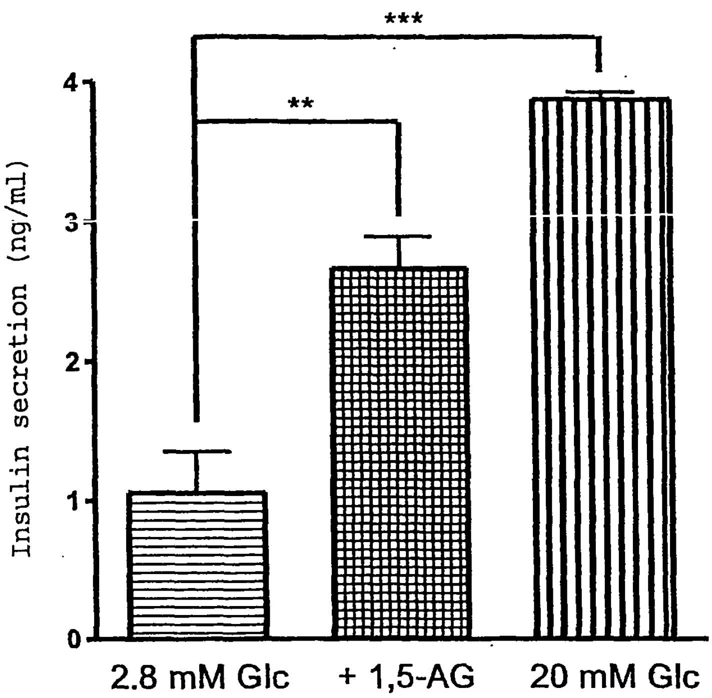
***
**
4
3
2
1
0
Insulin secretion (ng/ml)
2.8 mM Glc
+ 1,5-AG
20 mM Glc

FIG. 2 (b)

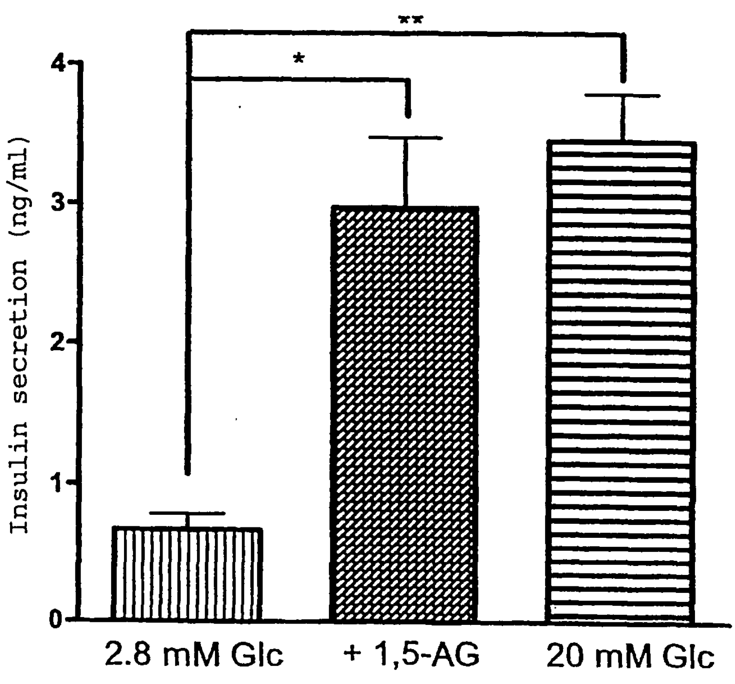
**
*
4
3
2
1
0
Insulin secretion (ng/ml)
2.8 mM Glc
+ 1,5-AG
20 mM Glc

FIG. 3

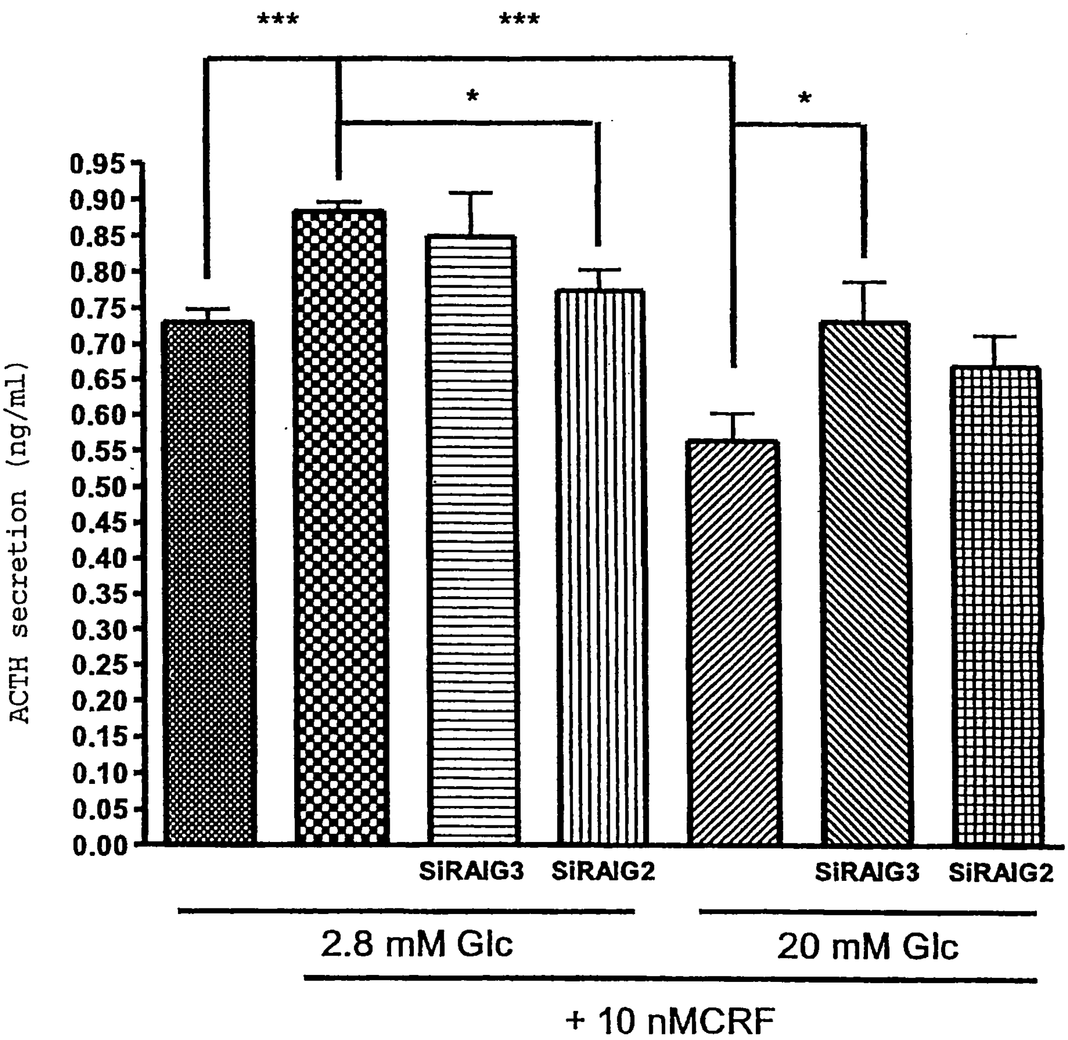
***
***
*
*
ACTH secretion (ng/ml)
0.95
0.90
0.85
0.80
0.75
0.70
0.65
0.60
0.55
0.50
0.45
0.40
0.35
0.30
0.25
0.20
0.15
0.10
0.05
0.00
SiRAIG3
SiRAIG2
SiRAIG3
SiRAIG2
2.8 mM Glc
20 mM Glc
+ 10 nMCRF

FIG. 4

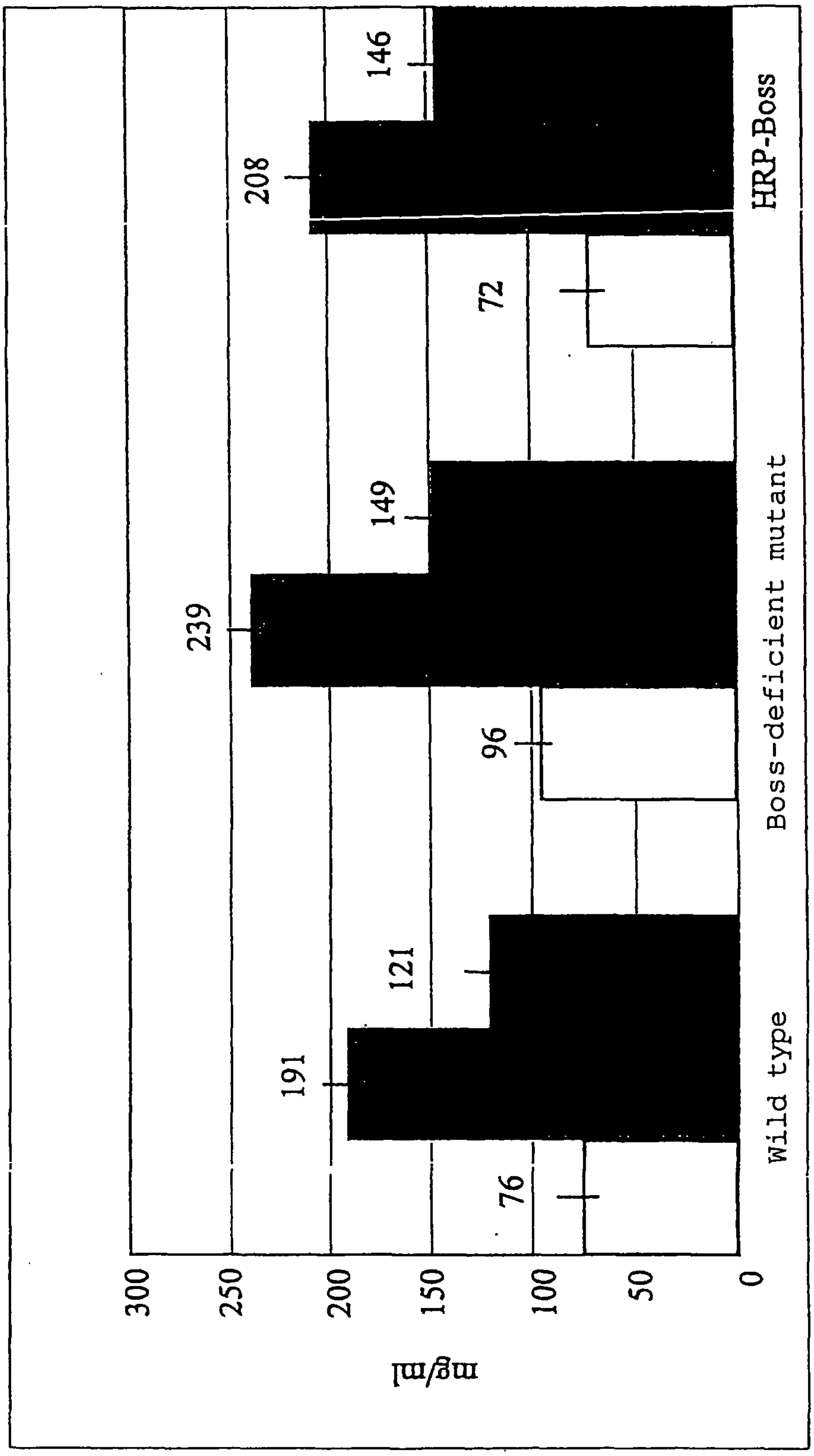
mg/ml
300
250
200
150
100
50
0
76
191
121
96
239
149
72
208
146
Wild type
Boss-deficient mutant
HRP-Boss

**INTERNATIONAL SEARCH REPORT**

International application No.
PCT/JP2007/057185

A. CLASSIFICATION OF SUBJECT MATTER
*A61K38/00*(2006.01)i, *A61K39/395*(2006.01)i, *A61K45/00*(2006.01)i, *A61K48/00*(2006.01)i, *A61P1/00*(2006.01)i, *A61P1/18*(2006.01)i, *A61P3/06*(2006.01)i, *A61P3/10*(2006.01)i, *A61P5/08*(2006.01)i, *A61P5/12*(2006.01)i,

According to International Patent Classification (IPC) or to both national classification and IPC

B. FIELDS SEARCHED

Minimum documentation searched (classification system followed by classification symbols)
A61K38/00, A61K39/395, A61K45/00, A61K48/00, C12Q1/02, C12Q1/68, G01N33/15, G01N33/50, G01N33/53

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS(STN), CAplus(STN), EMBASE(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| X | WO 2005/101012 A1 (BAYER HEALTHCARE AG.), 27 October, 2005 (27.10.05), Full text; particularly, pages 61 to 62 (Family: none) | 1-56 |
| X | WO 2005/101001 A2 (BAYER HEALTHCARE AG.), 27 October, 2005 (27.10.05), Full text; particularly, pages 61 to 62 (Family: none) | 1-56 |
| X<br>A | US 2003/235912 A1 (ISIS PHARM INC.), 25 December, 2003 (25.12.03), Full text (Family: none) | 45-46,51-52<br>1-44,47-50, 53-56 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 July, 2007 (09.07.07) | 24 July, 2007 (24.07.07) |
| Name and mailing address of the ISA/<br>Japanese Patent Office<br>Facsimile No. | Authorized officer<br><br>Telephone No. |

Form PCT/ISA/210 (second sheet) (April 2005)

INTERNATIONAL SEARCH REPORT

International application No.
PCT/JP2007/057185

C (Continuation). DOCUMENTS CONSIDERED TO BE RELEVANT

| Category* | Citation of document, with indication, where appropriate, of the relevant passages | Relevant to claim No. |
|---|---|---|
| A | Brauner-Osborne et al.,'Sequence and expression pattern of a novel human orphan G-protein-coupled receptor, GPRC5B, a family C receptor with a short amino-terminal domain', Genomics 65, 121-128 (2000) | 1-56 |
| A | Robbins et al, 'Molecular cloning and characterization of two novel retinoic acid-inducible orphan G-protein-coupled receptors (GPRC5B and GPRC5C)', Genomics 67, 8-18 (2000) | 1-56 |
| A | WO 2003/055507 A1 (SUMITOMO PHARM CO., LTD.), 10 July, 2003 (10.07.03), Full text & AU 2002367145 A1 & EP 1468694 A1 & US 2005/019320 A1 | 1-56 |

Form PCT/ISA/210 (continuation of second sheet) (April 2005)

INTERNATIONAL SEARCH REPORT

International application No.
PCT/JP2007/057185

Continuation of A. CLASSIFICATION OF SUBJECT MATTER
(International Patent Classification (IPC))

*A61P5/38*(2006.01)i, *A61P7/00*(2006.01)i, *A61P7/02*(2006.01)i, *A61P9/10*(2006.01)i, *A61P15/00*(2006.01)i, *A61P17/00*(2006.01)i, *A61P19/00*(2006.01)i, *A61P19/02*(2006.01)i, *A61P25/00*(2006.01)i, *A61P25/24*(2006.01)i, *A61P25/28*(2006.01)i, *A61P27/02*(2006.01)i, *C12Q1/02*(2006.01)i, *C12Q1/68*(2006.01)i, *G01N33/15*(2006.01)i, *G01N33/50*(2006.01)i, *G01N33/53*(2006.01)i

(According to International Patent Classification (IPC) or to both national classification and IPC)

Form PCT/ISA/210 (extra sheet) (April 2005)

**INTERNATIONAL SEARCH REPORT**

International application No.
PCT/JP2007/057185

**Box No. II Observations where certain claims were found unsearchable (Continuation of item 2 of first sheet)**

This international search report has not been established in respect of certain claims under Article 17(2)(a) for the following reasons:

1. [X] Claims Nos.: 57-59
because they relate to subject matter not required to be searched by this Authority, namely:
Claims 57 to 59 pertain to methods for treatment of the human body by therapy and thus relate to a subject matter which this International Searching Authority is not required, under the provisions of the PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03055507 A
- JP 2000333673 A
- US 6331415 B
- US 5225539 A
- US 5585089 A
- US 5693761 A
- US 5693762 A
- JP HEI5227970 B
- US 5939598 A
- US 5545806 A
- JP 2006101057 A


### Non-patent literature cited in the description


- **M.J. ROBBINS et al.** *Brain Res. Mol. Brain Res,* 2002, vol. 106, 136-144
- **H. BRAUNER-OSBORNE ; P. KROGSGAARD-LARSEN.** *Genomics,* 2000, vol. 65, 121-128
- **M.J. ROBBINS et al.** *Genomics,* 2000, vol. 67, 8-18
- **H. BRAUNER-OSBORNE et al.** *Biochim. Biophys. Acta,* 2001, vol. 1518, 237-248
- **BOWIE et al.** *Science,* 1990, vol. 247, 1306-1310
- **KARLIN et al.** *Proc. Natl. Acad. Sci. USA,* 1993, vol. 90, 5873-5877
- **ALTSCHUL et al.** *Nucleic Acids Res.,* 1997, vol. 25, 3389-3402
- **NEEDLEMAN et al.** *J. Mol. Biol.,* 1970, vol. 48, 444-453
- **MYERS ; MILLER.** *CABIOS,* 1988, vol. 4, 11-17
- **PEARSON et al.** *Proc. Natl. Acad. Sci. USA,* 1988, vol. 85, 2444-2448
- **M. BODANSZKY ; M.A. ONDETTI.** Peptide Synthesis. Interscience Publishers, 1966
- **SCHROEDER ; LUEBKE.** The Peptide. Academic Press, 1965
- **NOBUO IZUMIYA et al.** Peptide Gosei-no-Kiso to Jikken. Maruzen Co, 1975
- **HARUAKI YAJIMA ; SHUNPEI SAKAKIBARA.** Seikagaku Jikken Koza. *Tanpakushitsu no Kagaku,* 1977, vol. IV, 205
- Zoku Iyakuhin no Kaihatsu. Peptide Synthesis. Hirokawa Shoten, vol. 14
- **J. SAMBROOK et al.** Molecular Cloning. Cold Spring Harbor Lab. Press, 1989
- *Proceedings of the National Academy of Sciences of the USA,* 1968, vol. 60, 160
- *Nucleic Acids Research,* 1981, vol. 9, 309
- *Journal of Molecular Biology,* 1978, vol. 120, 517
- *Journal of Molecular Biology,* 1969, vol. 41, 459
- *Genetics,* 1954, vol. 39, 440
- *Gene,* 1983, vol. 24, 255
- *Journal of Biochemistry,* 1984, vol. 95, 87
- **VAUGHN, J.L. et al.** *In Vivo,* 1977, vol. 13, 213-217
- **MAEDA et al.** *Nature,* 1985, vol. 315, 592
- *Proceedings of the National Academy of Sciences of the USA,* 1972, vol. 69, 2110
- *Gene,* 1982, vol. 17, 107
- *Molecular & General Genetics,* 1979, vol. 168, 111
- *Methods in Enzymology,* 1991, vol. 194, 182-187
- *Proceedings of the National Academy of Sciences of the USA,* 1978, vol. 75, 1929
- *Bio/Technology,* 1988, vol. 6, 47-55
- Saibo Kogaku. Shin Saibo Kogaku Jikken Protocol. Shujunsha, 1995, vol. 8, 263-267
- *Virology,* 1973, vol. 52, 456
- **MILLER.** Journal of Experiments in Molecular Genetics. Cold Spring Harbor Laboratory, 1972, 431-433
- **BOSTIAN, K.L. et al.** *Proceedings of the National Academy of Sciences of the USA,* 1980, vol. 77, 4505
- **BITTER, G.A. et al.** *Proceedings of the National Academy of Sciences of the USA,* 1984, vol. 81, 5330
- **GRACE, T.C.C.** *Nature,* 1962, vol. 195, 788
- *Science,* 1952, vol. 122, 501
- *Virology,* 1959, vol. 8, 396
- *The Journal of the American Medical Association,* 1967, vol. 199, 519
- **PROCEEDING OF THE SOCIETY FOR THE BIOLOGICAL MEDICINE.** *Proceeding of the Society for the Biological Medicine,* 1950, vol. 73, 1

- **PRATT J.M. et al.** Transcription and Tranlation. IRL Press, 1984, 179-209
- **JOHNSTON F.B. et al.** *Nature,* 1957, vol. 179, 160-161
- **ERICKSON A.H. et al.** *Meth. Enzymol.,* 1996, vol. 96, 38-50
- **SPIRIN A.S. et al.** *Science,* 1988, vol. 242, 1162-1164
- **KIKAWA et al.** *The 21st Annual Meeting of the Molecule Biology Society of Japan*
- **J. KAWAKAMI et al.** *Pharm. Tech. Japan,* 1992, vol. 8, 247
- *PHARM. TECH. JAPAN,* 1992, vol. 8, 395
- Antisense Research and Applications. CRC Press, 1993
- *Proc. Natl. Acad. Sci. USA,* 2001, vol. 98 (10), 5572-5577
- *Nucleic Acids Res.,* 2001, vol. 29 (13), 2780-2788
- *Nature,* 2001, vol. 411 (6836), 494-498
- *Bio/Technology,* 1991, vol. 9, 88-89
- *Nat. Genet.,* 1997, vol. 15, 146-156
- *Nat. Biotechnol.,* 1996, vol. 14, 845-851
- **TOMIZUKA et al.** *Nat. Genet.,* 1997, vol. 16, 133-143
- *Nat. Biotechnol.,* 2000, vol. 18, 1086-1090
- *Nat. Biotechnol.,* 2002, vol. 20, 889-894
- **HOLT et al.** *Curr. Opin. Biotechnol.,* 2000, vol. 11, 445-449
- *J. Biol. Chem.,* 1999, vol. 274, 18218-18230
- *J. Mol. Biol.,* 1991, vol. 222, 581-597
- *Nat. Biotechnol.,* 1996, vol. 14, 309-314
- *Annu. Rev. Immunol.,* 1994, vol. 12, 433-455
- *J. Biol. Chem.,* 1999
- *Proc. Natl. Acad. Sci. USA,* 2000, vol. 14, 7969-7974
- *J. Mol. Biol.,* 2000, vol. 296, 57-86
- *Nat. Biotechnol.,* 2000, vol. 18, 852
- *Proc. Natl. Acad. Sci. USA,* 1995, vol. 92, 3938
- *J. Immunol. Methods,* 2003, vol. 272, 219-233
- *Genomics,* 1989, vol. 5, 874-879
- *Proceedings of the National Academy of Sciences of the United States of America,* 1989, vol. 86, 2766-2770
- Radioimmunoassay. Kodansha Ltd, 1974
- Sequel to the Radioimmunoassay. Kodansha Ltd, 1979
- *Yeast,* 2000, vol. 16, 11-22
- Enzyme Immonoassay. Igakushoin, 1978
- Enzyme Immonoassay. Igakushoin, 1982
- Enzyme Immonoassay. Igakushoin, 1987
- Immunochemical Techniques. Methods in ENZYMOLOGY. vol. 70
- Immunochemical Techniques. METHODS IN ENZYMOLOGY. vol. 73
- Immunochemical Techniques. METHODS IN ENZYMOLOGY. vol. 74
- Immunochemical Techniques. METHODS IN ENZYMOLOGY. vol. 84
- Immunochemical Techniques (Part E: Monoclonal Antibodies and General Immunoassay Methods. METHODS IN ENZYMOLOGY. vol. 92
- Immunochemical Techniques (Part I: Hybridoma Technology and Monoclonal Antibodies. METHODS IN ENZYMOLOGY. Academic Press Publishing, vol. 121
- **M.J. EVANS ; M.H. KAUFMAN.** *Nature,* 1981, vol. 292, 154
- **G. R. MARTIN.** *Proceedings of the National Academy of Sciences of the USA,* 1981, vol. 78, 7634
- **T.C. DOETSCHMAN et al.** *Journal of Embryology and Experimental Morphology,* 1985, vol. 87, 27